# EUROPÄISCHE PATENTSCHRIFT

(11) **EP 2 053 976 B1**
(45) Veröffentlichungstag und Bekanntmachung des Hinweises auf die Patenterteilung: **16.03.2016**
(21) Anmeldenummer: 07818732.5
(22) Anmeldetag: 05.10.2007
(51) Int. Cl.: A61B 17/00, A61B 90/00, A61B 17/12

(54) **IMPLANTIERBARE EINRICHTUNG**
IMPLANTABLE DEVICE
DISPOSITIF IMPLANTABLE

(30) Priorität: 05.10.2006 DE 102006047494; 20.10.2006 DE 102006050385
(43) Veröffentlichungstag der Anmeldung: 06.05.2009
(60) Teilanmeldung: 13000018.5
(73) Patentinhaber: pfm medical ag, 50996 Köln (DE)
(72) Erfinder: FREUDENTHAL, Franz, La Paz (BO)
(74) Vertreter: Ring & Weisbrodt
(86) Internationale Anmeldenummer: PCT/EP2007/008656
(87) Internationale Veröffentlichungsnummer: WO 2008/040555

(56) Entgegenhaltungen:
- EP-A- 0 923 912
- WO-A-2007/054117
- DE-A1- 10 302 447
- KR-A- 20010 084 836
- US-A- 6 149 665
- US-A1- 2004 073 155
- US-A1- 2005 070 992
- US-A1- 2006 058 872

## Beschreibung

Die Erfindung betrifft eine implantierbare Einrichtung zur Verwendung im menschlichen und/oder tierischen Körper zum Verschluss oder Teilverschluss von Defektöffnungen, Hohlräumen, Organwegen etc., oder zum Schaffen einer definierten Verbindungsöffnung zwischen Wandungen, Organen, Hohlräumen etc., mit einer Tragstruktur, die in einer Primärform ein großes Verhältnis von Länge zu Querausdehnung entlang einer Achse und in einer Sekundärform ein kleineres Verhältnis von Länge zu Querausdehnung entlang der Achse aufweist, wobei die Tragstruktur einen proximalen und distalen Abschnitt aufweist und nach Art eines Geflechts und/oder Gewebes und/oder Geleges und/oder Netzes geformt ist.

Die Achse ist üblicherweise die sich beim Langstrecken der implantierbaren Einrichtung ergebende Längsachse von dieser. Derartige implantierbare Einrichtungen sind im Stand der Technik bekannt. Beispielsweise offenbart die DE 103 02 447 A1 eine solche implantierbare Einrichtung, bei der der proximale und/oder der distale Abschnitt in der Sekundärform im Wesentlichen flach, scheiben- oder ringförmig ausgebildet ist, oder von einem den distalen oder proximalen Abschnitt verbindenden Zwischenschnitt nach außen weggebogen ist, einen Innenraum umgrenzend. In die implantierbaren Einrichtungen können zusätzliche Fasern oder Gewebeteile eingefügt werden, die als Membranelement dienen. Durch das Vorsehen solcher Membranelemente können beispielsweise Defektöffnungen im Herzen eines Menschen geschlossen werden. Gegebenenfalls kann dort Gewebe aufwachsen.

Auch aus der DE 100 00 137 A1 ist eine solche implantierbare Einrichtung zum Verschließen von Defektöffnungen im menschlichen oder tierischen Körper bekannt, bei der die Sekundärform in etwa die Form einer Doppelscheibe annimmt mit einem proximalen Scheibenelement und einem distalen Scheibenelement zur Aufnahme der Umgebung einer Defektöffnung zwischen den Scheibenelementen. Die Tragstruktur ist im Wesentlichen einstückig ohne Fügeverbindungen ausgebildet, wobei sie aus einem geschnittenen Röhrchen hergestellt wird. Innerhalb der implantierbaren Einrichtung kann wiederum eine Membran vorgesehen werden, die an verschiedenen Seiten der implantierbaren Einrichtung angeordnet oder sogar wie eine Socke mit entsprechenden Öffnungen für ein Platziersystem über die implantierbare Einrichtung selbst gezogen werden kann.

Auch die DE 103 38 702 B3 offenbart eine entsprechende implantierbare Einrichtung, bei der ein proximaler und ein distaler Retentionsbereich mit einem dazwischen angeordneten zylindrischen Steg vorgesehen ist, wobei der proximale Retentionsbereich eine zum proximalen Ende hin offene Form aufweist. Die Enden der Drähte oder Fäden des distalen Retentionsbereichs der implantierbaren Einrichtung sind mit einer Fassung zusammengefasst. Ferner kann eine Gewebeeinlage zum vollständigen Verschließen eines Shunts im zylindrischen Steg oder im proximalen Retentionsbereich angeordnet sein.

Weitere implantierbare Einrichtungen sind beispielsweise auch aus der US 5,846,261 und der US 5,725,552 bekannt, die beide eine Glockenform aufweisen. Auch in der WO 93/13712 ist ein Implantat zum Verschluss von Septumdefekten beschrieben, das im implantierten Zustand eine Doppelkonus- oder Doppelscheibenkonfiguration annimmt, wobei jeweils die äußeren Strukturen aus nicht direkt miteinander verbundenen Drahtelementen gebildet sind. Diese sind mit Stoffsegeln bespannt, wobei die Stoffsegel in einem den zu verschließenden Defekt entsprechenden Radius miteinander vernäht sind. Nachteilig ist an diesem System, dass das aus einer Vielzahl von Bauelementen aufgebaute Implantat einen hohen Montageaufwand erfordert.

In der WO 95/27448 ist ein Implantat beschrieben, das als Venenfilter eingesetzt und als tragende Struktur für einen Septumverschluss verwendet werden soll. Dabei wird aus einer Reihe von Einzeldrähten ein relativ langgestreckter Doppelkonus gebildet, wobei in einer Ausführungsform die Konen zueinander gerichtet sind nach Art eines Knochens und in einer weiteren Ausführungsform die Konen gleichgerichtet sind, ähnlich einem Fliegenpilz.

Aus der US 5,433,727 A ist ein Implantat zum Verschließen großer Defekte im Herzen bekannt, wie beispielsweise eines Atrium-Septum-Defekts (ASD) oder eines Ventrikel-Septum-Defekts (VSD). Bei diesem wird eine Art Schirm vor einen Septumdefekt gesetzt und durch den Defekt hindurch mit einem Gegenverschluss gesichert, der im Wesentlichen aus vier aus jeweils einem Draht hergestellten Schlaufen gebildet wird, die sich beim Ausstoßen aus dem Katheter entfalten und ein Durchrutschen des Implantats zu der Schirmseite verhindern sollen. Das Verschlussimplantat enthält ferner eine entfaltbare Schaumharzscheibe, die ein beschichtetes Drahtskelett in Form eines "X", aufgebracht auf die Schaumscheibe, sowie eine einstellbare Schlaufe, angebracht an der Mitte des Drahtskeletts, umfasst.

Gemäß der WO 97/28774 wird ein Implantat beschrieben, das sich beim Ausstoßen aus dem Katheter selbsttätig aufgrund einer eingeprägten Sekundärform entfaltet und durch elastische Kräfte selbst den Abmessungen des Defekts in einem weiten Rahmen anpasst. Die eingeprägte Struktur klemmt sich nach Art einer Doppelscheibe an beiden Seiten des Defekts gegen den diesen umgebenden Bereich an. Das Implantat ist aus einer Reihe von Drahtelementen gebildet, die durch geeignete Fügeverfahren, wie beispielsweise Ultraschallschweißen oder Hartlöten, miteinander verbunden sind. Außerdem ist das Implantat mit einer Bespannung versehen, die an den Drahtelementen befestigt ist.

Auch aus der WO 99/12478 A1 ist wiederum eine entsprechende implantierbare Einrichtung bekannt, bei der ein Geflecht aus einer Vielzahl verflochtener Nitinoldrähte in einer Hantel- bzw. Jojo-Form vorgesehen ist. Die implantierbare Einrichtung ist in ihrer Primärform als Rundgeflecht ausgebildet, das an seinen beiden Enden lose Drahtenden aufweist, die nachfolgend jeweils in einer Hülse gefasst und verschweißt werden. Hierdurch weisen die gebildeten implantierbaren Einrichtungen jeweils an ihren proximalen und distalen Seiten abstehende Hülsen auf.

Die US 2003/0191495 A1 offenbart einen Septumoccluder mit einem proximalen Ankerelement und einem distalen Ankerelement, die zwischen sich ein Verbindungselement aufweisen.

Weitere Implantate und Kathetersysteme zur Platzierung eines solchen Implantats sind beispielsweise in der US 5,108,420 A, der DE 42 22 291 A1, der DE 28 22 603 A, WO 96/01591 und der EP 0 474 887 A1 beschrieben. Eine intravasculäre Filtereinrichtung mit einem kuppelförmigen Schirm mit Halteseilen wird in der DE 695 29 338 T2 beschrieben.

Aus der US 2006/0058872 A1 ist eine Einrichtung gemäß dem Oberbegriff des Anspruchs 1 bekannt.

Mit all den in den vorstehenden Druckschriften des Standes der Technik beschriebenen implantierbaren Einrichtungen können zwar mehr oder weniger gut Defektöffnungen verschlossen werden, insbesondere wenn sie mit Membranelementen versehen sind. Allerdings sind all diese implantierbaren Einrichtungen des Standes der Technik nur dann sinnvoll einsetzbar, wenn genügend Platz für deren Entfaltung an dem jeweiligen Implantationsort zur Verfügung steht. Gerade im Bereich des linken Vorhofs des Herzens steht jedoch nur ein sehr begrenzter Raum für eine Implantation bzw. für ein Entfalten einer implantierbaren Einrichtung zur Verfügung.

Der vorliegenden Erfindung liegt daher die Aufgabe zugrunde, eine implantierbare Einrichtung nach dem Oberbegriff des Anspruchs 1 dahingehend fortzubilden, dass diese auch für die Implantation an Stellen im menschlichen und/oder tierischen Körper geeignet ist, an denen nur ein sehr geringer Raum zur Implantation zur Verfügung steht, wie beispielsweise im linken Vorhof eines Herzens, wobei die implantierbare Einrichtung aber auch eine besonders gute Stabilität gegen ein ungewolltes Deplatzieren nach der Implantation aufweisen sollte, sowie ein Verfahren zum Entfalten der implantierbaren Einrichtung vorzusehen.

Die Aufgabe wird durch eine implantierbare Einrichtung nach dem Oberbegriff des Anspruchs 1 dadurch gelöst, dass zumindest der eine Abschnitt in der Sekundärform einen nach außen von dem anderen Abschnitt wegweisenden ersten Teilabschnitt und zum Bilden zumindest einer Doppellagigkeit einen sich als erster aus der Primärform in die Sekundärform entfaltenden, in Richtung zu oder weg von dem anderen Abschnitt auf den ersten Teilabschnitt zurück gefalteten zweiten Teilabschnitt aufweist, wobei der umgefaltete Teilabschnitt (13) nach innen in die implantierbare Einrichtung (1) hinein zurück gefaltet ist. Die Aufgabe wird auch durch ein Verfahren zum Entfalten der implantierbaren Einrichtung aus einer Primärform in eine Sekundärform gelöst, bei dem a) die implantierbare Einrichtung in einer langgestreckten Primärform in einem Katheter angeordnet wird, b) die implantierbare Einrichtung aus dem Katheter herausgeschoben wird, um sich in ihre Sekundärform mit einem kleinen Verhältnis von Länge zu Querausdehnung zu entfalten, c) sich ein als erster aus dem Katheter herausgeschobener Teilabschnitt eines ersten Abschnitts der implantierbaren Einrichtung entfaltet und beim Herausschieben einen weiteren diesem benachbarten Teilabschnitt des Abschnitts der implantierbaren Einrichtung aus dem Katheter rückwärts in Richtung zu dem Katheter faltet, und d) sich die implantierbare Einrichtung beim weiteren Herausschieben aus dem Katheter vollständig entfaltet. Weiterbildung der Erfindung sind in den abhängigen Ansprüchen definiert.

Dadurch wird eine implantierbare Einrichtung geschaffen, die lediglich wenig Raum für die Implantation benötigt, da beim Herausschieben der implantierbaren Einrichtung aus einem entsprechenden Katheter am Implantationsort diese nur geringfügig üblicherweise nach distal aus dem Katheter herausgeschoben wird, der als erster herausgeschobene Teilabschnitt sich in Richtung proximal umfaltet und beim weiteren Herausschieben der implantierbaren Einrichtung aus dem Katheter sich der daran anschließende als zweiter herausgeschobene nach distal weisende Teilabschnitt des distalen Abschnitts entfaltet. Der als erster herausgeschobene Teilabschnitt faltet sich beim weiteren Herausschieben der implantierbaren Einrichtung aus dem Katheter rückwärts auf den anderen Teilabschnitt zurück, so dass eine Doppellagigkeit der beiden aufeinander gefalteten Teilabschnitte entsteht. Durch die Doppellagigkeit wird der Abschnitt der implantierbaren Einrichtung deutlich gegenüber einlagigen Einrichtungen des Standes der Technik verstärkt, so dass ein besonders sicherer Sitz an einem Implantationsort gegeben ist. Ferner erweist sich die zumindest Doppellagigkeit auch beim Entfalten gegenüber Einrichtungen des Standes der Technik als vorteilhaft, da beim Entfalten direkt der volle Durchmesser der implantierbaren Einrichtung entfaltet wird und hierdurch insbesondere auch große zu verschließende Öffnungen sogar bereits bei nicht vollständig entfalteter Einrichtung ohne die Gefahr eines Durchrutschens der Einrichtung durch die Öffnung verschlossen werden können.

Wird die implantierbare Einrichtung innerhalb einer Defektöffnung, beispielsweise in einer Herzwandung, angeordnet, durchdringt ein vorteilhaft zwischen dem proximalen und dem distalen Abschnitt vorgesehener Zwischenabschnitt mit gegenüber dem proximalen und dem distalen Abschnitt vermindertem Durchmesser die Defektöffnung bzw. die Wandung, wonach sich beim weiteren Herausschieben der implantierbaren Einrichtung aus dem Katheter proximal von der Defektöffnung bzw. der Wandung der proximale Abschnitt der implantierbaren Einrichtung entfaltet.

Aufgrund des beim Herausschieben der implantierbaren Einrichtung aus dem Katheter erfolgenden Zurückklappens und Aufeinanderfaltens der beiden Teilabschnitte des einen Abschnitts, insbesondere des distalen Abschnitts, ist nur ein geringer Raumbedarf distal erforderlich, was die erfindungsgemäße implantierbare Einrichtung gerade für beispielsweise die Implantation im linken Vorhof eines Herzens besonders geeignet macht.

Zumindest der eine Abschnitt, insbesondere der proximale Abschnitt, ist vorteilhaft in seinem Randbereich im Wesentlichen scheibenförmig oder bauchig konvex ausgebildet. Grundsätzlich können beide Abschnitte eine ähnliche Formgebung aufweisen. Ferner kann der eine Abschnitt scheibenförmig und der andere bauchig konvex ausgebildet sein. Vorteilhaft weist der eine Abschnitt eine Tragstruktur mit einem im Wesentlichen geschlossenen Ende auf. Insbesondere bildet die Tragstruktur proximal ein bezüglich der implantierbaren Einrichtung außenseitig angeordnetes zusammengefasstes proximales Ende aus. Das im Wesentlichen geschlossene Ende kann ferner als ein Geflecht- und/oder Gelege- und/oder Gewebe- und/oder Netzenden der Tragstruktur zusammenfassendes, in einem Hülsenelement aufgenommenes Ende ausgebildet sein. Es kann auch anderweitig zusammengefasst sein.

Die implantierbare Einrichtung kann ferner stentförmig ausgebildet sein mit einem doppellagig aufeinander gefalteten endseitigen Abschnitt. Vorteilhaft ist hierbei der zweite Teilabschnitt nach innen auf den ersten Teilabschnitt zurück gefaltet.

Bei der implantierbaren Einrichtung zur Verwendung im menschlichen und/oder tierischen Körper zum Verschluss oder Teilverschluss von Defektöffnungen, Hohlräumen, Organwegen etc. mit einer aus einer Primärform in eine eingeprägte Sekundärform reproduzierbar umwandelbaren Tragstruktur, wobei die Tragstruktur einen proximalen und einen distalen Abschnitt aufweist und nach Art eines Geflechts und/oder Gewebes und/oder Geleges und/oder Netzes geformt ist und wobei der eine Abschnitt eine sich nach außen hin öffnende Form aufweist und der andere Abschnitt eine bauchige geschlossene, insbesondere durch ein Hülsenelement geschlossene, Form aufweist, weist der mit der geöffneten Form versehene Abschnitt vorteilhaft zumindest zwei doppellagig aufeinander gefaltete Teilabschnitte auf.

Ist der proximale Abschnitt der implantierbaren Einrichtung etwa scheibenförmig ausgebildet, kann er sich besonders gut beispielsweise an einer Wandung, die eine Defektöffnung umgibt, innerhalb derer die implantierbare Einrichtung angeordnet ist, anlegen. Auch eine bauchige bzw. konvexe Ausbildung des proximalen Abschnitts kann einen sehr guten Halt der implantierbaren Einrichtung am Implantationsort von proximal aus gewährleisten, gerade bei Anordnung der implantierbaren Einrichtung an einem leicht gekrümmten Implantationsort. Bei Vorsehen einer solchen bauchigen Ausbildung wird das proximale Ende der implantierbaren Einrichtung vorteilhaft zusammengefasst, so dass eine evtl. Durchgangsöffnung durch die implantierbare Einrichtung so gering wie möglich gehalten werden kann. Hierdurch kann ein Verschluss einer Öffnung gebildet werden. Grundsätzlich kann auch eine gezielte Durchgangsöffnung durch die implantierbare Einrichtung hier vorgesehen werden. Wird ein Verschluss gewünscht, kann z.B. zusätzlich das bereits erwähnte Hülsenelement auf das zusammengefasste proximale Ende der implantierbaren Einrichtung aufgefügt werden.

Aufgrund des doppellagigen aufeinander zurückgefalteten Ausbildens des distalen Abschnitts kann eine besonders gute Stabilität geschaffen werden, so dass ein besonders guter Halt der implantierbaren Einrichtung an der Implantationsstelle ermöglicht wird. Das Entfalten der implantierbaren Einrichtung kann gerade im nicht besonders einfach proximal zugänglichen distalen Bereich besonders kritisch sein, da es beispielsweise zu einem so genannten Kobra-Effekt kommen kann, bei dem sich das Implantat nicht ordnungsgemäß entfaltet, sondern im zusammengefalteten Zustand einseitig wie der Kopf einer Kobra abknickt. Hier ist es besonders wichtig, dass sich die implantierbare Einrichtung besonders zuverlässig in der gewünschten Form entfaltet. Ein Eingreifen auf der distalen Seite einer Implantationsstelle ist in vielen Fällen schwierig, da die Zugänglichkeit dort nur von proximal, beispielsweise durch eine Defektöffnung, gegeben ist. Somit erweist es sich als besonders vorteilhaft, dass aufgrund der partiellen Verstärkung der implantierbaren Einrichtung auf der distalen Seite durch das Vorsehen der aufeinandergefalteten Teilabschnitte des distalen Abschnitts eine besonders gute Stabilität und somit Sicherheit gegeben ist, dass sich die implantierbare Einrichtung ordnungsgemäß distal entfaltet.

Die Doppellagigkeit des distalen Abschnitts stabilisiert die implantierbare Einrichtung randseitig, die Implantationsstelle, wie z.B. eine Defektöffnung, umgebend und hilft somit auch bei der Platzierung und Befestigung der implantierbaren Einrichtung an der Implantationsstelle. Der in Richtung zu dem proximalen Abschnitt zurückgefaltete zweite Teilabschnitt des distalen Abschnitts ist vorteilhaft schmal in Form eines umlaufenden dünnen Randes ausgebildet, da hierdurch eine noch geringere Entfaltlänge erforderlich ist, so dass der Raumbedarf beim Entfalten der implantierbaren Einrichtung noch geringer ist. Alternativ oder partiell zusätzlich kann der in Richtung zu dem proximalen Abschnitt zurückgefaltete zweite Teilabschnitt des distalen Abschnitts sich bis nahe an oder bis an den Zwischenabschnitt zwischen dem proximalen und dem distalen Abschnitt heran erstrecken, insbesondere an diesem zur Anlage kommen. Hierdurch ist über die gesamte Erstreckung des nach distal weisenden Teilabschnitts des distalen Abschnitts eine Stabilisierung aufgrund der Doppellagigkeit des distalen Abschnitts möglich. Ferner kann der zweite Teilabschnitt, der beispielsweise an einer Wandung im Herzen eines Menschen oder Tieres anliegt, federnd an dieser anliegen, indem entweder der gesamte distale Abschnitt leicht gekrümmt oder gewölbt ausgebildet ist, oder zumindest der eine Teilabschnitt gegenüber dem anderen Teilabschnitt des distalen Abschnitts mit einem vorbestimmbaren oder vorbestimmten Abstand aufeinanderliegen, so dass eine solche Federwirkung erzeugt werden kann, die einen besonders stabilen Sitz an der Implantationsstelle ermöglicht.

Vorteilhaft ist der Durchmesser eines zwischen dem proximalen und dem distalen Abschnitt vorgesehenen Zwischenabschnitts mit gegenüber dem proximalen und dem distalen Abschnitt vermindertem Durchmesser und/oder der Durchmesser einer sich durch die implantierbare Einrichtung hindurch erstreckenden Durchgangsöffnung vorbestimmbar dimensioniert zum Schaffen der definierten Verbindungsöffnung zwischen Wandungen, Organen, Hohlräumen in einem menschlichen und/oder tierischen Körper. Die Durchgangsöffnung durch die implantierbare Einrichtung bzw. den Zwischenabschnitt zwischen dem distalen und dem proximalen Abschnitt der implantierbaren Einrichtung kann also so dimensioniert sein, dass eine gewünschte definierte Verbindungsöffnung zwischen zwei Kammern, Hohlräumen, Wandungen, Organen etc. in einem menschlichen oder tierischen Körper geschaffen werden kann.

Vorteilhaft kann sich der distale Abschnitt der implantierbaren Einrichtung unabhängig von deren proximalem Abschnitt entfalten. Insbesondere kann sich der distale Abschnitt vollständig und unabhängig vom proximalen Abschnitt entfalten. Hierdurch ist es möglich, auch größere Defekte zu verschließen, was beispielsweise mit den implantierbaren Einrichtungen des Standes der Technik nicht möglich ist, da diese bei der Platzierung im Bereich von großen Defekten im Herzen eines Menschen oder Tieres häufig zur rechten Seite des Herzens hin abrutschen und somit ihre Wirkung nicht zeigen.

Im Bereich des proximalen und/oder distalen Abschnitts und/oder zwischen den proximalen oder distalen Abschnitten kann zumindest ein Membranelement vorgesehen sein. Das Membranelement kann an dem Geflecht, Gewebe, Gelege oder Netz der Tragstruktur der implantierbaren Einrichtung in geeigneter Weise, beispielsweise dort angenäht, angeklebt oder in anderer Weise befestigt sein. Vorteilhaft ist die Befestigung zwischen Membranelement und Tragstruktur durch Vernähen im Bereich des äußeren Randes der Tragstruktur vorgesehen. Insbesondere ist die Befestigung des Membranelements an der Tragstruktur durch Umschlingen eines Randes des Membranelements und von randseitigen Schlingen der Tragstruktur mit einem fadenartigen Element und Vorsehen zumindest einer Verknotung an einer randseitigen Schlinge der Tragstruktur vorgesehen. Durch das randseitige Vernähen von Membranelement und Tragstruktur kann nicht nur eine gute und sichere Befestigung, sondern auch eine vollständige Abdeckung der Tragstruktur durch das Membranelement problemlos vorgesehen werden, ohne die Gefahr eines Verrutschens von diesem. Das Membranelement kann vorteilhaft als echtes Mesh ausgebildet sein, sodass die einzelnen Fäden des Membranelements untereinander verbunden sind und ein Verschieben von diesen hierdurch unterbunden wird. Durch diese Formgebung des Membranelements kann die Epithelbildung gefördert und eine Thrombenbildung vermieden werden. Das zumindest eine Membranelement kann zwischen durch die Tragstruktur gebildete mehrlagig angeordnete Beinchen einfügbar oder eingefügt sein. Die Beinchen können im zentralen Bereich der implantierbaren Einrichtung angeordnet sein, so dass dort das Membranelement problemlos einfügbar ist.

Als weiter vorteilhaft erweist es sich, wenn die Abmessungen des Membranelements etwa denen einer zu verschließenden Defektöffnung entsprechen. Beispielsweise ist es aufgrund der zumindest doppellagigen rückwärts in Richtung zu dem proximalen Abschnitt gerichteten Ausbildung des distalen Abschnitts mit zwei Teilabschnitten möglich, das Membranelement so an der implantierbaren Einrichtung zu befestigen, dass dieses die linke Seite des Herzens im Bereich einer Defektöffnung vollständig abdeckt. Das Risiko, dass Thromben, die sich im Netz der Tragstruktur der implantierbaren Einrichtung bilden können, Embolien verursachen, ist dabei minimal im Gegensatz zum Stand der Technik, der eine Kombination von Polyester und Nitinol für die implantierbare Einrichtung als Membranelement verwendet. Bei diesem liegen im Bereich der Wandung, die eine Defektöffnung umgibt, Polyester und Nitinol aufeinander, was dadurch zu Thrombenbildung führen kann, die zu den auch in Fachzeitschriften beschriebenen Komplikationen führen, wie beispielsweise Kopfschmerzen oder sogar vorübergehende Blindheit. Diese Komplikationen treten aufgrund der Möglichkeit, das Membranelement erfindungsgemäß lediglich so groß auszubilden, wie es der Defektöffnung entspricht, bei Verwendung einer erfindungsgemäßen implantierbaren Einrichtung vorteilhaft nicht mehr auf.

Weiter erweist es sich als vorteilhaft, das Membranelement in der implantierbaren Einrichtung so zu befestigen, dass das Membranelement in der Sekundärform in der implantierbaren Einrichtung etwa die Abmessung einer zu verschließenden Defektöffnung annimmt. Somit ist es möglich, das Membranelement an der implantierbaren Einrichtung genau so zu befestigen, dass es exakt die gleiche Größe wie das jeweils zu verschließende Loch bzw. die zu verschließende Defektöffnung annimmt. Hierdurch können vorteilhaft kleinstmögliche Membranelemente verwendet werden, was gerade deswegen vorteilhaft ist, da das Thrombenrisiko immer geringer wird, je kleiner das Membranelement ausgebildet ist. Auf diese Art und Weise wird der eine Defektöffnung umgebende Rand, beispielsweise einer Wandung im Herzen eines Menschen oder Tieres, ausschließlich von dem Geflecht bzw. Netz der Tragstruktur der implantierbaren Einrichtung umgeben, nicht von einer Kombination von diesem Geflecht oder Netz mit dem Membranelement, wodurch das Thrombenrisiko erheblich verringert werden kann.

Als besonders vorteilhaft erweist es sich, wie erwähnt, wenn das Membranelement so bemessen und an der implantierbaren Einrichtung befestigt ist, dass das Membranelement in der Sekundärform der implantierbaren Einrichtung diese im Wesentlichen vollständig abdeckt, insbesondere den distalen Abschnitt im Wesentlichen vollständig abdeckt. Als vorteilhafter Effekt zeigt sich ein vermindertes Risiko einer Thrombenbildung. Dies erweist sich besonders auf der linken Seite des Herzens als vorteilhaft, da von dort aus das Blut zum Gehirn eines Menschen oder Tieres fließt, so dass eine Thrombenbildung besonders bedrohliche Folgen haben kann. Von der rechten Seite des Herzens eines Menschen oder Tieres fließt das Blut zur Lunge, wo Thromben gefiltert und aufgelöst werden können, so dass eine Thrombenbildung dort nicht so negative Folgen hat.

Ferner können selbstverständlich mehrere Membranelemente in einer implantierbaren Einrichtung verwendet werden, was zu einer deutlichen Verbesserung der Verschlussmöglichkeiten hinsichtlich der implantierbaren Einrichtung in einer zu verschließenden Öffnung führt. Ferner kann der Strukturaufbau (Netz, Gelege, Gewebe, Geflecht etc.) der Tragstruktur der implantierbaren Einrichtung so dicht ausgeführt werden, dass kein Membranelement mehr benötigt wird und die Tragstruktur der implantierbaren Einrichtung selbst die Funktion eines solchen Membranelements übernimmt. Der Strukturaufbau der Tragstruktur der implantierbaren Einrichtung ist dabei so dicht ausgebildet, dass die Tragstruktur als Membranelement wirkt.

Die Tragstruktur der implantierbaren Einrichtung kann beispielsweise aus einem Formgedächtnismaterial, insbesondere einem Metall oder einer Metalllegierung, insbesondere aus Nitinol, oder einem Kunststoffmaterial bestehen. Das Membranelement, sofern ein solches vorgesehen ist, kann aus einem Kunststoffmaterial, insbesondere aus Polyester oder einem anderen Polymer bestehen. Die Tragstruktur der implantierbaren Einrichtung ist vorteilhaft aus einem einzigen drahtartigen Element geformt. Sie kann jedoch auch aus mehreren drahtartigen Elementen geformt sein. Hierbei kann die Tragstruktur aus einem einfachen, aus einem zweifachen, aus einem zweifachen gewundenen, aus einem dreifachen, aus einem dreifachen geflochtenen drahtartigen Element, insbesondere Nitinol-Draht, oder aus einem mehrfach gewundenen, verdrillten oder geflochtenen drahtartigen Element, insbesondere Nitinol-Draht, geformt sein. Ferner können Kombinationen der vorstehenden Varianten vorgesehen sein. Als besonders vorteilhaft erweist es sich, dass bei einer solchen Ausbildung der implantierbaren Einrichtung diese bei einem Herausschieben aus einem Katheter nicht die bereits genannte Kobra-Form annimmt, was bei Systemen des Standes der Technik mit anderen Aufbauten der sie bildenden Struktur zu großen Problemen gerade bei großen implantierbaren Einrichtungen führt. Die Tragstruktur der implantierbaren Einrichtung kann mit Fasern durchflochten sein, insbesondere mit Polymerfasern. Hierdurch wird eine dichtere Tragstruktur geschaffen, die insbesondere bei einer stentförmigen implantierbaren Einrichtung oder zum Ersatz für ein separates Membranelement vorteilhaft genutzt werden kann.

Durch die Verwendung lediglich eines einzigen drahtartigen Elements zum Ausbilden der Tragstruktur (Netz, Gewebe, Geflecht, Gelege etc.) der implantierbaren Einrichtung müssen keine Schweiß- oder Lötstellen als Verbindungsstellen zwischen einzelnen drahtartigen Elementen vorgesehen werden, was dazu führt, dass kaum eine Korrosionsgefahr besteht und ebenfalls auch Bruchstellen, die ansonsten an solchen Verbindungsstellen auftreten könnten, nicht zu befürchten sind. Derartige Korrosions- und Bruchstellen können ansonsten zu einem Versagen der implantierbaren Einrichtung und sogar zu Verletzungen des Patienten bzw. zu Komplikationen nach der Implantation bzw. bei Bruchstellen ggf. auch während der Implantation, führen. Derartige Probleme des Standes der Technik treten somit bei der erfindungsgemäßen implantierbaren Einrichtung vorteilhaft nicht mehr auf.

Das zumindest eine drahtartige Element kann eine runde oder flache Querschnittsform oder eine beliebige Kombination aus einer flachen und runden Querschnittsform aufweisen. Ferner kann die Tragstruktur der implantierbaren Einrichtung endseitig eine oder mehrere Schlingen oder Ösen ausbilden. Die Schlingen oder Ösen können in Richtung des übrigen Teils der Tragstruktur in einen verdrillten oder verflochtenen Abschnitt übergehen, so dass partiell eine größere Stabilität bei gleichzeitigem Schaffen eines weniger dichten Geflechts bzw. Netzes ereicht werden kann. Die Enden des einen drahtartigen Elements sind vorteilhaft in der Fläche der Tragstruktur miteinander verwoben. Hierdurch ragen endseitig aus der Tragstruktur keine Drähte heraus, die die Implantationsstelle umgebendes Gewebe oder Adern verletzen könnten.

Vorteilhaft umfasst die Tragstruktur der implantierbaren Einrichtung miteinander verwobene oder verflochtene verdrillte drahtartige Elemente oder miteinander verdrillte Abschnitte drahtartiger Elemente, die an Kreuzungspunkten einzeln oder als verdrillte Elemente durcheinander hindurch geflochten sind. Die Tragstruktur der implantierbaren Einrichtung als solche kann aus einem oder mehreren miteinander verwobenen oder verflochtenen drahtartigen Elementen bestehen, wobei auch Abschnitte von drahtartigen Elementen miteinander verdrillt sein können, so dass sie ebenfalls als ein Elementpaar definiert werden können. Bei Vorsehen von miteinander verdrillten drahtartigen Elementpaaren können an den Kreuzungspunkten die einzelnen drahtartigen Elemente jeweils einzeln durchflochten werden oder das gesamte Elementpaar kann durch das andere Elementpaar hindurch geflochten werden. Bei letzterer Variante umgeben die beiden drahtartigen Elemente oder die beiden miteinander verdrillten Abschnitte der drahtartigen Elemente das andere Elementpaar. In entsprechender Weise können auch dickere Stränge aus drahtartigen Elementen oder Abschnitten drahtartiger Elemente miteinander verflochten werden. Durch das Verdrillen der drahtartigen Elemente oder von Abschnitten von diesen kann eine höhere Stabilität und durch Verflechten der verdrillten Elementpaare in der genannten Art und Weise kann die Position der einzelnen Elementpaare in der Tragstruktur gesichert werden, so dass nicht ungewollt unterschiedlich große Löcher in der Tragstruktur durch einseitigen Zug an der implantierbaren Einrichtung auftreten können.

Die Tragstruktur kann aus zumindest einem drahtartigen Element einlagig, doppellagig oder mehrlagig ausgebildet sein. Hierdurch lässt sich nicht nur die Stabilität der Tragstruktur beeinflussen, sondern natürlich auch deren Dichte, so dass ein Verschluss einer Defektöffnung ggf. sogar ohne ein Membranelement erfolgen kann.

Die einzelnen Schichten oder Lagen der Tragstruktur können aus demselben oder aus unterschiedlichen Materialien bestehen. Beispielsweise kann zumindest eine der Lagen aus Nitinol, zumindest eine weitere aus Polyester und zumindest eine weitere aus PTFE bestehen. Hierdurch lassen sich Tragstrukturen mit einer gewünschten Stabilität und ggf. partiell unterschiedlichen Festigkeiten herstellen. Ferner kann zwischen zumindest zwei Lagen zumindest ein Membranelement angeordnet sein. Dieses kann zwischen diesen Lagen der Tragstruktur gut festgehalten werden. Vorteilhaft kann die Materialwahl für das Membranelement an die der einzelnen Lagen der Tragstruktur, zwischen denen es angeordnet wird, angepasst werden.

Die implantierbare Einrichtung kann eine konzentrische oder exzentrische Form aufweisen. Vorteilhaft kann sich die Tragstruktur der implantierbaren Einrichtung mit dem zumindest einen Teilabschnitt des einen Abschnitts, der in der Sekundärform in Richtung zu dem anderen Abschnitt zurückgefaltet ist, selbstständig an die Formgebung an einer Implantationsstelle anpassen. Aufgrund der besonderen Ausbildung des insbesondere distalen Abschnitts der implantierbaren Einrichtung mit dem einen zurückgefalteten Teilabschnitt weist die implantierbare Einrichtung bzw. deren Tragstruktur keine feste Mitte auf, so dass sich die implantierbare Einrichtung an jede Form eines Defekts flexibel anpassen oder bereits hinsichtlich ihrer eingeprägten Sekundärform angepasst werden kann. Handelt es sich beispielsweise um eine Defektöffnung mit einer ovalen Form, kann auch die implantierbare Einrichtung eine ovale Form annehmen, wobei sie besonders gut in die Defektöffnung passt. Auch eine Anpassung an benachbarte Wandungen, an denen sich die implantierbare Einrichtung anlegt, ist problemlos möglich, wobei ein besonders sicherer und guter Halt an der Implantationsstelle aufgrund des zurückgefalteten Teilabschnitts und somit zumindest doppelt liegenden Abschnitts (z.B. distalen Abschnitts) gegeben ist. Oftmals kommt es vor, dass eine zu verschließende Defektöffnung benachbart zu einer Aorta angeordnet ist. Die implantierbare Einrichtung passt sich vorteilhaft problemlos in ihrer Formgebung an die Formgebung der Defektöffnung und von deren Umgebung, also auch der Aortenwand in jeder Richtung an. In entsprechender Weise kann die implantierbare Einrichtung sich beispielsweise auch an eine Defektöffnung im Bereich der Pulmonalis, der Wand des Vorhofseptums, des Senus cornarius, der Lungenvenen und von Klappenrändern umgebenden Strukturen, insbesondere Wandungen, anpassen.

Alternativ kann die implantierbare Einrichtung an die Formgebung an einer Implantationsstelle angepasst geformt sein. Hierdurch ist bereits ein angepasst vorgeformter, insbesondere vorgebogener oder vorgekrümmter, distaler und/oder proximaler Abschnitt in der Sekundärform der implantierbaren Einrichtung vorgesehen, so dass sich die implantierbare Einrichtung bereits in der für die Implantationsstelle geeigneten Form entfaltet werden kann. Hierdurch kann insbesondere ein Druck gegen eine Aorta vermieden werden, neben der die implantierbare Einrichtung angeordnet wird. Ein solcher Druck gegen eine Aorta kann ansonsten zu deren Perforation führen, was für einen Patienten lebensbedrohliche Folgen hätte. Die Möglichkeit, die implantierbare Einrichtung an die Formgebung der Implantationsstelle optimal anpassen zu können und diese Form bei vorteilhafter Verwendung einer Tragstruktur aus einem Formgedächtnismaterial durch Wärmebehandlung als Sekundärform einzuprägen, wird noch durch den den zurück oder rückwärts gefalteten Teilabschnitt enthaltenden, dadurch zumindest doppelt gelegten Abschnitt unterstützt, da hierdurch die eingeprägte Sekundärformgebung besonders stabil ist. Das Risiko, dass diese spezifische Formgebung verloren gehen könnte, kann aufgrund dessen im Wesentlichen ausgeschlossen werden. Die Möglichkeit einer Voreinprägung einer bestimmten Sekundärformgebung, die auch an schwierigste Situationen im Bereich einer zu verschließenden Defektöffnung angepasst werden kann, führt dazu, dass nun auch die bislang aufgrund der Gefahr einer Aortenschädigung nicht behandelbaren Patienten behandelt werden können. Bei mehr als 30% aller ASD-Patienten kann mit den bislang am Markt verfügbaren implantierbaren Einrichtungen keine Behandlung erfolgen, da das Risiko einer Schädigung der Aortenwand oder einer anderen an eine Defektöffnung angrenzenden Wandung oder Struktur zu groß ist. Da nun vorteilhaft die Sekundärform der implantierbaren Einrichtung an die Bedingungen an der Implantationsstelle angepasst werden kann, besteht die Gefahr eines Eindrückens bzw. Beschädigens der Aortenwand oder einer anderen an die zu verschließende Defektöffnung angrenzenden Wandung oder Struktur nicht mehr, so dass diese Patienten nun behandelt werden können. Aufgrund des zumindest doppelt gelegten Teilabschnitts des einen Abschnitts geht die an die Implantationsstelle angepasste Formgebung der implantierbaren Einrichtung nicht verloren, sondern bleibt vielmehr stabil bestehen.

Vorteilhaft ist zumindest ein Element als Marker zum Sichtbarmachen der implantierbaren Einrichtung unter einer Überwachungseinrichtung während eines Implantationsvorgangs vorgesehen. Das zumindest eine Element kann eine randseitig an der implantierbaren Einrichtung vorgesehene Mikrospirale sein. Eine solche Mikrospirale kann am Rand der implantierbaren Einrichtung um diesen abschnittsweise herumgewickelt sein. Besonders bevorzugt besteht das zumindest eine Element aus einem röntgensichtbaren Material, insbesondere aus einem 70 bis 90% Platin und 30 bis 10% Iridium enthaltenden Material. Selbstverständlich können auch andere Materialien verwendet werden, die ebenfalls eine Sichtbarmachung unter einer Überwachungseinrichtung erlauben.

Aufgrund der besonderen Ausbildung der erfindungsgemäßen implantierbaren Einrichtung ist diese MNR-kompatibel, führt somit bei der Überwachung eines Implantationsvorgangs nicht zu Problemen. Die implantierbare Einrichtung kann damit während der Implantation insbesondere durch bildgebende Verfahren, wie beispielsweise Röntgendarstellung, Echographie, Cintigraphie, etc., verfolgt werden. Sind, wie im Stand der Technik, Schweißstellen an Edelstahldrähten vorgesehen, entstehen bei bildgebenden Verfahren Artefakte, die zu Problemen bei der Verfolgung des Implantationsvorgangs führen.

Eine solche erfindungsgemäße implantierbare Einrichtung kann mit einem entsprechenden Abwurfsystem in einem beliebigen Winkel in einer Defektöffnung platziert werden, wobei sich die implantierbare Einrichtung dann entsprechend an den Implantationsort anpasst bzw. dort ausrichtet und sicher platziert werden kann. Die implantierbare Einrichtung kann zum Anpassen an den Implantationswinkel am Implantationsort mit einem gut abwinkelbaren Zwischenabschnitt versehen sein, insbesondere mit einem geraden und/oder ziehharmonikaartig aufgefalteten und/oder gestauchten. Auch das Vorsehen von miteinander einteilig verflochtenen Abschnitten der implantierbaren Einrichtung ist möglich, wobei die Verbindung beider Hälften oder Abschnitte der implantierbaren Einrichtung vorteilhaft in dem Zwischenabschnitt liegt, so dass ein beliebiges Krümmen dort möglich ist. Das erfindungsgemäße Abwurfsystem ist so ausgebildet, dass die implantierbare Einrichtung damit rotiert werden kann, insbesondere durch Betätigung von Haltedrähten gegenüber einem Führungs- bzw. Abwurfdraht. Vorteilhaft weist der Abwurfdraht eine sehr dünne Spitze mit einem Durchmesser von etwa 0,1 bis 0,3 mm auf. Hierdurch ist ein Durchfädeln durch die Tragstruktur der implantierbaren Einrichtung leicht möglich, sogar, wenn diese sehr engmaschig ausgebildet ist. Der Haltedraht weist zumindest eine Schlinge zum Durchführen des Abwurfdrahtes auf. Vorteilhaft kann der Haltedraht zum Befestigen an der implantierbaren Einrichtung durch endseitige Schlingen der Tragstruktur der implantierbaren Einrichtung hindurchgefädelt werden. Hierdurch ist ein besonders guter Halt an der Tragstruktur der implantierbaren Einrichtung möglich, um diese bei dem Implantationsvorgang halten und dirigieren zu können. Alternativ oder zusätzlich kann der Haltedraht durch einen endseitige Schlingen der implantierbaren Einrichtung miteinander verbindenden Tragring hindurchgefädelt werden.

Vorteilhaft kann die implantierbare Einrichtung um den zumindest einen Abwurfdraht und den zumindest einen Haltedraht herum rotiert und flotiert werden. Hierdurch ist eine exakte Positionierung auch an schwer zugänglichen Stellen in einem menschlichen oder tierischen Körper möglich.

Bei einem Verfahren zum Abwerfen einer implantierbaren Einrichtung an einer Implantationsstelle in einem menschlichen oder tierischen Körper unter Verwendung eines solchen Abwurfsystems wird zumindest ein Haltedraht durch endseitig an einer Tragstruktur der implantierbaren Einrichtung angeordnete Schlingen und/oder einen endseitig an einer Tragstruktur der implantierbaren Einrichtung angeordnete Schlingen verbindenden Tragring hindurchgefädelt und eine endseitige Schlinge an dem Haltedraht gebildet. Ferner wird ein Abwurfdraht durch die endseitige Schlinge des Haltedrahts und die implantierbare Einrichtung hindurchgefädelt und der Haltedraht dadurch an dem Abwurfdraht befestigt. Nachfolgend wird der Abwurfdraht zusammen mit der implantierbaren Einrichtung durch einen Katheter entlang dem Abwurfdraht mit dem Haltedraht zusammen an die Implantationsstelle vorgeschoben und die implantierbare Einrichtung dort entfaltet. Zum Abwerfen der implantierbaren Einrichtung wird der Abwurfdraht nach proximal zurück- und aus der endseitigen Schlinge des Haltedrahts herausgezogen und der Haltedraht aus den Schlingen der implantierbaren Einrichtung heraus- und durch den Katheter zurückgezogen. Hierbei können Abwurfdraht und Haltedraht zum Erleichtern des Vorschiebens in einem Ummantelungselement innerhalb des Katheters angeordnet werden.

Aufgrund der Möglichkeit, die implantierbare Einrichtung mit dem Abwurfsystem zu rotieren, können Löcher bzw. Defektöffnungen von sehr unterschiedlicher Formgebung exakt geschlossen werden. Daher ist auch bei einer exzentrischen Ausbildung der implantierbaren Einrichtung eine exakte Platzierung aufgrund der Rotationsfähigkeit mit dem Abwurfsystem möglich, wobei eine solche Exzentrizität sich gerade dann als vorteilhaft erweist, wenn einseitig nur wenig Platz zur Verfügung steht, wie beispielsweise im Bereich benachbart zu einer Aorta oder zu anderen Begrenzungen, beispielsweise Herzwandungen im Herzen eines Menschen oder Tieres.

Mit der vorteilhaft vorgesehenen sehr dünnen Spitze des Führungs- bzw. Abwurfdrahts mit einem Durchmesser von insbesondere 0,1 bis 0,3 mm, ist eine Flotation einer implantierbaren Einrichtung mit dem Abwurfdraht möglich. Ist die Position der implantierbaren Einrichtung nicht zufriedenstellend, kann man die implantierbare Einrichtung trotz der freien Flotation noch durch den Katheter entfernen. Ist die von der implantierbaren Einrichtung eingenommene Position hingegen zufriedenstellend, kann man diese aus dem Katheter mit dem Abwurfsystem abwerfen, ohne diese gute Position der implantierbaren Einrichtung zu verändern. Dies ist mit den Abwurfsystemen für implantierbare Einrichtungen des Standes der Technik nicht möglich. Zum Schutz gegen Verletzungen des Implantationsorts weist der Abwurfdraht vorteilhaft eine aufgerollte Spitze auf.

Obgleich vorstehend beschrieben ist, dass vorteilhaft der distale Abschnitt doppellagig mit zwei aufeinander gefalteten Teilabschnitten ausgebildet ist, ist es grundsätzlich natürlich möglich, beide Abschnitte, den proximalen und den distalen Abschnitt, in dieser Weise auszubilden. Ebenfalls ist es möglich, den mit den beiden in entsprechender Weise aufeinander gefalteten Teilabschnitten versehenen Abschnitt als proximalen Abschnitt anzuordnen.

Ferner kann die implantierbare Einrichtung zur Verwendung im menschlichen oder tierischen Körper zum Verschluss oder Teilverschluss von Defektöffnungen, Hohlräumen, Organwegen etc. oder zum Schaffen einer definierten Verbindungsöffnung zwischen Wandungen, Organen, Hohlräumen etc., mit einer Tragstruktur, die eine langgestreckte Primärform und eine demgegenüber kürzere Sekundärform aufweist, wobei die Tragstruktur einen proximalen und einen distalen Abschnitt aufweist und nach Art eines Geflechts und/oder Geleges und/oder Netzes geformt ist, versehen sein, wobei der zumindest eine Abschnitt der Tragstruktur schneckenartig aufgewickelt ist. Es können auch beide Abschnitte der Tragstruktur schneckenartig aufgewickelt sein. Durch das schneckenartige Aufwickeln wird die Tragstruktur in diesem Abschnitt noch stabiler als wenn dort nur eine Doppellagigkeit durch das Aufeinanderfalten von zwei Teilabschnitten des Abschnitts erfolgt. Der sich in der Sekundärform selbständig als Schnecke oder quer zur Längserstreckung der implantierbaren Einrichtung angeordnete Spirale konfigurierende Abschnitt ist ferner selbstverständlich ebenfalls stabiler als eine sonst im Stand der Technik bekannte Spirale, die aus einem drahtartigen Element gebildet wird, vor allem auch aufgrund des Vorsehens einer Tragstruktur in Form eines Netzes, Gewebes, Geflechts, Geleges etc. Ferner kann die implantierbare Einrichtung besonders leicht in einen dünnen Katheter eingeführt und aus diesem wieder herausgeschoben werden. Aufgrund der wiederholten Rückwärtsentfaltung, die zu der Schneckenform führt, kann die Sekundärform der implantierbaren Einrichtung sehr große Abmessungen aufweisen.

Vorteilhaft weist der zumindest eine schneckenartig aufgewickelte Abschnitt mehrere zu dem jeweils anderen Abschnitt weisende Teilabschnitte und mehrere von dem anderen Abschnitt weg weisende Teilabschnitte auf. Die Anzahl dieser aufeinander gefalteten Teilabschnitte des schneckenartigen Abschnitts kann beliebig gewählt werden. Beispielsweise können jeweils zwei zu dem anderen Abschnitt weisende und zwei von diesem weg weisende Teilabschnitte vorgesehen sein. Durch die Wahl der Anzahl der Teilabschnitte kann die Stabilität des schneckenartigen Abschnitts bestimmt werden.

Der andere Abschnitt kann die gleiche wie oder eine andere Formgebung als der schneckenartig aufgewickelte Abschnitt aufweisen. Insbesondere können beide Abschnitte schneckenartig aufgefaltete sein, wobei der eine Abschnitt auch weniger stark als der andere Abschnitt aufgefaltet sein kann.

Sind nicht beide Abschnitte schneckenartig aufgefaltet, kann der nicht schneckenartig aufgefaltete Abschnitt im Wesentlichen flach und/oder zumindest teilweise bauchig sein und/oder eine geschwungene Formgebung aufweisen. Bei einer flachen Formgebung kann der Abschnitt z.B. scheibenförmig oder als gerader Abschluss eines z.B. zylindrischen Zwischenabschnitts zwischen den beiden Abschnitten ausgebildet sein. Eine zumindest teilweise bauchige Ausbildung kann auch mit einer geschwungenen Form kombiniert werden. Umgekehrt kann eine geschwungene Formgebung ansonsten etwa scheibenförmig sein. Die beiden Abschnitte können ferner einen unterschiedlichen Durchmesser aufweisen.

Der schneckenartig gefaltete Abschnitt ist im Allgemeinen mit einer Durchgangsöffnung versehen. Der andere Abschnitt kann entweder ebenfalls mit einer Durchgangsöffnung versehen oder im Wesentlichen geschlossen ausgebildet sein. Grundsätzlich kann auf ein zusammengefasstes, also geschlossen ausgebildetes Ende auch ein Hülsenelement aufgefügt werden, wenn sich dies anwendungsspezifisch als vorteilhaft erweist.

Ferner kann in den zumindest einen schneckenartig gefalteten Abschnitt und/oder in den anderen Abschnitt zumindest ein Membranelement eingebracht oder an diesem befestigt werden. Grundsätzlich ist es ebenfalls möglich, dass beide Abschnitte schneckenartig aufgefaltet und in oder an einem oder beiden Abschnitten Membranelemente vorgesehen sind.

Des Weiteren kann die implantierbare Einrichtung zur Verwendung im menschlichen oder tierischen Körper zum Verschluss oder Teilverschluss von Defektöffnungen, Hohlräumen, Organwegen etc. oder zum Schaffen einer definierten Verbindungsöffnung zwischen Wandungen, Organen, Hohlräumen etc., mit einer Tragstruktur, die eine langgestreckte Primärform und eine demgegenüber kürzere Sekundärform aufweist, wobei die Tragstruktur einen proximalen und einen distalen Abschnitt aufweist und nach Art eines Geflechts und/oder Geleges und/oder Netzes geformt ist, versehen sein, wobei zumindest der eine Abschnitt in der Sekundärform zwei Teilabschnitte umfasst, von denen der eine sich als erster aus der Primärform in die Sekundärform entfaltet und in Richtung weg von dem anderen Abschnitt auf den zweiten zu diesem anderen Abschnitt hin gerichteten Teilabschnitt gefaltet ist. Der umgefaltete Teilabschnitt ist dabei vorteilhaft nach innen in die implantierbare Einrichtung hinein zurück gefaltet. Hierdurch wird ebenfalls eine Stabilisierung des einen Abschnitts bzw. insbesondere von dessen Rand geschaffen aufgrund des umgefalteten Teilabschnitts. Gegenüber dem Zurückfalten des einen Teilabschnitts nach außen, wie dies vorstehend beschrieben ist, kann ein nach innen eingefalteter Teilabschnitt nicht oder zumindest kaum wieder in die Primärform zurückgefaltet werden, wenn die implantierbare Einrichtung nach ihrem Entfalten ggf. wieder in einen Katheter hineingezogen werden soll. Ein Entfernen der einmal abgeworfenen implantierbaren Einrichtung von der Implantationsstelle erweist sich daher mit dieser Ausführungsform als schwierig.

Bei der Verwendung des Einfaltens des einen Teilabschnitts nach innen können gerade lange Geflechte oder Netze der Tragstruktur besonders gut endseitig verstärkt werden. Hierbei eignet sich eine maschinelle Herstellung besonders, da mit dieser gerade lange Gebilde einfacher als mit der Hand gefertigt werden können. Sehr komplex aufgebaute kürzere implantierbare Einrichtungen können hingegen zumeist effektiver mit der Hand gefertigt werden.

Die erfindungsgemäße implantierbare Einrichtung kann besonders vorteilhaft zum Verschluss eines Atrium-Septum-Defekts (ASD), Ventrikel-Septum-Defekts (VSD), persistent Foramen ovale (PFO), eines strukturellen Vorkammerdefekts des Herzens, eines Left Appendix, als vascular plug, also peripheres Gefäßverschlusssystem für periphere Flusssysteme, zur Behandlung von Aortenaneurysmen sowie im persistierenden ductus arteriosus botalli (PDA) verwendet werden. Insbesondere kann durch die Verwendung der Doppellagigkeit des zumindest einen Abschnitts der implantierbaren Einrichtung eine solche Stabilität geschaffen werden, dass auch große Ducten bzw. Öffnungen mittels dieser Einrichtung verschlossen werden können, die insbesondere größer als 10 bis 12 F sind.

Zur Verwendung der implantierbaren Einrichtung bei einem peristierenden ductus arteriosus erweist es sich als vorteilhaft, die implantierbare Einrichtung hutförmig mit zumindest einem doppellagigen hutkrempenförmigen Abschnitt auszubilden. Durch diese Hutform kann der kopfteilförmige Abschnitt der implantierbaren Einrichtung in den persitierenden ductus arteriosus zu dessen Verschluss eingefügt werden. Der hutkrempenförmige Abschnitt legt sich an der Aortenwand an, so dass die Aorta frei bleibt.

Als weiter vorteilhaft erweist es sich, wenn der doppellagige hutkrempenförmige Abschnitt als zumindest zwei aufeinander gefaltete Teilabschnitte aufweisender Abschnitt ausgebildet ist. Hierdurch weist dieser Abschnitt zum Abstützen an der Aortenwand eine besondere Stabilität auf, so dass ein besonders sichererer Sitz möglich ist. Vorzugsweise wird der sich an der Aortenwand abstützende hutkrempenförmige Abschnitt entsprechend der Krümmung der Aorta geformt, so dass er sich besonders gut dort anlagern kann. Ferner ist es jedoch aufgrund der aufeinander gefalteten Teilabschnitte des hutkrempenförmigen Abschnitts möglich, dass dieser sich selbständig an die Formgebung der Aorta anpasst und dort sicher festhält. Aufgrund der aufeinander rückwärts oder zurück gefalteten Teilabschnitte des Abschnitts und der sonstigen Formgebung als Hut ist es möglich, dass Löcher mit höchst unterschiedlichem Durchmesser von ein und derselben implantierbaren Einrichtung verschlossen werden können. Beispielsweise kann ein Loch mit einem Durchmesser von 8 mm mit derselben hutförmigen implantierbaren Einrichtung dicht verschlossen werden wie ein Loch mit einem Durchmesser von weniger als 3 mm. Die Flexibilität der Verwendung einer solchen hutförmigen implantierbaren Einrichtung ist daher sehr groß, ebenso ihre spezielle Ausbildung der Hutform.

Der dem hutkrempenförmigen Abschnitt gegenüberliegende kopfteilförmige Abschnitt der implantierbaren Einrichtung kann insbesondere nach innen in das Innere der implantierbaren Einrichtung eingestülpt sein. Hierdurch kann dieser Abschnitt geschlossen und ohne herausragendes Ende ausgebildet werden, so dass es nicht nur leichter ist, die Form des Abschnitts stabil aufrechtzuerhalten, sondern auch keine ggf. verletzenden Enden aus der implantierbaren Einrichtung an diesem Ende herausragen.

Alternativ kann der dem hutkrempenförmigen Abschnitt gegenüberliegende kopfteilförmige Abschnitt der implantierbaren Einrichtung nach außen aus der implantierbaren Einrichtung ausgestülpt sein, so dass eine Tropfenform des kopfteilförmigen Abschnitts gebildet ist. Diese Form ist zum Einfügen in tubuläre Gefäße besonders geeignet.

Vorteilhaft kann sich bei dem erfindungsgemäßen Verfahren zum Entfalten der implantierbaren Einrichtung der sich rückwärts in Richtung zu dem Katheter faltende Teilabschnitt im Wesentlichen flach auf den anderen Teilabschnitt auffalten. Ferner kann sich, wie bereits erwähnt, der sich rückwärts in Richtung zu dem Katheter faltende Teilabschnitt schneckenartig um sich selbst herum aufwickeln. Auch die Ausbildung der anderen vorstehend genannten Formgebungen der implantierbaren Einrichtung können als Sekundärformen beim Herausschieben der implantierbaren Einrichtung aus dem Katheter entfaltet werden. Ferner ist eine ballonartige oder ankerförmig auskragende Formgebung des zumindest einen Abschnitts der implantierbaren Einrichtung möglich, insbesondere mit einem nach innen in die implantierbare Einrichtung hinein umgefalteten Teilabschnitt. Außerdem kann der zumindest eine Abschnitt der implantierbaren Einrichtung im Wesentlichen flach und/oder zumindest teilweise bauchig konvex oder konkav ausgebildet sein und/oder eine geschwungene konvex und/oder konkave Formgebung aufweisen. Auch Mischformen der vorstehend genannten Formgebungen sind möglich und können ebenso wie die Abmessungen der einzelnen Abschnitte der implantierbaren Einrichtung anwendungsspezifisch ausgewählt werden.

Als vorteilhaft erweist es sich, wenn zumindest eine der Oberflächen der implantierbaren Einrichtung, insbesondere der Tragstruktur und/oder des Membranelements, mit zumindest einem Funktionsmaterial beschichtet oder behandelt ist. Hierdurch kann vor allem die Wirksamkeit der implantierbaren Einrichtung noch weiter verbessert werden, da einerseits negative Reaktionen des Körpers auf die implantierbare Einrichtung vermieden und andererseits durch Medikamentierung die Umgebung der Implantationsstelle direkt behandelt werden kann. Insbesondere kann das für die Beschichtung oder Behandlung des für die implantierbare Einrichtung verwendeten Materials bzw. der Oberflächen der implantierbaren Einrichtung verwendete Funktionsmaterial ausgewählt sein aus der Gruppe bestehend aus anorganischen Materialien, keramischen Materialien, synthetischen Polymeren, humanen Biopolymeren, Medikamentenbeschichtungen, medikamentenfreisetzenden Polymeren, Biomolekülen, funktionalen Gruppen, genetischen Materialien. Es können hierbei ein oder mehrere Materialien verwendet und miteinander kombiniert werden, insbesondere, um eine Kombination von verschiedenen Wirkungen bzw. Funktionen zu erhalten.

Besonders bevorzugt ist das Funktionsmaterial ausgewählt aus der Gruppe bestehend aus Gold, Biogold, diamantartigem Carbon (DLC), diamantartigem Nanocomposit (DLN), Iridiumoxid, nanoporösem AL₂O₃, Siliciumkarbid, Hydroxylapatit, Titannitritoxid, Poly(2-chlor-p-xylylen), Polybutylmethacrylat, Phosphorylcholin, Polyethylen, Polyethylen Vinylacetat, Polyhexylmethacrylat, Poly[bis](trifluoroethoxy)phosphazen, Polytetrafluorethylen, Polyurethan, Collagen, Chondroitinsulfat, Elastin, Fibrin, Hydraluronsäure, Abciximab, Heparin, Paclitaxel, Abciximab freisetzender Cellulose, Angiopeptin freisetzendem Phosphorylcholin, DNA freisetzendem Polyethylenvinylacetat oder Polybutylmethacrylat, ein Medikament freisetzendem Poly(L-lactid), Paclitaxel oder Hirudin oder Iloprost freisetzendem Polylactid, viral vector freisetzendem Polylactid-Phosphorylcholin, Forskolin freisetzendem Polyurethan, Stammzellen. Durch Iridiumoxid kann dabei z.B. eine Oberflächenpassivierung bzw. eine anti-oxidative Beschichtung vorgesehen werden. Polyurethan kann z.B. als Dünnschicht aufgetragen werden. Funktionalisierte Oberflächen mit Biomolekülen und funktionalen Gruppen können ferner im Rahmen des sog. Molecular Surface Engineering vorgesehen werden, also der Bildung von molekularen Materialoberflächen durch Einbringen funtioneller Gruppen. Zudem können die Oberflächen der implantierbaren Einrichtung z.B. gezielt für die Adhesion im Blut zirkulierender Progenitorzellen mit Zell-Ligandstrukturen ausgestattet werden. Poly(L-lactid) kann mit einer Vielzahl von Medikamenten kombiniert werden, insbesondere mit Medikamenten aus der Gruppe der Zystostatika, wobei jede Art von Medikament, das zu einem jeweils gewünschten Therapieerfolg führt, verwendet werden kann. Zum Verschluss z.B. eines strukturellen Defekts im Herzen eines Patienten bzw. eines strukturellen Herzfehlers, wie eines PDA, können die mit den Polymeren kombinierbaren Medikamente oder Substanzen insbesondere Substanzen zur Beschleunigung der Endothelisierung, also des Bewuchses mit körpereigenen Zellen sein.

Für die Oberflächenbehandlung der implantierbaren Einrichtung können verschiedene Verfahren verwendet werden, wie z.B. eine Plasmabehandlung , gerade bei Auftrag von DLC (diamantartigem Kohlenstoff) oder DLN (einem diamantartigen Nanocomposit), ein PVD (Physical Vapour Deposition)-Verfahren, ein CVD (Chemical Vapour Deposition)- Verfahren, Ionenimplantation, Sputtern, ein lonenstrahlverfahren, ein Laserverfahren, ein thermisches Verfahren, Spin Coating, Dip Coating, Ätzen, Elektropolieren.

Zur näheren Erläuterung werden im Folgenden Ausführungsbeispiele näher anhand der Zeichnungen beschrieben. Diese zeigen in:
- Figur 1A: eine Draufsicht einer ersten Ausführungsform einer implantierbaren Einrichtung,
- Figur 1 B eine: Draufsicht auf eine zweite Ausführungsform einer implantierbaren Einrichtung
- Figur 1C bis 1F: Detailansichten der implantierbaren Einrichtung gemäß Figur 1B,
- Figur 1G und 1H: skizzenhafte seitliche Schnittansichten durch eine Wandung, in der die implantierbare Einrichtung gemäß Figur 1A angeordnet ist,
- Figur 1J: eine Draufsicht auf eine dritte Ausführungsform einer implantierbaren Einrichtung mit einer großformatigen Durchgangsöffnung,
- Figur 1K: eine perspektivische Ansicht einer vierten Ausführungsform einer hutförmigen implantierbaren Einrichtung,
- Figuren 2A bis 2C: Detailansichten eines ein-, zwei und eines dreilagigen Strukturaufbaus der Tragstruktur einer implantierbaren Einrichtung,
- Figur 2D, 3 und 4: eine Detailansicht eines einlagigen Strukturaufbaus der Tragstruktur einer implantierbaren Einrichtung mit verflochtenen Elementpaaren,
- Figuren 5 bis 13: Detailansichten verschiedener Flechtarten zur Bildung der Tragstruktur einer implantierbaren Einrichtung,
- Figuren 14 bis 16: eine Draufsicht auf eine weitere Ausführungsform einer implantierbaren Einrichtung sowie von Detailansichten des zentralen Bereichs und eines Randbereichs von dieser,
- Figur 17: eine Detailansicht eines rückwärts gefalteten Abschnitts.
- Figuren 18, 21, 22, 23: eine Draufsicht auf eine weitere Ausführungsform einer implantierbaren Einrichtung sowie von Detailansichten des zentralen Bereichs und zweier Randbereiche von dieser,
- Figuren 19 und 20: eine Draufsicht auf eine Vorder- und eine Rückseite einer weiteren Ausführungsform einer implantierbaren Einrichtung,
- Figuren 24A und 24B: skizzenhafte Seitenansichten weiterer Ausführungsformen einer implantierbaren Einrichtung,
- Figuren 25 bis 29: eine Draufsicht auf eine weitere Ausführungsform einer implantierbaren Einrichtung sowie von Detailansichten des zentralen Bereichs, zweier Randbereiche und eines dazwischen angeordneten Bereichs,
- Figuren 30 bis 34: Seitenansichten verschiedener Möglichkeiten einer Anordnung eines Membranelements in einer implantierbaren Einrichtung,
- Figuren 35 bis 42B: seitliche Prinzipskizzen eines Katheters mit implantierbarer Einrichtung während eines Abwurfvorgangs der implantierbaren Einrichtung an einer Implantationsstelle,
- Figuren 43 bis 58: perspektivische Ansichten eines Abwurfvorgangs einer implantierbaren Einrichtung aus einem Katheter,
- Figur 59: eine Draufsicht auf eine Ausführungsform einer implantierbaren Einrichtung mit einem Membranelement,
- Figur 60: eine Draufsicht auf eine weitere Ausführungsform einer implantierbaren Einrichtung mit einer anderen Anordnung eines Membranelements,
- Figuren 61 bis 64: perspektivische Ansichten von an eine Implantationsstelle angepassten implantierbaren Einrichtungen,
- Figur 65: eine perspektivische Ansicht einer weiteren Ausführungsform einer an eine Implantationsstelle angepasst geformten implantierbaren Einrichtung,
- Figur 66: eine skizzenhafte Draufsicht auf eine Aorta und eine implantierbare Einrichtung in Anordnung an der Aorta,
- Figuren 67 und 68: skizzenhafte Seitenansichten neben einer Aorta angeordneter implantierbarer Einrichtungen,
- Figuren 69 bis 74: skizzenhafte Schnittansichten weiterer Ausführungsformen von implantierbaren Einrichtungen mit schneckenartig mehrfach rückwärts gefalteten Abschnitten,
- Figur 75: eine perspektivische Ansicht einer weiteren Ausführungsform einer implantierbaren Einrichtung in Form eines Hutes mit einem auskragenden hutkrempenartigen Abschnitt und einem diesem gegenüberliegend angeordneten auskragenden Abschnitt,
- Figur 76: eine perspektivische Ansicht einer weiteren Ausführungsform einer hutförmigen implantierbaren Einrichtung mit einem im Wesentlichen zylindrischen Kopfteil-Abschnitt,
- Figur 77: eine perspektivische Ansicht einer implantierbaren Einrichtung mit einem scheibenförmigen und einem bauchigen Abschnitt in Anbringung an einem Abwurfdraht,
- Figuren 78 bis 81: perspektivische Ansichten eines Auffädelns von endseitigen Schlingen einer implantierbaren Einrichtung auf ein erfindungsgemäßes Abwurfsystem,
- Figur 82: eine Draufsicht auf eine endseitige Schlinge gemäß Figur 78,
- Figuren 83 bis 85: Draufsichten auf endseitige Schlingen einer implantierbaren Einrichtung,
- Figuren 86 bis 88: Prinzipskizzen der Positionierung eines Abwurfsystems für eine implantierbare Einrichtung,
- Figur 89: eine perspektivische Prinzipskizze einer mit einem Abwurfsystem in Form eines Haltedrahts und eines Abwurfdrahts versehenen implantierbaren Einrichtung,
- Figur 90 bis 101: perspektivische Ansichten eines Herausschiebens einer Ausführungsform einer erfindungsgemäßen implantierbaren Einrichtung aus einem Katheter,
- Figur 102 bis 105: Prinzipskizzen einer weiteren Ausführungsform einer sich entfaltenden erfindungsgemäßen stenförmigen implantierbaren Einrichtung,
- Figur 106: eine perspektivische Ansicht einer stentförmigen implantierbaren Einrichtung mit auskragendem Ende,
- Figur 107 bis 111: Detailansichten zentraler Bereiche einer implantierbaren Einrichtung mit langen Beinchen,
- Figuren 112, 113: perspektivische Ansichten zweier implantierbarer Einrichtungen mit langen Beinchen im zentralen Bereich,
- Figur 114 bis 119: Prinzipskizzen der Anordnung von Membranelementen an der implantierbaren Einrichtung nach Figur 112, 113,
- Figuren 120, 121: Detailansicht des zentralen Bereichs der einer implantierbaren Einrichtung mit zwei Tragringen für Schlingen,
- Figur 122: eine Prinzipskizze als seitliche Querschnittsansicht einer implantierbaren Einrichtung auf einem Abwurfdraht mit gerollter Spitze (pigtail),
- Figur 123 bis 125: Prinzipskizzen eines gekrümmten Schleusenkatheters zum Zuführen der implantierbaren Einrichtung nach Figur 122 an eine Implantationsstelle,
- Figur 126: eine Platziervorrichtung mit implantierbarer Einrichtung,
- Figur 127A bis 127C: eine Skizze eines Patienten mit vergrößert dargestelltem Herzen mit einem Shunt in der Wand zwischen rechter und linker Herzkammer,
- Figur 128: das Detail eines Schleusenkatheters,
- Figuren 129, 130: eine perspektivische und eine Detailansicht einer stentförmigen implantierbaren Einrichtung mit Polyesterfasern,
- Figur 131 bis 133: eine perspektivische Ansicht und Querschnitts-Prinzipskizzen einer Variante der Ausführungsform der implantierbaren Einrichtung nach Figur 75,
- Figur 134 bis 137A: verschiedene Prinzipskizzen von implantierbaren Einrichtungen zum Verschluss eines Left Appendix,
- Figuren 137B, 137C: Detailansichten des Anschlussbereichs zweier Hälften einer der implantierbaren Einrichtung nach Figur 137A,
- Figur 138 bis 140: Prinzipskizzen von Varianten der implantierbaren Einrichtungen nach Figuren 134 bis 136,
- Figur 141 bis 148B: Prinzipskizzen von weiteren Ausführungsformen implantierbarer Einrichtungen zum Verschluss eines Left Appendix, und
- Figur 149 bis 172: perspektivische Ansichten verschiedener Ausführungsvarianten einer hutförmigen implantierbaren Einrichtung.

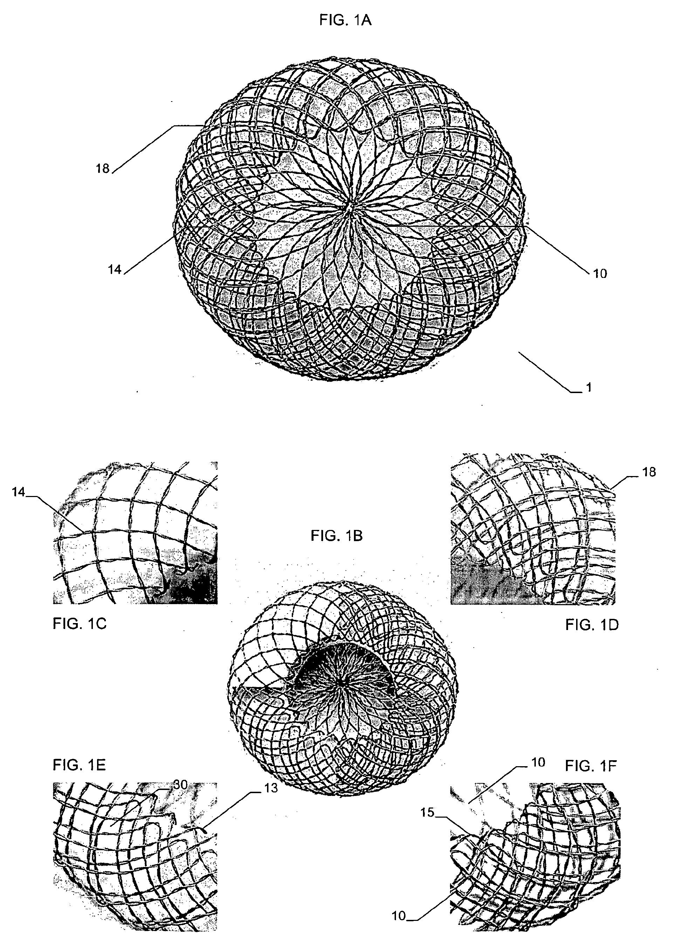

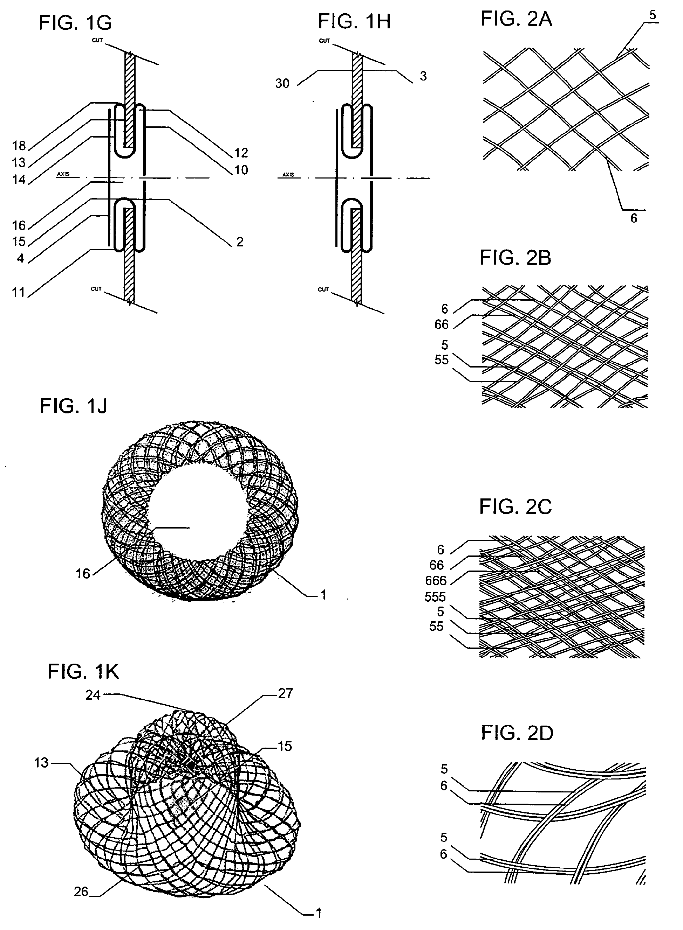

Figur 1A zeigt eine Draufsicht auf eine implantierbare Einrichtung 1 . Wie besser den Seitenansichten der implantierbaren Einrichtung in den Figuren 1G und 1H zu entnehmen ist, bei denen die implantierbare Einrichtung 1 in einer Öffnung 2 einer Wandung 3, z.B. eines Herzens eines Menschen oder Tieres angeordnet ist, wobei sich die Figuren 1G und 1H lediglich dadurch unterscheiden, dass der besseren Übersichtlichkeit halber die die Wandung 3 betreffenden Bezugszeichen in Figur 1H gezeigt sind, weist die implantierbare Einrichtung einen proximalen Abschnitt 10 und einen distalen Abschnitt 11 auf. Der proximale Abschnitt 10 ist einen Innenraum 12 umgebend geformt, wohingegen der distale Abschnitt 11 doppellagig durch zwei aufeinander gefaltete Teilabschnitte 13, 14 gebildet ist. Der eine Teilabschnitt 13 ist in Richtung zu dem proximalen Abschnitt 10 zurückgebogen und lagert an der Außenseite 30 der Wandung 3 an, wohingegen der andere Teilabschnitt 14 distal außenseitig angeordnet und mit einem Zwischenabschnitt 15 verbunden ist, der den proximalen und den distalen Abschnitt 10,11 miteinander verbindet und in der Öffnung 2 in der Wandung 3 angeordnet ist. Ferner ist außenseitig auf dem distalen Teilabschnitt 14 ein Membranelement 4 aufgebracht. Dieses verschließt eine Öffnung 16, die von dem Teilabschnitt 14 innenseitig umgrenzt wird und durch den Zwischenabschnit hindurchgeht. Hierdurch ist auch der distale Abschnitt 11 nach distal geschlossen, ebenso wie der proximale Abschnitt, der dies aufgrund seiner im Wesentlichen geschlossenen Formgebung bereits ist.

Die implantierbare Einrichtung 1 ist aus einer Tragstruktur aufgebaut, die in Figur 1 A sowie den Figuren 1 B bis 1 F besonders gut zu erkennen ist. Diese kann verschiedenste Ausführungsformen aufweisen und z.B. als Netz, Geflecht, Gelege oder Gewebe ausgebildet sein. Die Figur 1 B zeigt eine implantierbare Einrichtung 1 mit vier verschiedenen Tragstrukturaufbauten. Der in Figur 1C als Detail gezeigte Aufbau in der linken oberen Ecke in Figur 1 B zeigt eine einlagige Tragstruktur in Form eines Gewebes. Dieses ist durch Verweben von jeweils zwei miteinander verdrillten drahtartigen Elementen 5, 6 gebildet. Der in Figur 1 E gezeigte Aufbau ist zweilagig, ebenfalls gebildet durch Verweben zweier miteinander verdrillter drahtartiger Elemente 5, 6. Der in Figur 1 D gezeigte Aufbau ist dreilagig und der in Figur 1 F gezeigte Aufbau ist vierlagig, beide ebenfalls gebildet durch Verweben zweier miteinander verdrillter drahtartiger Elemente 5, 6. Die in Figur 1A gezeigte Ausführungsform der implantierbaren Einrichtung weist eine vierlagige Tragstruktur auf, so dass diese im Bereich der beiden aufeinander gefalteten Teilabschnitte 13, 14 besonders dicht ist. Ein umlaufender äußerer Rand 18 der implantierbaren Einrichtung ist in den Ausführungsformen der Figuren 1 A bis 1 F gleichmäßig gebogen ausgebildet.

In Figur 1 J ist eine weitere Ausführungsform einer implantierbaren Einrichtung 1 gezeigt, bei der im Unterschied zu den Ausführungsformen der Figuren 1A bis 1H eine große Durchgangsöffnung 16 durch die implantierbare Einrichtung hindurch vorgesehen ist. Mit einer solchen großen Durchgangsöffnung kann beispielsweise eine definierte Durchgangsöffnung bei einem Teilverschluss einer Wandung oder eines Organwegs geschaffen werden.

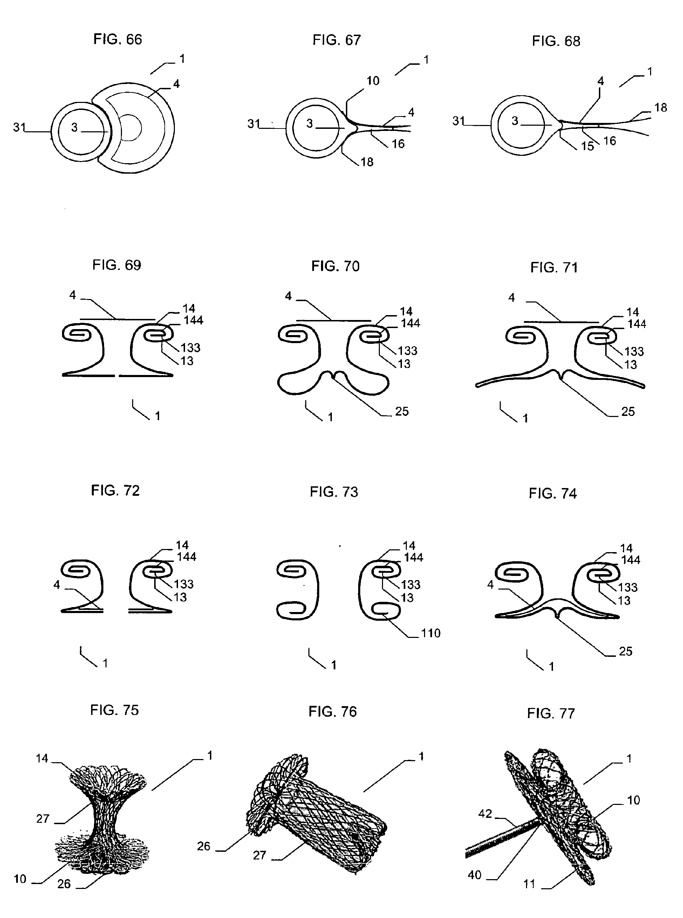

Die Figur 1 K zeigt eine Ausführungsform einer implantierbaren Einrichtung 1, die besonders für die Verwendung einer Behandlung eines persitierenden durctus arteriosus (PDA) entworfen ist. Entsprechende Ausführungsformen zum Verschluss eines solchen PDA sind auch den Figuren 75 und 76 zu entnehmen, auf die weiter unten noch näher eingegangen werden soll. Alle diese Ausführungsformen weisen annähernd eine Hutform auf.

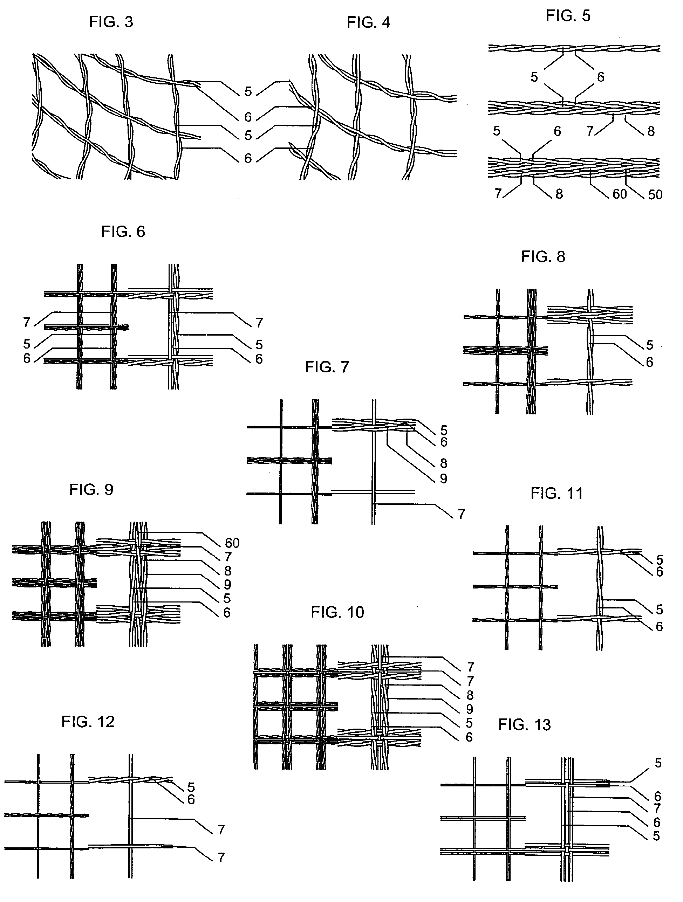

Den Figuren 2A bis 13 sind Beispiele diverser Varianten des Verwebens bzw. Vernetzens von drahtartigen Elementen zu entnehmen, wobei diese nur beispielhaft zeigen, welche Formen grundsätzlich möglich sind. Selbstverständlich können noch zahlreiche weitere Varianten und Kombinationen der dargestellten Formen gebildet werden. Figur 2A zeigt ein einlagiges Gewebe aus zwei drahtartigen Elementen 5, 6. Figur 2B zeigt ein doppellagiges Gewebe aus in der einen Lage drahtartigen Elementen 5, 6 und in der anderen Lage drahtartigen Elementen 55, 66. Figur 2C zeigt ein dreilagiges Gewebe aus in der einen Lage drahtartigen Elementen 5, 6, in der zweiten Lage drahtartigen Elementen 55, 66 und in der dritten Lage drahtartigen Elementen 555, 666. Figur 2D zeigt ein einlagiges Gewebe von jeweils zwei parallel nebeneinander laufenden drahtartigen Elementen 5, 6. Figuren 3 und 4 zeigen Varianten, bei denen die beiden parallel zueinander laufenden drahtartigen Elemente 5, 6 miteinander verdrillt und die jeweiligen verdrillten Paare von drahtartigen Elementen 5, 6 wiederum miteinander verflochten sind. Unterschiedlich ist in diesen beiden Ausführungsformen lediglich die Art der Durchflechtung der einzelnen Paare untereinander. In der Ausführungsform nach Figur 3 sind jeweils die verdrillten Paare zusammen zwischen den verdrillten anderen Paaren von drahtartigen Elementen hindurchgeführt, in der Ausführungsform in Figur 4 sind die einzelnen drahtartigen Elemente 5, 6 an den Kreuzungspunkten aneinander vorbei geführt.

Figur 5 zeigt weitere Möglichkeiten, wie einzelne drahtartige Elemente miteinander verflochten werden können, wobei im ersten Fall zwei drahtartige Elemente 5, 6, im zweiten Fall vier drahtartige Elemente 5, 6, 7, 8 und im dritten Fall sechsdrahtartige Elemente 5, 6, 7, 8, 9, 50, 60 miteinander verflochten sind.

Figur 6 zeigt eine Verflechtungs- bzw. Netzvariante, bei der einzelne glatte drahtartige Elemente 7 mit verflochtenen Paaren von drahtartigen Elementen 5, 6 verflochten bzw. verwoben sind. Figur 7 zeigt eine Variante, bei der nur ein einzelnes glattes drahtartiges Element 7 wechselweise mit einem entsprechend glatten drahtartigen Element 7 und mit einem Geflecht aus vier drahtartigen Elementen 5, 6, 8, 9 verflochten bzw. verwoben ist. Bei Figur 8 ist es eine Kombination aus einem Geflecht aus sechs drahtartigen Elementen und einem Paar drahtartiger Elemente 5, 6 im Wechsel angeordnet und mit einem Paar verdrillter drahtartiger Elemente verflochten. Figuren 9 und 10 zeigen Kombinationen aus jeweils fünf bzw. sechs miteinander verflochtenen drahtartigen Elementen 5, 6, 7, 8, 9, 60 die untereinander wiederum verflochten bzw. verwoben sind. Figur 11 zeigt ein Geflecht aus jeweils einem verdrillten Paar von drahtartigen Elementen 5, 6, Figur 12 zeigt ein Geflecht aus wechselweise einem glatten drahtartigen Element 7 und einem verdrillten Paar drahtartiger Elemente 5, 6. Figur 13 zeigt ein Geflecht aus drei glatten nicht untereinander verflochtenen drahtartigen Elementen 5, 6, 7 und abwechselnd entsprechend drei glatten drahtartigen Elementen und nur zwei glatten drahtartigen Elementen 5, 6. Die einzelnen drahtartigen Elemente können eine runde, flache oder beliebig anderweitig geformte Querschnittform aufweisen.

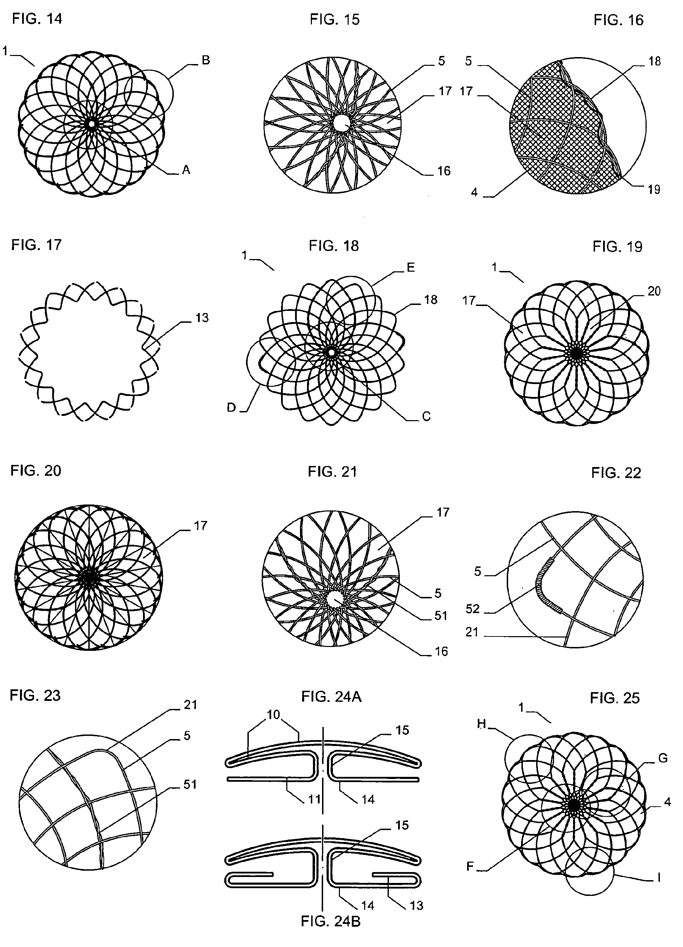

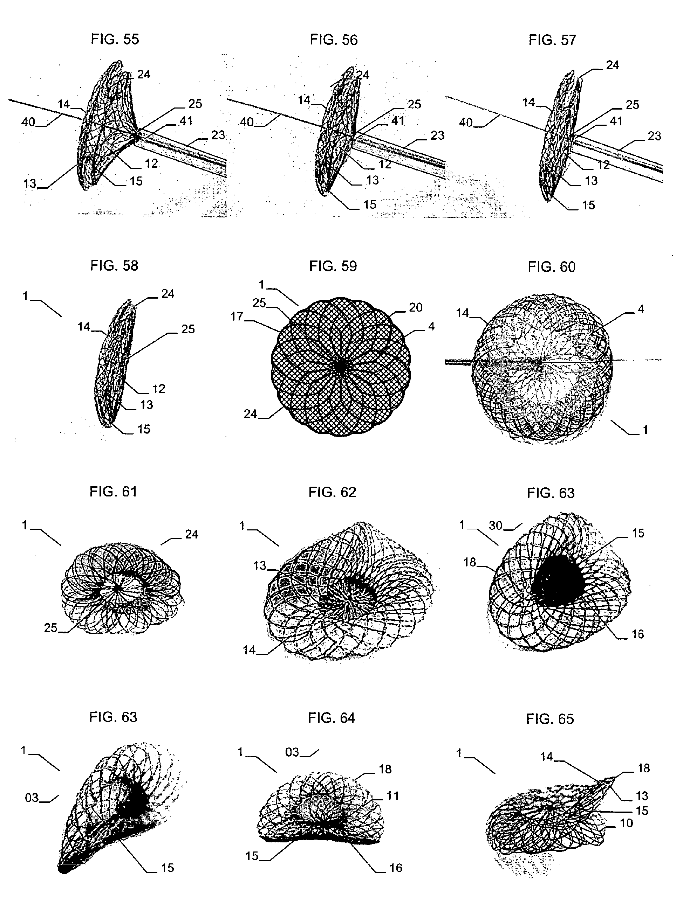

In Figur 14 ist eine Ausführungsform einer implantierbaren Einrichtung 1 skizziert, die eine regelmäßige konzentrische Form aufweist. Grundsätzlich ist es ebenfalls möglich, die implantierbare Einrichtung mit einer exzentrischen Form zu versehen, wenn diese beispielsweise an einer Stelle im Körper eines Patienten implantiert werden soll, die einseitig begrenzt ist, z.B. durch eine Wandung oder eine Aorta oder in anderer Form. Auf derartige Ausführungsformen wird zu den Figuren 61 bis 68 noch eingegangen. In Figur 14 sind zwei Detailausschnitte A, B der implantierbaren Einrichtung 1 markiert, die in den Detailansichten der Figuren 15 (Detail A) und 16 (Detail B) dargestellt sind. Hierbei wird aus Figur 15, die das Zentrum der implantierbaren Einrichtung zeigt, die kleine zentrale Durchgangsöffnung 16 deutlich, die sich durch den distalen Abschnitt, den Zwischenabschnitt und den proximalen Abschnitt hindurch erstreckt. Die Tragstruktur 17 ist hier als regelmäßiges Geflecht aus einem drahtartigen Element 5 gebildet. Das in Figur 16 gezeigte Detail B des Randes der implantierbaren Einrichtung 1 zeigt, dass dieser äußere Rand 18 mit einem zusätzlichen Element 19 umwickelt ist, das zum Befestigen des Membranelements 4 dient. Das Element 19 kann daher beispielsweise als Faden, z.B. aus Prolene, einem herzchirurgischen Nahtmaterial, oder als Drahtelement ausgebildet sein. Ferner kann ein zusätzliches Element als Marker zur Kenntlichmachung der implantierbaren Einrichtung unter einer Überwachungseinrichtung während eines Implantationsvorgangs vorgesehen sein, wie weiter unten noch beschrieben wird. Dieser äußere Rand 18 der implantierbaren Einrichtung ist der Bereich, in dem der nach distal weisende Teilabschnitt 14 und der in Richtung proximal weisende Teilabschnitt 13 des distalen Abschnitts aneinander angrenzen bzw. ineinander übergehen. Dies ist auch Figur 1G und 1H zu entnehmen.

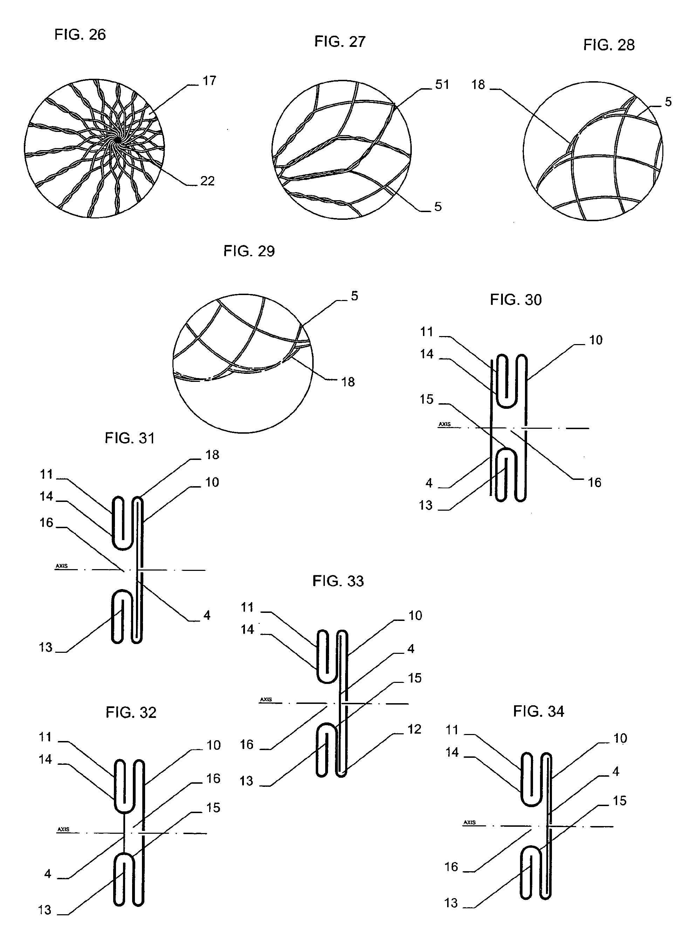

Die Tragstruktur 17 der implantierbaren Einrichtung 1 kann so dicht ausgebildet sein, dass diese bereits eine Art Membran bildet. Ist dies jedoch nicht der Fall oder ein völlig dichter Verschluss einer Öffnung, wie einer Defektöffnung im Herzen eines Patienten, erwünscht, kann das zusätzliche Membranelement 4 vorgesehen sein, das in Figur 16 unter dem Geflecht oder Gewebe der Tragstruktur beispielhaft skizziert ist. Wie den Figuren 30 bis 34 zu entnehmen ist, kann das Membranelement 4 nicht nur distal auf dem dortigen Teilabschnitt 14 des distalen Abschnitts 11 der implantierbaren Einrichtung 1 aufgefügt sein, wie in Figur 1 bzw. auch Figur 30 gezeigt, sondern kann auch an anderen Stellen innerhalb und außenseitig auf der implantierbaren Einrichtung vorgesehen sein. Die in Figur 32 gezeigte Variante eines sehr klein ausgebildeten Membranelements 4 ist innerhalb der Öffnung 16 des Zwischenabschnitts 15 angeordnet. Je kleiner ein Membranelement ausgebildet ist, desto geringer ist das Thromboserisiko, da um die zu verschließende Öffnung 2 in der Wandung 3 herum nur das Netz der Tragstruktur 17, also nur deren Material, vorliegt und nicht eine Kombination aus dem Material des Membranelements und der Tragstruktur, was ansonsten das Thromboserisiko erhöht und ggf. zu Kopfschmerzen und vorübergehender Blindheit aufgrund der entstehenden und wandernden Embolie führen kann. Diese Komplikationen können hier sicher vermieden werden. Dasselbe gilt auch für die übrigen Anordnungsvarianten in den Figuren 31, 33, und 34, wobei in Figur 31 das Membranelement in dem Innenraum des proximalen Anschnitts 10 angeordnet und beispielsweise an dem umlaufenden Rand 18 auf dessen Innenseite befestigt ist. In Figur 33 ist das Membranelement in dem Innenraum 12 des proximalen Abschnitts 10 anliegend an dem distalen Teil des proximalen Abschnitts 10 angeordnet und dort befestigt, der in den Zwischenabschnitt übergeht und bei einer Anordnung der implantierbaren Einrichtung in einem Loch in einer Wandung an der Wandung anliegt. In Figur 34 ist das Membranelement im Innenraum des proximalen Abschnitts 10 auf dessen proximaler Innenseite vorgesehen. Das Membranelement kann z.B. aus einem Kunststoffmaterial, wie einem Polymer, bestehen.

In Figur 17 ist das in den Figuren 14 und 16 nicht sichtbare Detail des zurück- oder rückwärts gefalteten Teilabschnitts 13 des distalen Abschnitts 11 gezeigt. Dieses ist hier vergleichsweise schmal ausgebildet. Es kann ebenso breiter ausgebildet werden und sich sogar bis an das Zentrum der implantierbaren Einrichtung heran erstrecken.

Die Figuren 18 und 21 bis 23 zeigen eine gegenüber der Ausführungsform der implantierbaren Einrichtung 1 in Figur 14 modifizierte Ausführungsform mit einer anderen Ausbildung des umlaufenden äußeren Randes 18. Figur 18 zeigt dabei eine Draufsicht auf den proximalen Abschnitt 10, Figur 21 eine Detailansicht des Zentrums der implantierbaren Einrichtung mit der kleinen Durchgangsöffnung 16 und zwei Enden 51 des drahtartigen Elements 5, die zentral in der Tragstruktur 17 mit einem anderen Abschnitt des drahtartigen Elements 5 verflochten sind.

Wie der Detailansicht des Details D nach Figur 18 in Figur 22 entnommen werden kann, ist anstelle eines Umwickelns des äußeren Randes 18 des proximalen Abschnitts 10 hier lediglich partiell ein Umwickeln von Schlaufen der Tragstruktur mit einem drahtartigen Element 52 oder ähnlichem vorgesehen. Dies dient als Marker zum Sichtbarmachen der implantierbaren Einrichtung z.B. unter einem Röntgenschirm. Zu diesem Zweck ist das drahtartige Element 52 beispielsweise, wie dargestellt, als Mikrospirale aus einem Platin und Iridium enthaltenden Material hergestellt, beispielsweise mit einem Anteil von 70 bis 90% Platin und 30 bis 10% Iridium. Wie der Detailansicht des Details E aus Figur 18 in Figur 23 zu entnehmen ist, ist auch diese implantierbare Einrichtung 1 aus einem einzigen drahtartigen Element gebildet. Dessen Ende 51 ist hier im Bereich des Randes 21 verflochten, um ein ungewolltes Lösen von diesem zu vermeiden.

Figur 19 eine frontseitige Ansicht des proximalen Abschnitts 10 und Figur 20 eine Draufsicht auf den distalen Abschnitt 11 einer weiteren Ausführungsform der implantierbaren Einrichtung 1. Hierbei wird deutlich, dass in dieser Ausführungsform der proximale Abschnitt 10 mit einem rückwärts in Richtung zu dem distalen Abschnitt 11 gefalteten Teilabschnitt und einem nach proximal weisenden Teilabschnitt versehen ist, also einen dem Aufbau des distalen Abschnitts gemäß Figur 1 entsprechenden Aufbau aufweist. Die Tragstruktur 17 ist dichter als beispielsweise bei der Ausführungsform nach Figur 14. Dies ist insbesondere auch im zentralen Bereich sichtbar, da dort im Wesentlichen keine Durchgangsöffnung in dem proximalen Abschnitt 10 belassen ist. Die einzelnen regelmäßig angeordneten Segmente der Tragstruktur 17 mit sich wiederholender Formgebung können z.B. jeweils einen Winkel von α = 20° mit einer Toleranz von 3 % einnehmen.

Figur 24A zeigt eine Seitenansicht als Prinzipskizze einer implantierbaren Einrichtung, bei der der distale Abschnitt 11 flach bzw. eben ausgebildet ist, wohingegen der proximale Abschnitt 10 gekrümmt geschlossen ausgebildet ist. In Figur 24B ist der distale Abschnitt 11 mit aufeinander zurückgefalteten Teilabschnitten 13, 14 ausgebildet.

Die Ausführungsform der implantierbaren Einrichtung gemäß Figur 25 weist im Unterschied zu der in Figur 14 gezeigten, ebenso wie die in den Figuren 19 und 20 gezeigte, im Wesentlichen keine durchgehende Durchgangsöffnung auf, da der proximale Abschnitt 10 keine solche mittlere Öffnung aufweist, sondern in seinem zentralen Bereich 22 im Wesentlichen geschlossen ausgebildet ist, wie der Detailansicht des in Figur 25 markierten Details F in Figur 26 entnommen werden kann. Um die Tragstruktur im den zentralen Bereich umgebenden Bereich stabil, jedoch offener zu gestalten, sind die einzelnen drahtartigen Elemente in einem den zentralen Bereich 22 der Tragstruktur 17 umgebenden Bereich miteinander verflochten, wie der Detailansicht des Details G aus Figur 25 in Figur 27 entnommen werden kann. Das Ende 51 des drahtartigen Elements 5 ist in diesem zentralen Bereich 22 mit einem Teilstück des drahtartigen Elements verflochten, um dieses gegen ein ungewolltes Lösen zu sichern. Der äußere Rand 18 ist hier nicht mit einem ein Membranelement befestigenden Element umwickelt, da kein Membranelement vorgesehen ist. Dies ist sowohl der Detailansicht des Details H des Randes 18 in Figur 28 zu entnehmen als auch der Detailansicht des Details I aus Figur 25 in Figur 29.

Figur 59 zeigt die Ausführungsform der implantierbaren Einrichtung gemäß Figur 25 in modifizierter Form mit einem daran befestigten Membranelement 4. Das Membranelement 4 liegt auf der gesamten Tragstruktur 17 auf, so dass kein umlaufender Rand der Tragstruktur frei bleibt. Das Risiko einer Embolisierung im Randbereich der implantierbaren Einrichtung 1 ist somit gering. Das Membranelement 4 erstreckt sich auch über den in Figur 59 gezeigten inneren Bereich 29 des proximalen Abschnitts und über das proximale Ende 25 hinweg, das hier im Wesentlichen geschlossen ausgebildet ist. Das Membranelement 4 ist an der umlaufenden Kante 24 der Tragstruktur 17 der implantierbaren Einrichtung 1 befestigt, z.B. dort angenäht.

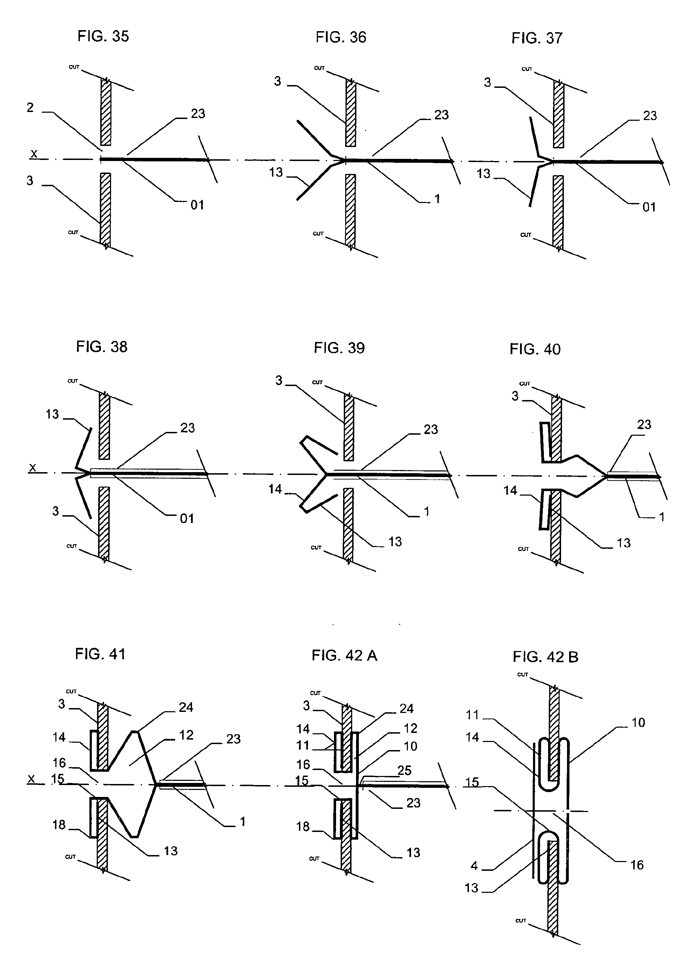

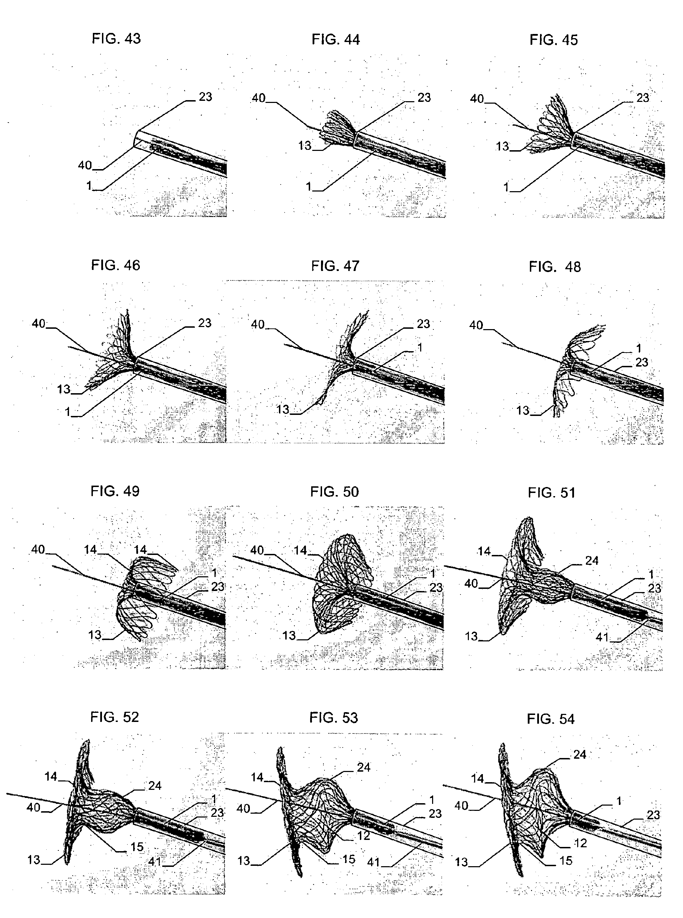

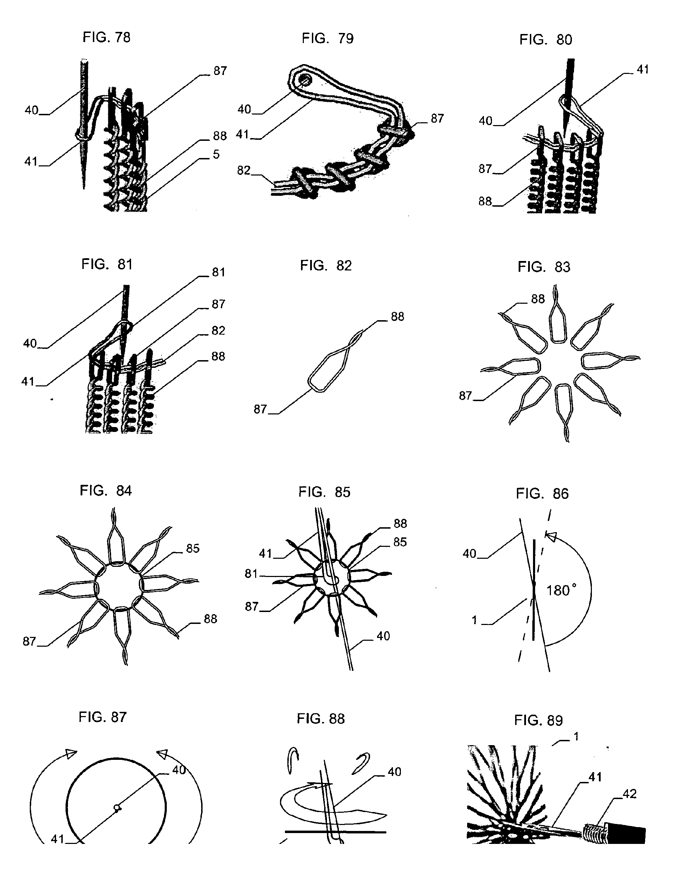

Die Figuren 35 bis 42B zeigen skizzenhaft den Vorgang des Absetzens einer implantierbaren Einrichtung 1 in der Öffnung 2 der Wandung 3. Die Figuren 43 bis 58 zeigen dies am Beispiel einer detailreicher, perspektivisch dargestellten implantierbaren Einrichtung 1. Im ersten Schritt in Figur 35 bzw. 43 wird ein Führungs- oder Abwurfdraht 40 und nachfolgend ein diesen umgebender Katheter 23, in dem sich die implantierbare Einrichtung innerhalb des Katheters 23 im entlang der Achse x langgestreckten Primärzustand, den Abwurfdraht umgebend, befindet, durch die Öffnung 2 geschoben. Zum Vorschieben wird beispielsweise ein Haltedraht 41, der an der implantierbaren Einrichtung 1 befestigt ist, relativ zu dem Abwurfdraht 40 bewegt. Die Anbringung des Haltedrahtes 41 an der implantierbaren Einrichtung wird zu den Figuren 78 bis 89 noch näher beschrieben. Im zweiten Schritt wird die implantierbare Einrichtung 1 aus dem Katheter 23 entlang dem Abwurfdraht 40 herausgeschoben. Dabei entfaltet sich zunächst der Teilabschnitt 13 des distalen Abschnitts 11, der später an der Wandung 3 anliegen wird. Dies ist insbesondere den Figuren 36 und 37 sowie 44 bis 47 zu entnehmen. Hierbei ist klar erkennbar, dass dem Teilabschnitt 13 eingeprägt ist, sich in Richtung proximal zurückzufalten. Vorteilhaft wird als Material für die implantierbare Einrichtung 1 ein Formgedächtnismaterial, insbesondere Nitinol, ein Kunststoffmaterial oder ein anderes Formgedächtnismaterial oder eine Kombination aus einem Formgedächtnismaterial und einem anderen Material, beispielsweise bei einer mehrlagigen Tragstruktur der implantierbaren Einrichtung, verwendet.

Beim weiteren Herausschieben der implantierbaren Einrichtung 1 aus dem Katheter 23 entfaltet sich der zweite Teilabschnitt 14 des distalen Abschnitts 11, wie den Figuren 39 sowie 48 bis 50 zu entnehmen. Je weiter die implantierbare Einrichtung aus dem Katheter herausgeschoben wird, desto weiter faltet sich der Teilabschnitt 13 auf den Teilabschnitt 14 zurück und legt sich beim weiteren Her-Teilabschnitt 13 auf den Teilabschnitt 14 zurück und legt sich beim weiteren Herausschieben der implantierbaren Einrichtung 1 aus dem Katheter 23 an diesem an.

Wird die implantierbare Einrichtung 1 weiter aus dem Katheter herausgeschoben, entfaltet sich auch der proximale Abschnitt 10 nach und nach, wie in den Figuren 40 bis 42 und 51 bis 57 zu entnehmen. Dabei expandiert sich der proximale Abschnitt 10 radial und bildet eine umlaufende Kante 24, so dass auch der proximale Abschnitt doppellagig ausgebildet ist. Allerdings ist das proximale Ende 25 des proximalen Abschnitts 10 zusammengefasst geschlossen oder mit einer minimalen Öffnung versehen ausgebildet, so dass der Innenraum 12 umgrenzt wird.

Ist die implantierbare Einrichtung vollständig aus dem Katheter herausgeschoben und hat sich hierbei in ihre Sekundärform entfaltet, weist sie z.B. die in den Figuren 42A oder 42Bund 58 gezeigte Form auf. Im Unterschied zu der Ausführungsform gemäß Figur 42A ist die Ausführungsform gemäß Figur 42B mit einem Membranelement 4 auf ihrem distalen Abschnitt versehen. Anstelle einer im Wesentlichen flachen Form des proximalen Abschnitts 10, wie er in diesen Figuren gezeigt ist, kann dieser auch bauchig ausgebildet sein, also einen größeren Innenraum umgrenzen, und/oder gekrümmt, wie z.B. in Figur 24B gezeigt.

Sitzt die implantierbare Einrichtung so an der Implantationsstelle, dass der gewünschte Verschluss oder Teilverschluss gegeben ist, kann die implantierbare Einrichtung von dem Abwurfsystem in Form des Abwurfdrahts 40 und Haltedrahts 41 getrennt werden. Eine entsprechende Darstellung ist in den Figuren 42B und 58 zu finden. Ist noch eine Nachjustierung erforderlich, kann diese durch weitere Relativbewegung von Abwurf- und Haltedraht erfolgen, bis die gewünschte Position erreicht ist. Hierbei ist auch eine Rotation der implantierbaren Einrichtung möglich, wie beispielhaft in Figur 87, einer skizzenhaften Draufsicht auf eine implantierbare Einrichtung mit durchgeführtem Abwurf- und Haltedraht, gezeigt. Ebenso ist eine Flotation der implantierbaren Einrichtung um den Abwurfdraht 40 und Haltedraht 41 herum möglich, wie in Figur 88 skizziert. Der Führungs- bzw. Abwurfdraht 40 ist insgesamt sehr dünn ausgebildet und weist eine sehr dünne Spitze auf, wodurch eine Flotation einer implantierbaren Einrichtung mit dem Abwurfdraht möglich ist. Die Spitze des Abwurfdrahtes kann einen Durchmesser von 0,1 bis 0,3 mm aufweisen. Die implantierbare Einrichtung kann jeden beliebigen Winkel gegenüber dem Abwurfdraht 40 einnehmen, wie in Figur 86 angedeutet. Somit ist eine Direktionierung der implantierbaren Einrichtung auch an ansonsten schlecht zugängliche Implantationsstellen in einem menschlichen oder tierischen Körper problemlos möglich.

Wie der Draufsicht auf die implantierbare Einrichtung 1 in Figur 60 entnommen werden kann, weist diese Ausführungsform gegenüber der Figur 58 gezeigten die Modifikation auf, dass diese mit einem Membranelement 4 versehen ist. Dieses weist einen geringeren Durchmesser als die implantierbare Einrichtung 1 auf, so dass umlaufend um das Membranelement 4 ein Rand der Tragstruktur 17 frei bleibt. Das Membranelement 4 ist auf dem nach distal gerichteten Teilabschnitt 14 des distalen Abschnitts 11 befestigt, insbesondere dort angenäht oder angeklebt. Durch das Zentrum der implantierbaren Einrichtung 1 und des Membranelements 4 ist der Abwurfdraht 40 hindurchgeführt, wie in Figur 60 ebenfalls angedeutet.

Die Positionierung der implantierbaren Einrichtung kann beispielsweise über bildgebende Verfahren verfolgt werden, wobei z.B. die implantierbare Einrichtung an einigen Stellen die bereits genannten Marker 52 aufweisen kann, die bei den bildgebenden Verfahren sichtbar sind. Diese Marker können z.B. randseitig vorgesehen sein, wie in Figur 22 gezeigt, oder in der Fläche der Tragstruktur 17, wobei sich eine randseitige Anordnung als vorteilhafter erweist, da die Umgrenzung der implantierbaren Einrichtung eindeutig erfassbar ist.

Die implantierbare Einrichtung 1 kann sich einer beliebigen Form einer Öffnung, 2, innerhalb derer sie angeordnet wird, anpassen. Beispielsweise wird sie oval, wenn sie in einer ovalen Öffnung abgesetzt wird, oder mehreckig, wenn sie in einer mehreckigen Öffnung abgesetzt wird. Die Figuren 61 bis 64 zeigen Ausführungsformen solcher Deformationen. Hierbei kann sich die gesamte implantierbare Einrichtung um alle Achsen krümmen, um sich der Formgebung an der Implantationsstelle, insbesondere an Wandungen dort, anzupassen. Aufgrund des durch den zurück gefalteten Teilabschnitt des einen Abschnitts doppelt aufeinander gelegten Abschnitts, passt sich die implantierbare Einrichtung problemlos an alle Formgebungen der Implantationsstelle an und nimmt eine stabile Position trotz der Deformation ein, klemmt sich sogar an der Implantationsstele fest.

Um die Implantation noch weiter zu verbessern, kann die implantierbare Einrichtung auch bereits beim Einprägen ihrer Sekundärform bei ihrer Herstellung mit einer Krümmung und/oder ovalen, mehreckigen oder anderen Formgebung versehen werden, um besser in eine entsprechend geformte Öffnung zu passen. Eine derartige Ausführungsform ist beispielhaft in Figur 65 zu sehen. Hier sind distaler und proximaler Abschnitt 10, 11 partiell auseinander gebogen, wie ein offenes Maul eines Tieres. Zur besseren Orientierung sind in Figur 65 nicht nur die beiden aufeinander gefalteten Teilabschnitte 13, 14 des Abschnitts 11 und der Abschnitt 10, sondern auch der Zwischenabschnitt 15 und der äußere Rand 18 des Abschnitts 11 gezeigt. Diese eingeprägte besondere Formgebung der implantierbaren Einrichtung 1 wird beispielsweise vorgesehen, um einen negativen Druck auf eine Aorta zu vermeiden. Wie in den Figuren 66 bis 68 zu sehen, kann die implantierbare Einrichtung sich um eine solche Aorta 31 herumlegen, so dass die Gefahr einer Beschädigung der Aortenwand nicht mehr besteht. Figur 66 zeigt dabei eine Draufsicht auf die implantierbare Einrichtung 1, die mit einem Membranelement 4 versehen ist, die Figuren 67 und 68 Seitenansichten der implantierbaren Einrichtung 1. In allen Fällen legt sich die implantierbare Einrichtung erkennbar um die Aorta 31 herum. Wie der Figur 68 zu entnehmen, kann auch der Zwischenabschnitt außermittig bezüglich der implantierbaren Einrichtung angeordnet sein, so dass in der Draufsicht eine exzentrische Formgebung der implantierbaren Einrichtung entsteht.

Die implantierbare Einrichtung kann bei den verschiedensten Problematiken verwendet werden, z.B. als ASD-, VSD- oder PFO-Verschlusseinrichtung, PDA-Verschlusseinrichtung, vaskulare Verschlusseinrichtung oder linke Herzohr-Verschlusseinrichtung.

Die Figuren 69 bis 74 zeigen weitere Ausführungsformen der implantierbaren Einrichtung 1, bei denen im Unterschied zu den vorhergehenden Ausführungsformen jedoch zumindest ein Abschnitt schneckenartig aufgewickelt ist. Hierbei wird zusätzlich zu den beiden Teilabschnitten 13 und 14, bei denen der Teilabschnitt 13 zurück oder rückwärts auf den Teilabschnitt 14 gefaltet wird, zumindest eine weitere Rückwärts- oder Zurückfaltung beim weiteren Herausschieben der implantierbaren Einrichtung aus einem Katheter und der Ausbildung der Sekundärform von dieser vorgenommen. Die beiden Teilabschnitte 13, 14 werden noch zumindest einmal um sich selbst herum gefaltet. Hierdurch wird dieser Abschnitt noch stabiler im Vergleich zu den implantierbaren Einrichtungen des Standes der Technik.

Der zunächst bei einem Entfalten der implantierbaren Einrichtung 1 aus einer Primärform in eine Sekundärform sich entfaltende Teilabschnitt ist der Teilabschnitt 133. Diesem folgt beim weiteren Herausschieben der implantierbaren Einrichtung aus einem Katheter 23 der Teilabschnitt 144, der sich in Richtung distal faltet, wohingegen der Teilabschnitt 133 sich in Richtung proximal faltet. Beim weiteren Herausschieben der implantierbaren Einrichtung aus dem Katheter faltet sich der Teilabschnitt 13 auf den Teilabschnitt 133 und nachfolgend der Teilabschnitt 14 auf den Teilabschnitt 144. Wenngleich in den Figuren nicht dargestellt, können auf diese Weise noch weitere Teilabschnitte aufeinander gefaltet werden, so dass eine mehr oder minder dicke Schnecke entsteht.

Die übrige Formgebung der implantierbaren Einrichtung 1 kann wie bei den übrigen Ausführungsformen beliebig sein. Gemäß Figur 69 ist lediglich der eine Abschnitt mit einer schneckartigen Formgebung versehen, wohingegen der andere im Wesentlichen flach scheibenförmig oder ambossartig ausgebildet und mit einer kleinen Durchgangsöffnung versehen ist. Die Durchgangsöffnung 16 ist bei dem schneckenartigen Abschnitt mit einem außenseitig angeordneten Membranelement 4 verschlossen. Bei der Ausführungsform gemäß Figur 70 ist wiederum der eine Abschnitt schneckenartig ausgebildet. Der andere Abschnitt ist bauchig geschwungen geformt, mit einem nach innen eingezogenen zusammengefassten proximalen Ende 25. Im Unterschied zu der Ausführungsform nach Figur 70 ist bei der Ausführungsform gemäß Figur 71 der proximale Abschnitt im Wesentlichen flach geschwungen geformt und weist einen größeren Durchmesser als der schneckenartige Abschnitt auf. Das innere Ende 25 des proximalen Abschnitts ist ebenfalls zusammengefasst. Im Unterschied hierzu ist der proximale Abschnitt gemäß Figur 74 mit etwa dem gleichen Durchmesser versehen wie der schneckartige Abschnitt und weist innerhalb des geschlossenen geschwungenen Abschnitts ein Membranelement 4 auf.

Die Ausführungsform gemäß Figur 72 ähnelt der gemäß Figur 69, jedoch mit dem Unterschied, dass eine vergleichsweise große Durchgangsöffnung in dem scheibenförmigen Abschnitt vorgesehen und ein Membranelement 4 dort angeordnet ist. Die Durchgangsöffnung in dem scheibenförmigen Abschnitt entspricht nahezu der Durchgangsöffnung durch die implantierbare Einrichtung selbst.

Die Ausführungsform gemäß Figur 73 weist zwei schneckenartig aufgefaltete oder aufgewickelte Abschnitte auf. Auch der proximale Abschnitt 10 weist hierbei einen nach proximal zurück oder rückwärts gefalteten Teilabschnitt 110 auf, der in einen nach distal weisenden Teilabschnitt, der seinerseits in einen nach proximal weisenden Teilabschnitt übergeht. Auch der proximale Abschnitt kann also schneckenartig durch mehrfaches Aufeinanderfalten von Teilabschnitten ausgebildet werden.

Die Figuren 75 und 76 zeigen ebenso wie die bereits erwähnte Figur 1 K eine für die Verwendung zum Verschluss eines PDA geeignete hutförmige Ausbildung der implantierbaren Einrichtung 1. Hierbei wird die implantierbare Einrichtung in die Defektöffnung eingefügt und der eine Abschnitt legt sich an die Aortenwand an. Vorzugsweise ist dieser der mit dem zumindest einmal zurückgefalteten Teilabschnitt versehene Abschnitt, da dieser eine entsprechende Formgebung annehmen oder sogar als Sekundärform eingeprägt bekommen kann. Die Hutformen in den genannten Figuren eignen sich daher besonders, da die jeweilige Hutkrempe als zumindest einmal zurück oder rückwärts gefalteter Abschnitt 26 sich an die Formgebung der Aortenwand anpassen und dort stabil festhalten kann. Bei der Ausführungsform gemäß Figur 1 K ist nur ein kurzer gerundeter das Kopfteil des Hutes bildender Abschnitt 27 vorgesehen, wohingegen gemäß Figur 76 ein besonders langer etwa zylindrischer Kopfteil-Abschnitt vorgesehen ist. Letzterer weist ein geschlossenes Ende auf, das ferner zu dem anderen die Hutkrempe bildenden Abschnitt nach innen eingezogen geformt ist. Der die Hutkrempe bildende Abschnitt kann quer zur Längserstreckung der implantierbaren Einrichtung gekrümmt geformt sein, um sich besser an eine Aortenwand oder eine andere Wandung an der Implantationsstelle anpassen zu können.

Eine an beiden Abschnitten auskragende Formgebung der implantierbaren Einrichtung 1 ist in Figur 75 gezeigt. Die Hutform weist nicht nur den auskragenden Hutkrempen-Abschnitt 26, sondern auch einen endseitig auskragenden Kopfteil-Abschnitt 27 auf. Beide Abschnitte können eine der Formgebung an der Implantationsstelle abgepasste Krümmung bzw. Formgebung und zurück oder rückwärts gefaltete Teilabschnitte, wie die Teilabschnitte 13, 14, aufweisen.

Neben den in den Figuren gezeigten hutförmigen Ausbildungen der implantierbaren Einrichtung können auch schiefe Formen, birnenartig ausbauende Formen des Kopfteilabschnitts, ein- oder mehrfach eingestülpte Formen etc. zum Anpassen an eine jeweilige Implantationsstelle vorgesehen sein.

In Figur 77 ist eine Ausführungsform der implantierbaren Einrichtung 1 an einem in einer Ummantelung 42 zur Stabilisierung angeordneten Abwurfdraht 40 gezeigt. Die implantierbare Einrichtung 1 weist einen bauchigen geschlossenen Abschnitt 10 und einen scheibenförmigen Abschnitt 11 mit aufeinander zurück gefalteten Teilabschnitten auf. Den Vorgang des Auffädelns des zumindest einen Haltedrahtes 41 an der implantierbaren Einrichtung 1 ist dies in den Figuren 78 bis 81 detailliert gezeigt. Ähnlich wie bei der Ausführungsform der implantierbaren Einrichtung 1 gemäß Figur 25 bzw. 26 sind um eine Durchgangsöffnung durch insbesondere den proximalen Abschnitt 10 eines zusammengefassten oder zusammenfassbaren proximalen Endes 25 herum Schlingen 87 als Enden von verdrillten Abschnitten 88 der drahtartigen Elemente 5 gebildet. Durch diese Schlingen 87 wird der Haltedraht 41 gefädelt, wie in Figur 78 bis 81 gezeigt. Eine solche Schlinge 87 mit daran anschließendem verdrillten Abschnitt 88 ist der Detailansicht in Figur 82 sowie den skizzenhaften Draufsichten auf eine Anzahl von acht kreisförmig angeordneten Schlingen 87 mit verdrillten Abschnitten 88 in den Figuren 83 bis 85 zu entnehmen.

Der Haltedraht liegt innerhalb und außerhalb der Schlingen 87 doppelt. Am Ende des Haltedrahtes 41 bildet dieser daher eine Schlaufe 81. Der Abwurfdraht 40 weist, wie bereits erwähnt, eine sehr dünne Spitze auf, um besonders einfach durch die endseitige Schlaufe 81 des Haltedrahts 41 hindurchgefädelt werden zu können. Zumeist wird der Abwurfdraht ferner durch das Zentrum, also die Durchgangsöffnung in dem proximalen Abschnitt und natürlich möglichst auch des distalen Abschnitts sowie eines ggf. vorgesehenen Membranelements 4 hindurchgefädelt.

Das in Figur 82 gezeigte Ende 81 des Haltedrahtes 41 wird zusammen mit dem Abwurfdrahtende zum Manipulieren des Abwurfvorgangs proximal beim Operateur gehalten. Das Ende 82 des Haltedrahtes 41 ist daher nicht so dicht an der implantierbaren Einrichtung vorgesehen, wie aus Figur 81 zu vermuten, da der zumindest eine Haltedraht 41 eine möglichst über die Länge des für die Implantation verwendeten Katheters 23 hinausgehende Länge aufweist.

Wie der Figur 84 zu entnehmen, können die Schlingen nicht nur unverbunden nebeneinander, die innere Durchgangsöffnung umgrenzend angeordnet sein, wie in Figur 83 zu sehen, sondern auch mit einem Tragring 85 verbunden sein. Der durch die Schlaufe 81 des Haltedrahts 41 durchgefädelte Abwurfdraht 40 geht dann z.B., wie in Figur 85 gezeigt, durch den Tragring hindurch, wobei der Haltedraht nicht durch alle Schlingen 87 gefädelt werden zu braucht, sondern auch ein Durchfädeln einer Schlinge 87 und/oder ein Umschlingen des Tragrings 85 ausreicht, um den Haltedraht 41 an der implantierbaren Einrichtung zu befestigen.

Figur 89 zeigt eine Detailansicht einer von dem Abwurfdraht 40 und dem Haltedraht 41 durchdrungenen implantierbaren Einrichtung 1, wobei Haltedraht 41 und Abwurfdraht 40 in der Ummantelung 42 innerhalb des Katheters 23 angeordnet sind, um eine bessere Stabilität beim Durchschieben durch den Katheter zu erzielen.

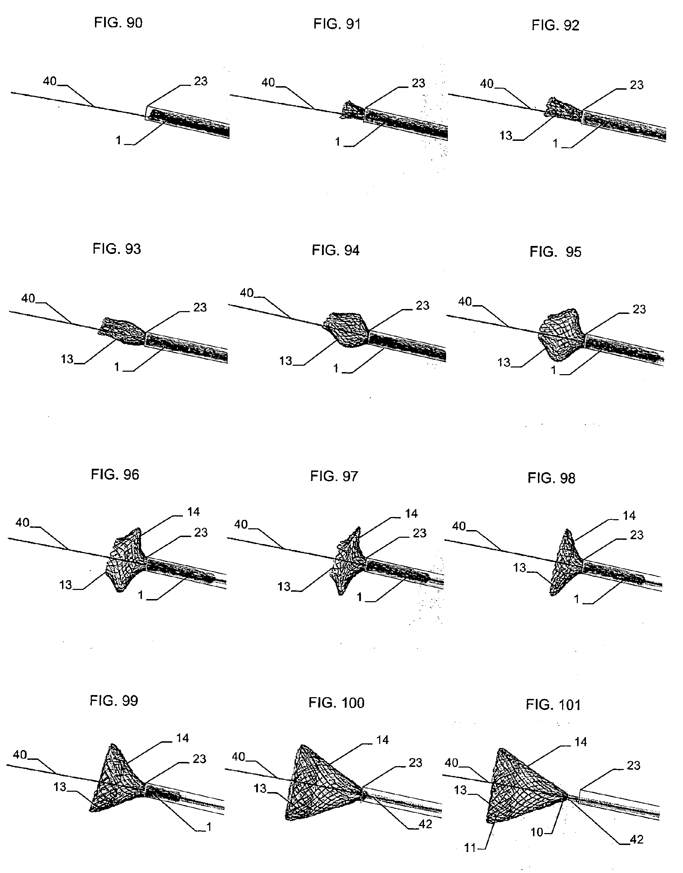

Die Figuren 90 bis 101 zeigen das Entfalten einer anderen Ausführungsform der implantierbaren Einrichtung 1 aus einer langgestreckten Primärform, die sie in dem Katheter 23 einnimmt, in eine Sekundärform, die sie außerhalb von diesem einnimmt, sobald sie sich entfaltet hat und die ihr zuvor bei ihrer Herstellung eingeprägt wurde. Figur 90 zeigt dabei die noch innerhalb des Katheters 23 angeordnete langgestreckte Primärform der implantierbaren Einrichtung, wobei sich durch den Katheter 23 und die implantierbare Einrichtung 1 hindurch deer Abwurfdraht 40 erstreckt. Der Abwurfdraht 40 ist an der implantierbaren Einrichtung 1 in entsprechender Weise befestigt, z.B. wie in den Figuren 78 bis 85 gezeigt.

Die Figuren 91 und 92 zeigen die implantierbare Einrichtung in einer aus dem Katheter 23 bereits ein Stück herausgeschobenen Position, wobei der Teilabschnitt 13 der implantierbaren Einrichtung als erster aus dem Katheter herausgeschoben wird. In der Darstellung in Figur 93 beginnt sich der Teilabschnitt 13 bereits einzufalten. Dieser Einfaltvorgang erfolgt nun nicht, wie bei den vorstehend beschriebenen Ausführungsformen nach außen, sondern nach innen in Richtung zu dem Abwurfdraht 40 hin. Dies kann den Figuren 94 bis 97 besonders gut entnommen werden.

Der bei dieser Ausführungsform in Richtung zu dem anderen noch in dem Katheter 23 befindlichen Abschnitt 10 weisende Teilabschnitt 14 der implantierbaren Einrichtung 1 entfaltet sich beim weiteren Herausschieben der implantierbaren Einrichtung aus dem Katheter ebenso weiter. Wie der Figur 98 entnommen werden kann, bildet sich eine Trichterform, dessen distales Ende doppelt gelegt ist, aufgrund des sich auf den äußeren Teilabschnitt 14 nach innen zurück faltenden (inneren) Teilabschnitts 13 und daher distal eine größere Stabilität aufweist.

Beim weiteren Herausschieben der implantierbaren Einrichtung aus dem Katheter wird die Trichterform der gesamten implantierbaren Einrichtung noch deutlicher (siehe Figuren 99 bis 101). Der äußere Teilabschnitt 14 des distalen Abschnitts 11 geht dabei in den proximalen Abschnitt 10 über. Letzterer weist ein zusammengefasstes im Wesentlichen geschlossenes proximales Ende auf. An diesem greift die Ummantelung 42 des Abwurfdrahts 40 zur Stabilisierung von diesem an, damit die implantierbare Einrichtung 1 problemlos aus dem Katheter 23 herausgeschoben werden kann.

Ersichtlich kann bei dem Einfalten des Teilabschnitts 13 nach innen in Richtung zu dem Abwurfdraht hin zwar ebenfalls eine Stabilisierung dieses Abschnitts 11 der implantierbaren Einrichtung 1 erzeugt werden. Diese Art der Einfaltung zur Stabilisierung eignet sich bei zahlreichen Anwendungen und Formen der implantierbaren Einrichtung, wie z.B. der in Figur 101 dargestellten Trichterform. Allerdings besteht bei dem Einfalten des Teilabschnitts 13 nach innen in Richtung zu dem Abwurfdraht 40 das Problem, dass ein Falten in die Primärform einer insbesondere vollständig entfalteten Sekundärform kaum möglich ist. Ein Zurückziehen der entfalteten implantierbaren Einrichtung in den Katheter hinein erweist sich aufgrund des doppelt gelegten Materials als schwierig, das dieses durch den Katheter 23 kaum hindurchpassen wird und somit ein anderer Katheter mit einem größeren Innendurchmesser verwendet werden müsste. Dies ist jedoch recht aufwendig. Daher eignet sich für den Fall eines nach dem Entfalten des Teilabschnitts 13 auf den Teilabschnitt 14 erfolgenden Hineinziehens der implantierbaren Einrichtung in den Katheter 23 das Zurückfalten des Teilabschnitts nach außen, wie es in den zuvor beschriebenen Ausführungsformen der Fall ist, als vorteilhafter, da hierbei ein Zurückfalten aus der Sekundär- in die Primärform problemlos möglich ist.

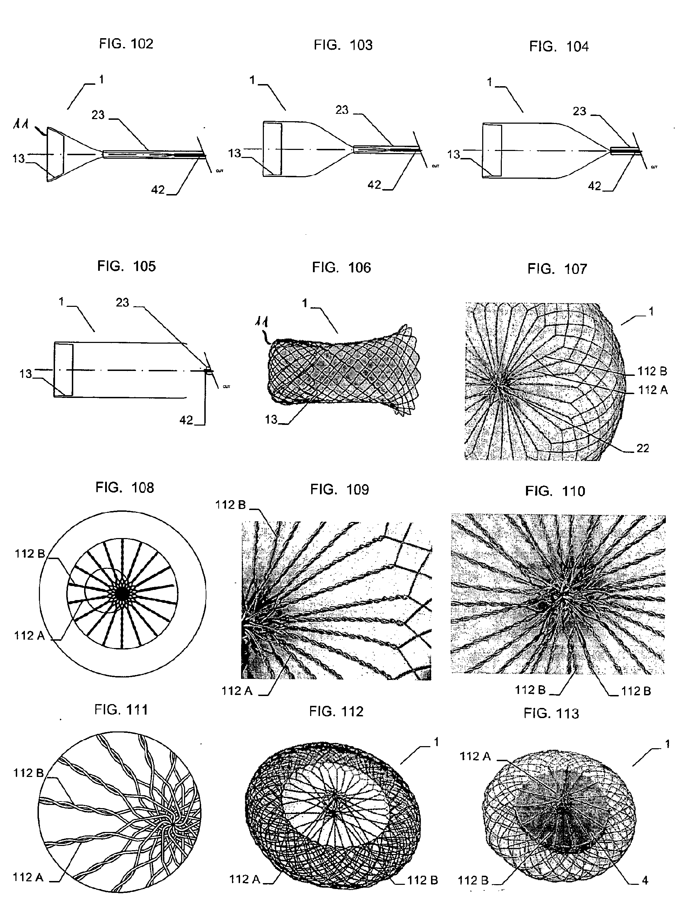

Figur 102 bis 106 zeigt eine Modifikation zur Ausführungsform gemäß Figuren 90 bis 101. Auch hier ist der Teilabschnitt 13 wiederum nach innen eingefaltet, um einen stabileren Rand zu bilden. Beim Herausschieben der implantierbaren Einrichtung aus dem Katheter 23 entfaltet sich diese letztendlich jedoch in Stentform, wie in Figur 105 und 106 zu sehen. Auch in dieser Ausführungsform ist wiederum eine Ummantelung 42 des Abwurfdrahts zur Stabilisierung von diesem vorgesehen, um die implantierbare Einrichtung 1 problemlos aus dem Katheter 23 herausschieben zu können. Erst nach dem Lösen der implantierbaren Einrichtung 1 von der Ummantelung 42 bzw. dem Katheter 23 entfaltet sich diese aus der vormaligen Trichter- in die gewünschte Stentform (Figur 105). Wie der Figur 106 entnommen werden kann, kann das proximale Ende der stentförmigen implantierbaren Einrichtung 1 auskragend ausgebildet sein. Durch das Vorsehen des zumindest einen doppellagigen Endes der stentförmigen implantierbaren Einrichtung kann an diesem Ende eine größere Steifigkeit erzeugt werden, so dass ein Wandern der Einrichtung innerhalb eines Gefäßes bei dessen Dilatation verhindert werden kann. Aufgrund der Doppellagigkeit ist das Gewebe bzw. Geflecht bzw. die Tragstruktur dort dichter, und es kann eine stabilere radiale Verankerungskraft ausgeübt werden.

Unter Bezug auf die Figuren 25 bis 29 ist in den Figuren 107 bis 119 eine weitere Ausführungsform der implantierbaren Einrichtung 1 gezeigt. Die sich durch das Verflechten zumindest jeweils zweier drahtartiger Elemente 5 ergebenden Beinchen 112A und 112B können je nach Anwendungsfall länger oder kürzer ausgebildet werden. Bei der Ausführungsform gemäß Figur 107ff. sind diese länger ausgebildet als bei der Ausführungsform gemäß Figur 25 bis 29. Die Beinchen erstrecken sich von dem zentralen Bereich 22 hin zu dem äußeren Rand 18 bzw. einen äußeren Bereich 21 und können anstelle eines flächigen Geflechts, wie es beispielsweise in den Figuren 18 bis 21 gezeigt ist, vorgesehen sein. In der Ausführungsform nach Figur 107ff. sind sie länger als in den Figuren 25 bis 29 ausgebildet und sind in Kombination mit einem flächigen Geflecht im äußeren Bereich 21 vorgesehen. Beim Ausbilden langer Beinchen 112A, 112B ist eine jm Vergleich zum Vorsehen kürzerer Beinchen bessere Konfigurierung möglich, da es zu keinem ungewollten Strecken (Stretchen) kommt. Ferner ist auch eine bessere Befestigung eines Membranelements am äußeren Rand bzw. den Beinchen möglich. Zudem lässt sich eine implantierbare Einrichtung mit solchen längeren Beinchen besser in einen Katheter hineinziehen als eine überwiegend als flächiges Gebilde ausgebildete implantierbare Einrichtung.

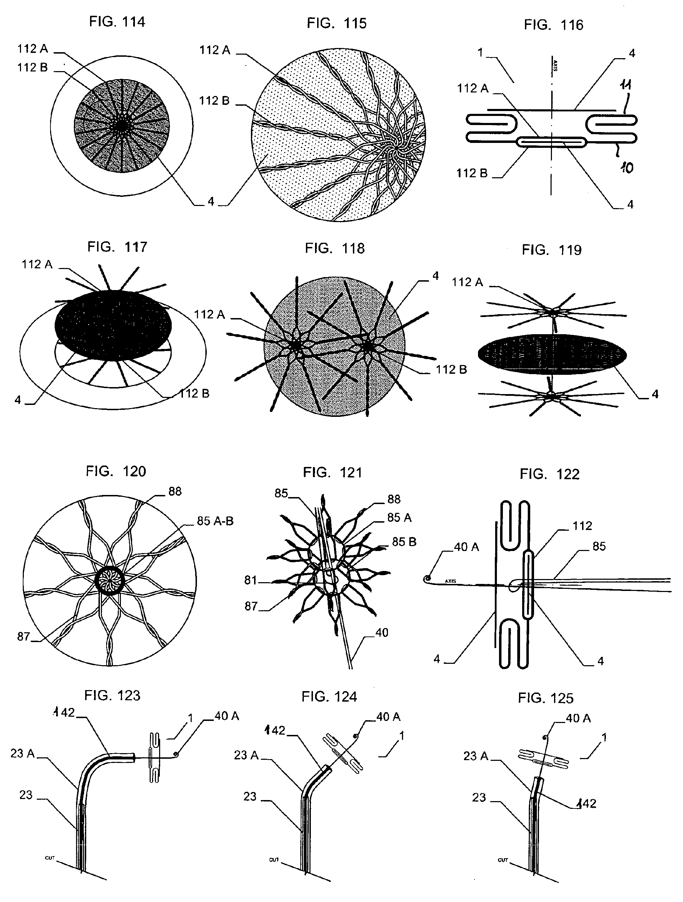

Wie den Figuren 109ff. entnommen werden kann, sind die Beinchen 112A und 112B in unterschiedlichen Ebenen angeordnet, so dass ein Membranelement 4 zwischen diesen angeordnet werden kann, wie in Figur 113 gezeigt. Ein solches Einfassen des Membranelements auf dessen Ober- und Unterseite kann ein ungewolltes Perforieren desselben verhindern. Besonders deutlich ist dieser Aufbau auch in Figur 4 skizziert, wo der eine aus den Beinchen 112A und der andere aus den Beinchen 112B gebildete Stern übereinander unter Zwischenfügen des Membranelements 4 angeordnet sind. In der Draufsicht auf die Sterne ist dies auch in Figur 118 angedeutet, ebenso in der perspektivischen Ansicht in Figur 117.

Das Membranelement 4 kann jedoch auch auf nur einer Seite, also nicht zwischen den Beinchen 112A und 112B, angeordnet werden, wie dies in Figur 114 gezeigt ist. Figur 118 zeigt eine Detailansicht der Figur 114 in dem gekennzeichneten Bereich. Hier ist deutlich erkennbar, dass die Beinchen 112A und 112B auf ein und derselben Seite des Membranelements 4 angeordnet sind.

Figur 116 zeigt eine Skizze eines Querschnitts durch die implantierbare Einrichtung 1, bei der zwei Membranelemente 4 vorgesehen sind. Das eine ist außenseitig auf dem Abschnitt 11 bzw. Teilabschnitt 14 vorgesehen, wohingegen das andere Membranelement 4 zwischen den Beinchen 112A und 112B angeordnet ist, an dem Abschnitt 10. Es können also an den verschiedensten Stellen Membranelemente 4 vorgesehen werden und es kann eine beliebige Anzahl von Membranelementen an der implantierbaren Einrichtung befestigt werden.

Wie bereits zu den Figuren 84 und 85 beschrieben, können die Schlingen 87 im zentralen Bereich durch einen Tragring 85 miteinander verbunden sein. Eine Modifikation hierzu ist in den Figuren 120 und 121 gezeigt. Hierbei sind mehrere Tragringe 85A und 85B vorgesehen. Ein Teil der Schlingen wird in dem einen Tragring aufgenommen und der andere in dem anderen (siehe Figur 121). Grundsätzlich können auch mehr als zwei Tragringe vorgesehen werden, wenn sich dies bei einer bestimmten Anwendungsform als vorteilhaft erweist.

In Figur 122 ist die implantierbare Einrichtung 1 nach Figur 116 auf einem Abwurfdraht 40 angeordnet. Der Abwurfdraht 40 weist eine aufgerollte Spitze 40A auf. Durch Vorsehen einer solchen aufgerollten Spitze können ungewollte Perforationen einer Gefäßwand vermieden werden, die beim Anstoßen mit dem Abwurfdraht ansonsten auftreten könnten. Auch ein Verhaken an der Gefäßwand kann hierdurch sicher vermieden werden.

Zum besseren Sichtbarmachen der Spitze kann diese mit einem Goldmarker versehen sein, insbesondere auch bei Ausbilden der Spitze als gerollte Spitze. Für das Sichtbarmachen der implantierbaren Einrichtung 1 kann diese ferner an einer oder mehreren Stellen, insbesondere im Bereich des Membranelements, das aus einem Polymer, wie Dacron, bestehen kann, mit einem Röntgen- und/oder Ultraschallmarker versehen sein. Durch Vorsehen eines Röntgenmarkers kann auch ein aus einem unter dem Röntgenschirm üblicherweise nicht sichtbaren Material, wie einem Polymer, bestehendes Membranelement beim Röntgen zum Überwachen des Platzierens der implantierbaren Einrichtung sichtbar gemacht werden. Bei einer Ultraschallüberwachung kann umgekehrt üblicherweise zwar das Membranelement gesehen werden, nicht jedoch das übrige Material der implantierbaren Einrichtung, so dass sich das Vorsehen von Ultraschallmarkern an diesem als vorteilhaft erweist. Auch Magnetresonanzmarker oder jede andere Art von Markern, die ein Sichtbarmachen mit einem gewünschten Gerät ermöglichen, können vorgesehen werden.

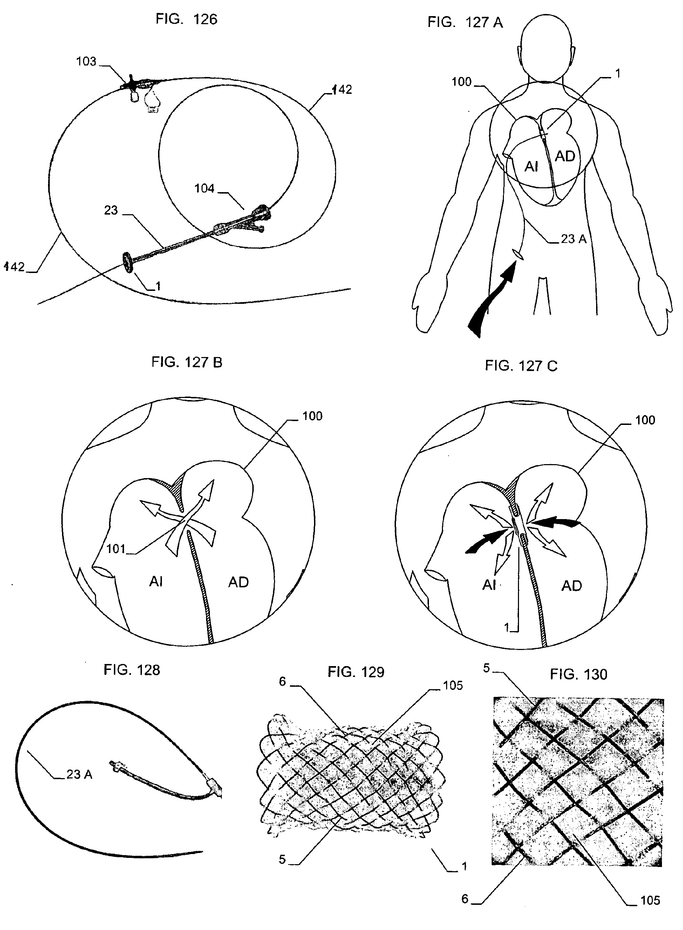

Ferner ist auch das Vorsehen von Echomarkern möglich, die insbesondere durch das Einfügen von Polyesterfasern, z.B. Dacronfasern, in eine Tragstruktur aus Nitinol erzielt werden kann. Solche Fasern dienen ferner der Abdichtung der Tragstruktur, also dem Verdichten der Struktur, was sich beispielsweise den Figuren 129 und 130 entnehmen lässt. Hierbei sind Polyesterfasern 105 zwischen die drahtartigen Elemente 5 und 6 eingeflochten. Eine solche Ausführungsform kann ferner sehr gut als Aortenstent zur Behandlung von Aortenaneurysmen eingesetzt werden, insbesondere in der Stentform nach Figur 105 bzw. 106.

Die Figuren 123 bis 125 zeigen unterschiedlich gekrümmte Schleusenkatheter 23A zum Zuführen der implantierbaren Einrichtungen an die jeweilige Implantationsstelle. Da die jeweiligen Implantationsstellen gegenüber einem Gefäß, über das der Katheter mit der implantierbaren Einrichtung zugeführt wird, in einem von Fall zu Fall unterschiedlichen Winkel stehen können, besteht die Möglichkeit einer individuellen Anpassung des Winkels des Katheters bzw. eines Pushers 142 an diesen Winkel. Hierzu ist der eigentliche Katheter 23 innerhalb des Schleusenkatheters 23A angeordnet. Der Katheter 23 ist als Führungskatheter üblicherweise nicht flexibel ausgebildet und kann gerade oder gekrümmt ausgebildet sein. In den Ausführungsformen gemäß Figur 123 bis 125 ist er gerade ausgebildet. Innerhalb des Katheters 23 ist ferner der Pusher 142 vorgesehen, der flexibel ist und einen gewünschten bzw. vorgebbaren Winkel einnehmen kann. Der innerhalb des Pushers 142 angeordnete Abwurfdraht 40 - mit seiner hier gerollten Spitze 40A - kann steif, jedoch durch den Pusher in seinem Krümmungswinkel anpassbar ausgebildet sein. Zur Variation des Krümmungswinkels des Schleusenkatheters kann entweder dieser eine an den jeweiligen Anwendungsfall angepasste Krümmung aufweisen und hierdurch die in diesem angeordneten Elemente des Pushers und des Abwurfdrahts in diese Krümmung zwingen oder der Pusher oder der Abwurfdraht weisen die gewünschte Krümmung auf und zwingen damit auch den Schleusenkatheter in die gewünschte Krümmungsform. Es können auch alle Elemente eine Krümmung aufweisen und durch die Kombination der gewünschte Krümmungswinkel erzeugt werden. Beispielsweise kann der Schleusenkatheter einen Krümmungswinkel von 45° aufweisen, wie in Figur 124 gezeigt und damit eine Implantation in diesem Winkel ermöglichen. Auch ein Krümmungswinkel von etwa 90° ist möglich, wie in Figur 123 gezeigt, ebenso ein Winkel von weniger als 45° wie in Figur 125 gezeigt.

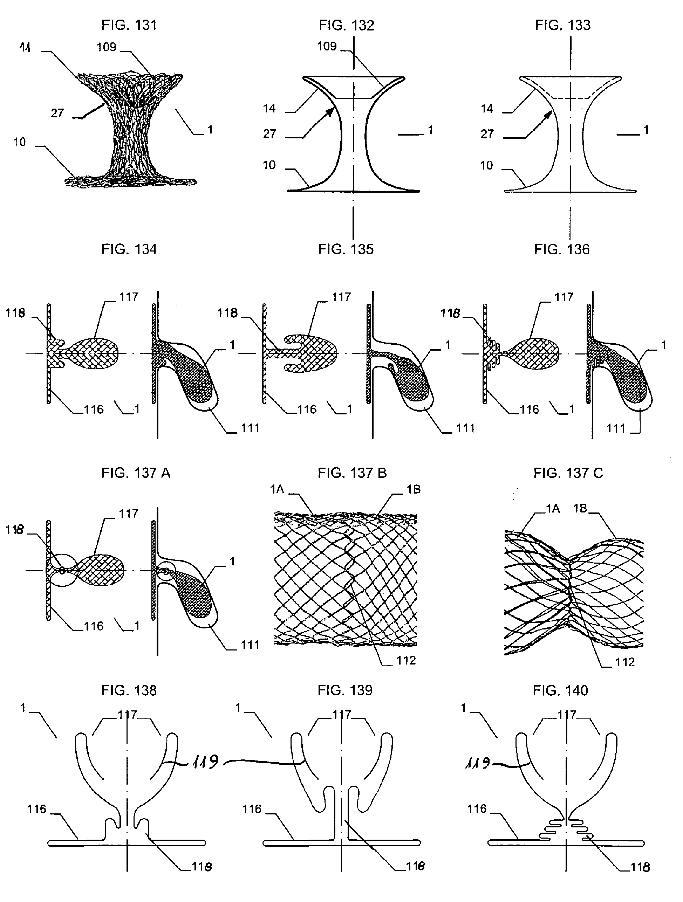

Gerade für die Implantation im Left Appendix ist das Einstellen eines richtigen Krümmungswinkels wichtig, wie aus den Figuren 134 bis 137A ersichtlich. Da der Left Appendix eine besonders schwierige Formgebung aufweist und in einem von Patient zu Patient unterschiedlichen Winkel zur umgebenden Wandung steht, ist ein korrektes Platzieren hier besonders schwer. Da vorliegend der Platzierungswinkel über die Kombination von Schleusenkatheter, Pusher und Abwurfdraht gesteuert werden kann, ist ein genaues Platzieren der implantierbaren Einrichtung 1 in dem Left Appendix möglich.

Eine für die Implantation im Left Appendix geeignete Formgebung der implantierbaren Einrichtung 1 ist in den Figuren 131 bis 133 gezeigt. Diese weist am distalen Abschnitt 11 einen nach innen eingefalteten Teilabschnitt 109 auf, wobei der Abschnitt 11 nach außen auskragend ausgebildet ist, ebenso wie der proximale Abschnitt 10, der allerdings scheibenförmig ist. Die Formgebung entspricht der in Figur 75 gezeigten. Die Figuren 132 und 133 zeigen jeweils Querschnittsansichten als Skizzen, aus denen der eingefaltete Teilabschnitt 109 ebenso wie die auskragende Formgebung beider Abschnitte 10 und 11 besonders gut entnommen werden kann.

Die Figuren 134 bis 140 zeigen verschiedene Formen implantierbarer Einrichtungen 1, die für die Implantation in einem Left Appendix 111 geeignet sind. Die in Figur 134 gezeigte Ausführungsform weist einen scheibenförmigen Abschnitt 116, einen gestauchten Zwischenabschnitt 118 und einen distalen Abschnitt 117 in Ballonform auf. In dem rechten Teil der Figur 134 ist der ballonförmige Abschnitt 117 in den Left Appendix 111 eingefügt, der Zwischenabschnitt 118 lagert in dem schmalen Abzweig des Left Appendix und der scheibenförmige Abschnitt 116 lagert an der Herzwand an, aus der der Left Appendix abzweigt. Durch das nach innen gerichtete Rückwärtswickeln des einen in den Left Appendix eingefügten Abschnitts 117 bzw. des Abschnitts 11 bei der Ausführungsform nach Figur 131 ist eine Versteifung und besonders gute Verankerung in dem Left Appendix möglich. Aufgrund des langen (Figuren 75 und 131 bis 133) bzw. verformbaren Zwischenabschnitts 118 kann eine Anpassung an den Winkel zwischen Herzwand und Left Appendix problemlos vorgenommen werden, wobei ein sehr gutes Abdichten durch die Formanpassungsfähigkeit möglich ist. Der scheibenförmige Abschnitt 116 kann entweder nur einlagig oder doppellagig ausgebildet werden. Zur Verbesserung des Halts durch erhöhte Steifigkeit in diesem Bereich wird der scheibenförmige Abschnitt 118 vorzugsweise doppellagig durch Zurückfalten eines Teilabschnitts auf den anderen, wie vorstehend zu den übrigen implantierbaren Einrichtungen bereits mehrfach ausgeführt, ausgebildet.

Anstelle des Ausbildens eines ballonartigen Abschnitts 117 kann dieser auch ankerartig sein, wie in Figur 135 gezeigt. Der Zwischenabschnitt 118 ist in dieser Ausführungsform im Wesentlichen gerade ausgebildet. Auch mit dieser Formgebung der implantierbaren Einrichtung 1 ist ein guter Verschluss des Left Appendix möglich, wie in der rechten Darstellung in Figur 135 gezeigt.

Eine ähnliche Ausführungsform wie in Figur 134 ist in Figur 136 gezeigt, wobei bei letzterer im Unterschied zur Figur 134 ein ziehharmonikaartig aufgefalteter Zwischenabschnitt 118 vorgesehen ist. Wie dem rechten Teil der Figur 136 zu entnehmen ist, kann auch mit dieser Formgebung ein guter Verschluss des Left Appendix erzielt werden.

Bei der Ausführungsform nach Figur 137A ist anstelle eines ziehharmonikaartigen Zwischenabschnitts 118 dieser schmal ausgebildet und zum vereinfachten Krümmen aus zwei miteinander verknüpften Hälften 1 A und 1 B zusammengesetzt. In einem Anschlussbereich 112 sind die beiden Hälften aneinandergefügt, insbesondere unter Verwendung nur des einen drahtartigen Elementes zum Erzeugen beider Hälften. In diesem Anschlussbereich 112 kann eine definierte Steifigkeit der Tragstruktur erzielt werden. Dies erweist sich bei der Anwendung für den Verschluss eines Left Appendix als besonders vorteilhaft, da auch nach dem Einfügen in den Left Appendix bzw. die Herzwand in diesem Bereich ein guter Halt möglich ist. Anstelle des Vorsehens eines gleichmäßigen Durchmessers beider Hälften 1A und 1 B, wie in Figur 137B gezeigt, kann in dem Anschlussbereich 112 ein geringerer Durchmesser vorgesehen werden, wie in Figur 137C zu sehen ist. Auch hierüber ist ein variables Krümmen für die Anpassung an den Left Appendix möglich. Eine Bewegung beider Abschnitte 116 und 117 ist ebenfalls weiterhin in dem erforderlichen Maße möglich.

In Figur 138 ist eine Variante der Ausführungsform nach Figur 134 zu sehen, in Figur 139 eine Variante der Ausführungsform nach Figur 135 und in Figur 140 eine Variante der Ausführungsform nach Figur 136. Bei allen drei Figuren 138 bis 140 ist jeweils der Abschnitt 117 doppellagig ausgebildet, wobei ein Teilabschnitt 119 nach innen eingefaltet ist, um eine größere Steifigkeit des Abschnitts 117 vorzusehen. Die äußere bauchige Formgebung des Abschnitts 117 entspricht ansonsten der in den Figuren 134 bis 136 dargestellten. Der Abschnitt 117 ist in den Figuren 138 bis 140 im Unterschied zu diesen allerdings offen und nicht geschlossen ausgebildet.

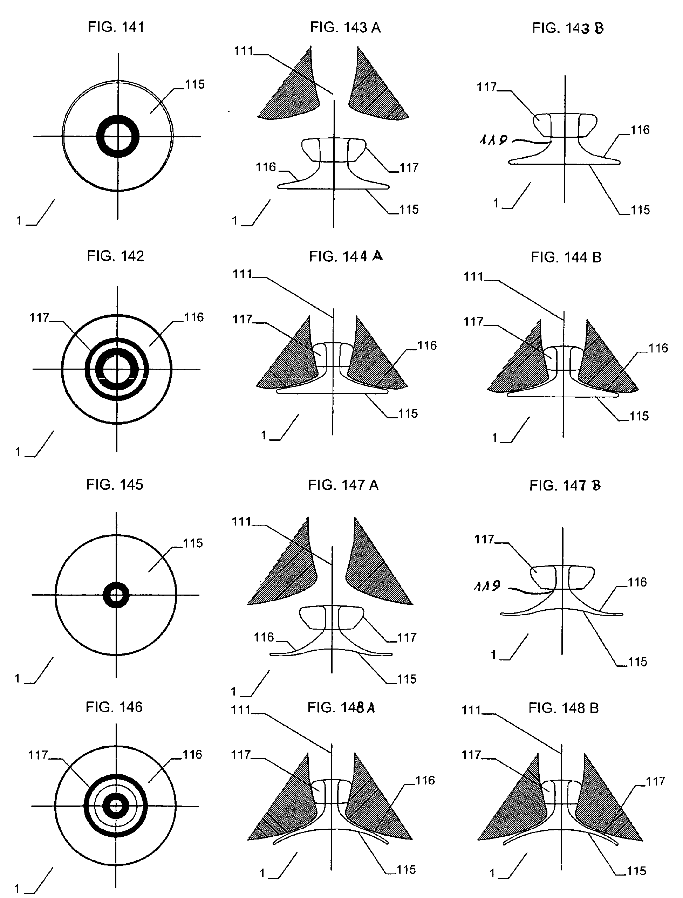

Die Figuren 141 bis 148 zeigen weitere Ausführungsvarianten von implantierbaren Einrichtungen 1 für den Verschluss eines Left Appendix 111. Die Figuren 141, 142 und 145, 146 zeigen jeweils einmal die Ansicht von unten auf den Teilabschnitt 115 des scheibenförmigen Abschnitts 116 (Figuren 141 und 145) und einmal die Ansicht von oben auf den distalen Abschnitt 117 und den scheibenförmigen Abschnitt 116 (Figuren 142 und 146). Die implantierbaren Einrichtungen 1 weisen jeweils eine ähnliche Formgebung auf, wie aus den Figuren 143, 144, 147, 148 hervorgeht. Der distale Abschnitt 117 ist nach außen umgefaltet ausgebildet, in den verschiedenen Figuren mit einem unterschiedlichen Durchmesser. Auch der Zwischenabschnitt 119 weist jeweils einen unterschiedlichen Durchmesser auf, so dass der Unterschied zwischen dem Durchmesser des Abschnitts 117 und des Zwischenabschnitts 119 variiert. Ferner ist auch die Formgebung des Abschnitts 116 variiert, wobei dieser in allen Ausführungsformen doppellagig mit nach außen gerichtetem Teilabschnitt 115 ausgebildet ist. In der Ausführungsform nach Figur 143 ist der Abschnitt 115 im Wesentlichen eben ausgebildet. In der Variante nach Figur 144 ebenfalls. In der Variante nach Figur 147 ist der Teilabschnitt 115 wechselnd konkav-konvex, also geschwungen ausgebildet, wobei ein Anlegen an einer entsprechend geformten Wandung, wie gezeigt, besonders gut und sicher möglich ist. Die Variante nach Figur 148 weist eine konkave Formgebung des Abschnitts 115 auf, wodurch ebenfalls ein guter Halt an der gezeigten Wandungsform möglich ist. Zusätzlich zu den gezeigten Ausführungsvarianten können noch zahlreiche weitere, insbesondere auch Zwischenformen der gezeigten gebildet werden, die jeweils an die konkrete Formgebung der Wandung und der zu verschließenden Öffnung sowie deren Abmessungen angepasst sein können.

Figur 126 zeigt eine implantierbare Einrichtung 1 an einer Platziervorrichtung. Die implantierbare Einrichtung 1 ist auf dem Abwurfsdraht angeordnet, bereits aus dem Katheter 23 herausgeschoben und vollständig entfaltet. Am proximalen Ende des Katheters 23 ist ein Griffteil 104 der Platziervorrichtung angeordnet bzw. der Katheter auf dessen eines Ende aufgeschoben. Der Pusher 142 geht durch den Griffteil 104 hindurch zu einem Betätigungsteil 103, vermittels dessen Haltedrähte, Abwurfdraht und Pusher in der gewünschten Weise betätigt werden können. Figur 128 zeigt das Detail des Schleusenkatheters 23A.

Die Figuren 127A bis 127C zeigen eine Skizze eines Patienten mit vergrößert dargestelltem Herzen 100 mit einem Shunt 101 in der Wand zwischen rechter und linker Herzkammer (Al und AD). Der Implantationsweg durch einen kleinen Schnitt in der Leiste des Patienten über die Blutgefäße zum Herzen ist gezeigt. Die implantierbare Einrichtung wird innerhalb des Schleusenkatheters 23A durch die Blutgefäße in das Herz 100 des Patienten vorgeschoben. Wie Figur 127B zeigt, ist durch den Shunt 101 ein Austausch von Blut aus den beiden Herzkammern Al und AD möglich, also ein Leckagestrom, was jedoch unterbunden werden soll. Zu diesem Zweck wird die implantierbare Einrichtung 1, wie in Figur 127C gezeigt, innerhalb der Öffnung bzw. des Shunts 101 platziert und verschließt diese. Dies ist durch die schwarzen und weißen Pfeile verdeutlicht.

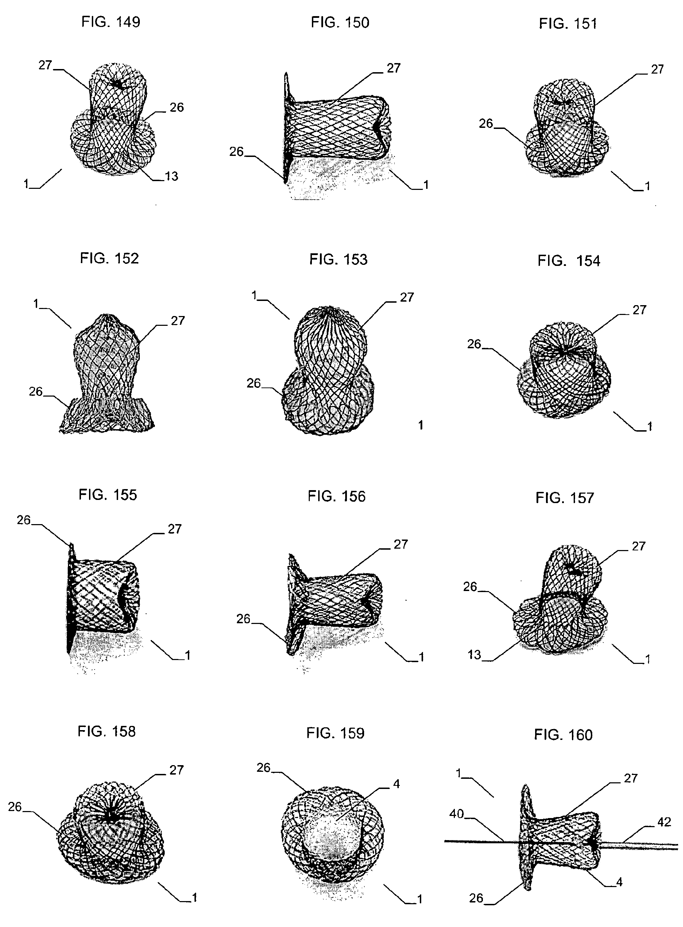

Die Figuren 149 bis 172 zeigen zahlreiche Varianten der Hutform der implantierbaren Einrichtung 1, die bereits in den Figuren 1 K sowie 76 dargestellt ist. Der die Hutkrempe bildende Abschnitt 26 ist jeweils zur Versteifung doppellagig ausgebildet und der Kopfteil-Abschnitt 27 ist jeweils zusammengefasst und - außer in den Figuren 152 und 153 - nach innen eingezogen ausgebildet. Es können gerade und schiefe Hutformen gebildet werden. Auch die Abmessungen der Hutformen kann variiert werden. Ferner kann zumindest ein Membranelement 4 in die implantierbare Einrichtung 1 eingebracht werden, wie in Figur 159 und 160 gezeigt. In Figur 159 ist das Membranelement 4 innerhalb des Hutkrempenabschnitts 26 und in Figur 160 in dem Kopfteilabschnitt 27 angeordnet. Auch ein Anordnen zwischen den beiden Abschnitten 26 und 27 oder ein Anordnen mehrerer Membranelementen in diesen Abschnitten 26 und 27 ist möglich.

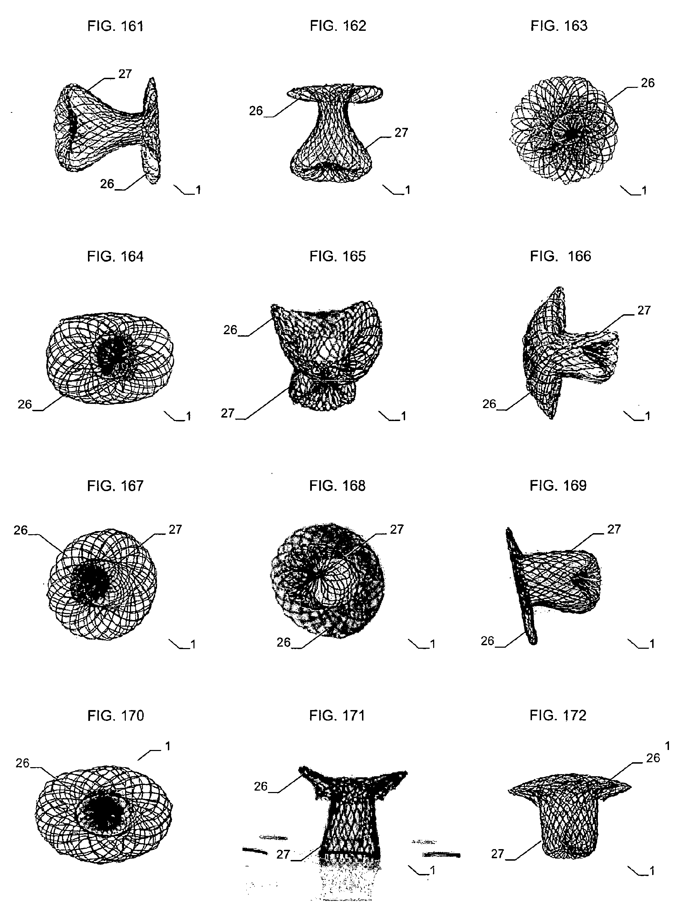

Der Hutkrempenabschnitt 26 kann etwa eben scheibenförmig ausgebildet sein und/oder eine Krümmung aufweisen, wie in Figur 156 und den Figuren 164, 165, 166, 171 und 172 gezeigt. Die Krümmung kann konkav und/oder konvex oder beliebig sein, wodurch eine Anpassung an die Formgebung der jeweiligen Implantationsstelle möglich ist. Auch der Durchmesser des Hutkrempenabschnitts 26 im Vergleich zu dem des Kopfteilabschnitts 27 kann an die jeweiligen Gegebenheiten an der Implantationsstelle angepasst werden. Entsprechend kann auch die Formgebung des Kopfteilabschnitts 27, wie in den Figuren 161 und 162 gezeigt, ballonartig auskragend oder gerade ausgebildet werden, wie z.B. in Figur 172 gezeigt. Der Kopfteilabschnitt 27 und der Hutkrempenabschnitt 26 köpnnen ferner eine runden oder ovalen Durchmesser aufweisen oder beliebig geformt sein. Durch das Vorsehen des doppellagigen Abschnitts 26 ist jedoch auch eine Abpassung an die unterschiedlichsten Formgebungen einer Implantationsstelle möglich, wobei zugleich ein stabiler Sitz dort sichergestellt werden kann.

Die Ausführungsform der hutförmigen implantierbaren Einrichtung nach Figur 152 und 153 weist einen nach außen gewölbten Kopfteilabschnitt 27 auf. Diese Variante ist besonders geeignet für den Verschluss tubulärer Gefäße. Bei dieser ist der Hutkrempenabschnitt 26 nicht flach eben ausgebildet, sondern zu dem Kopfteilabschnitt 27 zurück gefaltet, wobei letzterer eine Tropfenform aufweist.

Die vorstehend beschriebenen implantierbaren Einrichtungen können auf ihren Oberflächen behandelt oder beschichtet sein. Diese Oberflächenbehandlung bzw. -beschichtung kann an dem für das Formen der Tragstruktur verwendeten Material und/oder an der fertig geformten Tragstruktur vorgesehen werden. Im Folgenden wird eine Übersicht über hierbei verwendbare Materialien und Verfahren zum Behandeln der Oberflächen gegeben.

Als anorganische und keramische Materialien eignen sich vor allem Biogold, Gold, DLC (Diamond-like Carbon, also diamantartiger Kohlenstoff), DLN (Diamond-like Nanocomposite, also diamantartiges Nanocomposit), Iridiumoxid, das sich besonders für die Oberflächenpassivierung und antioxidative Beschichtungen eignet, nanoporöses AL₂O₃ (Arzneimittel eluierend), Siliciumkarbid, HaP (Hydroxylapatit) und Titannitritoxid.

Als synthetische Polymere eignen sich besonders Parylene C (Poly(2-chlor-p-xylylen)), PBMA (Polybutylmethacrylat), PC (Phosphorylcholin), PE (Polyethylen), PEVA (Polyethylen Vinylacetat), PHMA (Polyhexylmethacrylat), Polyzene®-F (PTFEP bzw. Poly[bis](trifluoroethoxy)phosphazene), PTFE (Polytetrafluorethylen) und PU (Polyurethan), wobei sich dieses besonders auch als Dünnschicht eignet. Weitere synthetische Polymere mit entsprechender positiver Wirkung in Bezug auf die Implantationsstelle (Unterstützung eines Embolisiervorgangs, Verhindern einer Thrombenbildung etc.) können ebenfalls verwendet werden.

Im Bereich der ebenfalls vorteilhaft verwendbaren humanen Biopolymere eignen sich vor allem Collagen, Chondroitin Sulfat, Elastin, Fibrin und Hyaluronsäure.

Ferner können die Oberflächen auch mit reinen Medikamentenbeschichtungen versehen werden, wie z.B. mit Abciximab (als Glykoproteinhemmer), Heparin (als Blutgerinnungshemmer) und/oder Paclitaxel (herkömmlich zum Stören der Zellteilung eingesetzt). Auch andere reine Medikamentenbeschichtungen können vorgesehen werden.

Zudem können medikamentenfreisetzende Polymere verwendet werden, wie beispielsweise Cellulose zum Freisetzen von Abciximab, PC zum Freisetzen von Angiopeptin, PE zum Freisetzen von DNA, PEVA/PBMA zum Freisetzen von Rapamycin, Poly(L-lactid) (PLLA) zum Freisetzen eines anwendungsspezifisch auswählbaren Medikaments, Polylactid (PLA) zum Freisetzen von Paclitaxel bzw. Hirudin oder Iloprost, PLA-PC zum Freisetzen eines viral vector, PU zum Freisetzen von Forskolin. Ferner können auch andere Kombinationen von Polymeren und Medikamenten wirksam sein. Ein viral vector kann dabei zum Einbringen genetischen Materials in Zellen lebender Organismen verwendet werden. Die vorstehend genannten Polymere sind zumeist resorbierbare Biomaterialien, so dass die genannten Medikamente entsprechend ihre Wirkung entfalten können, wohingegen die Polymere keine negative Wirkung auf den Organismus des Patienten haben. Beispielsweise weist Rapamycin zumeist eine stark zellabtötende Wirkung auf und wird dazu verwendet, zu verhindern, dass es zu Abstoßungsreaktionen kommt. Hirudin weist eine blutgerinnungshemmende Wirkung auf und Iloprost wirkt gefäßerweiternd und gefäßschützend.

Es ist ebenfalls möglich, funktionalisierte Oberflächen mit Biomolekülen und funktionalen Gruppen als Oberflächenbeschichtung der implantierbaren Einrichtung vorzusehen, z.B. im Rahmen des sog. Molecular Surface Engineering. Hierbei werden funktionalisierte Oberflächen aus unmodifizierten Oberflächen durch Einbringen funktioneller Gruppen geschaffen. U.a. können hierbei mit speziellen Funktionen ausgestattete Grenzflächen erzeugt werden.

Im Bereich der Gentechnik hat sich herausgestellt, dass auch Stammzellen vorteilhaft zur Oberflächenbeschichtung von implantierbaren Einrichtungen eingesetzt werden können. Beispielsweise kann die Oberfläche von Stents oder anderen implantierbaren Einrichtungen gezielt für die Adhesion im Blut zirkulierender Progenitorzellen mit Zell-Ligandstrukturen versehen werden..

Zur Oberflächenbehandlung eignen sich vor allem Verfahren zur Abscheidung, Abtragung bzw. Veränderung von Grenzschichten, wie beispielsweise die Plasmabehandlung, die besonders bei DLC und DLN (Diamond-like Carbon, also diamantartiger Kohlenstoff, Diamond-like Nanocomposite, also diamantartiges Nanocomposit) verwendet wird, das PVD-Verfahren (Physical Vapour Deposition) oder das CVD-Verfahren (Chemical Vapour Deposition), die Ionenimplantation, das Sputtern, das lonenstrahlverfahren, das Laserverfahren, ein thermisches Verfahren, das Spin Coating, das Dip Coating, das Ätzen oder das Elektropolieren. Auch andere Oberflächenbehandlungsverfahren können verwendet werden. Unter dem Spin Coating wird ein Aufbringen eine gleichmäßig dünnen Films auf einem flachen rotationssymmetrischen Substrat durch Verteilen eines Materials unter Fliehkraft beim schnellen Rotieren des Substrats verstanden. Unter dem Dip Coating wird das Beschichten eines translationssymmetrischen Substrats durch Eintauchen desselben in ein Material (Sol), Herausziehen von diesem unter konstanter Geschwindigkeit, so dass ein flüssiger Film auf der Substratoberfläche haften bleibt und Trocknen desselben verstanden.

Neben den im vorstehenden beschriebenen und in den Figuren gezeigten Ausführungsformen von implantierbaren Einrichtungen können noch zahlreiche weitere gebildet werden, bei denen zumindest einer der distalen und proximalen Abschnitte in der Sekundärform einen nach außen weisenden Teilabschnitt und einen sich als erster aus der Primärform in die Sekundärform entfaltenden, auf den ersten Teilabschnitt zurück oder rückwärts gefalteten zweiten Teilabschnitt aufweist. Hierbei können die beiden Teilabschnitte doppellagig in der vollständig entfalteten Form der implantierbaren Einrichtung aufeinander liegen oder mit einem Abstand zueinander liegen, wobei sich der zu dem anderen Abschnitt weisende Teilabschnitt an der Wandung einer Defektöffnung oder der Implantationsstelle abstützen kann. In jedem der Fälle wird aufgrund der zumindest Doppellagigkeit eine Stabilisierung dieses Abschnitts der implantierbaren Einrichtung erzielt. Anstelle der gezeigten und beschriebenen flachen Ausbildung der proximalen Außenseite des proximalen Abschnitts kann dieser auch ein derart z.B. zentral zusammengefasstes Ende aufweisen, dass dieses nach außen absteht. Die beschriebenen Merkmale können einzeln oder in beliebiger Kombination miteinander vorgesehen werden und dabei vorteilhafte Ausgestaltungen der Erfindung bilden. Alle zu den verschiedenen Ausführungsformen von implantierbaren Einrichtungen genannten Merkmale, insbesondere die, die die Tragstruktur und/oder ein Membranelement betreffen, können bei allen diesen Ausführungsformen vorgesehen sein, auch wenn diese ggf. nur zu der einen oder anderen beschrieben sind. Somit ist eine beliebige Kombination der einzelnen Ausführungsformen und ihrer Merkmale möglich.

### Bezugszeichenliste

- 1: implantierbare Einrichtung
- 1A: erste Hälfte
- 1 B: zweite Hälfte
- 2: Öffnung
- 3: Wandung
- 4: Membranelement
- 5: drahtartiges Element
- 6: drahtartiges Element
- 7: drahtartiges Element
- 8: drahtartiges Element
- 9: drahtartiges Element
- 10: proximaler Abschnitt
- 11: distaler Abschnitt
- 12: Innenraum
- 13: Teilabschnitt
- 14: Teilabschnitt
- 15: Zwischenabschnitt
- 16: Öffnung / zentrale Durchgangsöffnung
- 17: Tragstruktur
- 18: Äußerer Rand
- 19: Element
- 20: Innerer Bereich
- 21: Äußerer Rand
- 22: Zentraler Bereich
- 23: Katheter
- 23A: Schleusenkatheter
- 24: Umlaufende Kante
- 25: Proximales Ende
- 26: Hutkrempen-Abschnitt
- 27: Kopfteil-Abschnitt
- 30: Außenseite der Wandung
- 31: Aorta
- 40: Abwurfdraht
- 40A: aufgerollte Spitze (pigtail)
- 41: Haltdraht
- 42: Ummantelung
- 50: drahtartiges Element
- 51: Ende
- 52: drahtartiges Element
- 55: drahtartiges Element
- 60: drahtartiges Element
- 66: drahtartiges Element
- 81: Schlaufe
- 82: Ende
- 85: Tragring
- 87: Schlinge
- 88: verdrillter Abschnitt
- 100: Herz
- 101: Shunt
- 103: Betätigungsteil
- 104: Griffteil
- 105: Polyesterfaser
- 109: eingefalteter Teilabschnitt
- 110: Teilabschnitt
- 111: Left Appendix
- 112: Anschlussbereich
- 112A: Beinchen
- 112B: Beinchen
- 115: Teilabschnitt
- 116: Scheibenförmiger Abschnitt
- 117: Distaler Abschnitt
- 118: Zwischenabschnitt
- 119: Teilabschnitt
- 133: Teilabschnitt
- 142: Pusher
- 144: Teilabschnitt
- 555: drahtartiges Element
- 666: drahtartiges Element
- x: Achse

## Patentansprüche

1. Implantierbare Einrichtung (1) zur Verwendung im menschlichen und/oder tierischen Körper zum Verschluss oder Teilverschluss von Defektöffnungen (2), Hohlräumen, Organwegen etc. oder zum Schaffen einer definierten Verbindungsöffnung zwischen Wandungen, Organen, Hohlräumen, etc., mit einer Tragstruktur (17), die in einer Primärform ein großes Verhältnis von Länge zu Querausdehnung entlang einer Achse (x) und in einer Sekundärform ein kleineres Verhältnis von Länge zu Querausdehnung entlang der Achse (x) aufweist, wobei die Tragstruktur (17) einen proximalen und einen distalen Abschnitt (10, 11) aufweist und nach Art eines Geflechts und/oder Gewebes und/oder Geleges und/oder Netzes geformt ist,
wobei
zumindest der eine Abschnitt (11) in der Sekundärform einen von dem anderen Abschnitt (10) wegweisenden ersten Teilabschnitt (14) und zum Bilden zumindest einer Doppellagigkeit einen sich als erster aus der Primärform in die Sekundärform entfaltenden in Richtung zu oder weg von dem anderen Abschnitt (10) auf den ersten Teilabschnitt (14) zurück gefalteten zweiten Teilabschnitt (13) aufweist,
**dadurch gekennzeichnet, dass**
der umgefaltete Teilabschnitt (13) nach innen in die implantierbare Einrichtung (1) hinein zurück gefaltet ist.

2. Implantierbare Einrichtung (1) nach Anspruch 1,
**dadurch gekennzeichnet, dass** zwischen dem proximalen und dem distalen Abschnitt (10, 11) ein Zwischenabschnitt (15) mit gegenüber dem proximalen und dem distalen Abschnitt (10, 11) verminderten Durchmesser vorgesehen ist.

3. Implantierbare Einrichtung (1) nach Anspruch 1 oder 2,
**dadurch gekennzeichnet, dass**
zumindest der eine Abschnitt zumindest in seinem Randbereich im Wesentlichen scheibenförmig oder bauchförmig konvex ausgebildet ist.

4. Implantierbare Einrichtung (1) nach einem der vorstehenden Ansprüche,
**dadurch gekennzeichnet, dass**
der eine Abschnitt scheibenförmig und der andere Abschnitt konvex ausgebildet ist.

5. Implantierbare Einrichtung (1) nach Anspruch 3,
**dadurch gekennzeichnet, dass**
der eine Abschnitt eine Tragstruktur (17) mit einem im Wesentlichen geschlossenen Ende aufweist.

6. Implantierbare Einrichtung (1) nach Anspruch 5,
**dadurch gekennzeichnet, dass**
das im Wesentlichen geschlossene Ende als ein Geflecht- und/oder Gelege- und/oder Gewebe- und/oder Netzenden der Tragstruktur (17) zusammenfassendes, in einem Hülsenelement aufgenommenes Ende ausgebildet ist.

7. Implantierbare Einrichtung (1) nach Anspruch 1,
**dadurch gekennzeichnet, dass**
die implantierbare Einrichtung (1) stentförmig ausgebildet ist mit einem doppellagig aufeinander gefalteten endseitigen Abschnitt (11).

8. Implantierbare Einrichtung (1) nach Anspruch 7,
**dadurch gekennzeichnet, dass**
der zweite Teilabschnitt (13) nach innen auf den ersten Teilabschnitt (14) zurück gefaltet ist.

9. Implantierbare Einrichtung (1) nach einem der vorstehenden Ansprüche,
**dadurch gekennzeichnet, dass**
im Bereich des proximalen und/oder distalen Abschnitts (10, 11) und/oder zwischen dem proximalen und distalen Abschnitt (10, 11) zumindest ein Membranelement (4) vorgesehen ist.

10. Implantierbare Einrichtung (1) nach Anspruch 9,
**dadurch gekennzeichnet, dass**
das Membranelement (4) an einem Geflecht, Gewebe, Gelege oder Netz an der Tragstruktur (17) der implantierbaren Einrichtung (1) befestigt ist.

11. Implantierbare Einrichtung (1) nach Anspruch 9 oder 10,
**dadurch gekennzeichnet, dass**
die Befestigung zwischen Membranelement (4) und Tragstruktur (17) durch Vernähen im Bereich des äußeren Randes (18) der Tragstruktur vorgesehen ist.

12. Implantierbare Einrichtung (1) nach Anspruch 9, 10 oder 11,
**dadurch gekennzeichnet, dass**
die Befestigung des Membranelements (4) an der Tragstruktur (17) durch Umschlingen eines Randes des Membranelements (4) und von randseitigen Schlingen der Tragstruktur (17) mit einem fadenartigen Element (19) und Vorsehen zumindest einer Verknotung an einer randseitigen Schlinge der Tragstruktur (17) vorgesehen ist.

13. Implantierbare Einrichtung (1) nach einem der Ansprüche 9 bis 12,
**dadurch gekennzeichnet, dass**
die Abmessungen des Membranelements (4) etwa denen einer zu verschließenden Defektöffnung (2) entsprechen.

14. Implantierbare Einrichtung (1) nach einem der Ansprüche 9 bis 12,
**dadurch gekennzeichnet, dass**
das Membranelement (4) so bemessen und an der implantierbaren Einrichtung (1) befestigt ist, dass das Membranelement (4) in der Sekundärform der implantierbaren Einrichtung (1) diese im Wesentlichen vollständig abdeckt, insbesondere den distalen Abschnitt (11) im Wesentlichen vollständig abdeckt.

15. Implantierbare Einrichtung (1) nach einem der vorstehenden Ansprüche,
**dadurch gekennzeichnet, dass**
das Geflecht und/oder Netz und/oder Gewebe und/oder Gelege der Tragstruktur (17) der implantierbaren Einrichtung so dicht ausgebildet ist, dass die Tragstruktur (17) als Membranelement wirkt.

16. Implantierbare Einrichtung (1) nach einem der Ansprüche 9 bis 14,
**dadurch gekennzeichnet, dass**
das zumindest eine Membranelement (4) zwischen durch die Tragstruktur gebildete mehrlagig angeordnete Beinchen (112A, 112B) einfügbar oder eingefügt ist.

17. Implantierbare Einrichtung (1) nach einem der vorstehenden Ansprüche,
**dadurch gekennzeichnet, dass**
die implantierbare Einrichtung (1) eine konzentrische oder exzentrische Form aufweist.

18. Implantierbare Einrichtung (1) nach einem der vorstehenden Ansprüche,
**dadurch gekennzeichnet, dass**
die Tragstruktur (17) der implantierbaren Einrichtung (1) selbstständig an die Formgebung an einer Implantationsstelle anpassbar ausgebildet ist.

19. Implantierbare Einrichtung (1) nach Anspruch 18,
**dadurch gekennzeichnet, dass**
die Tragstruktur (17) mit dem zumindest einen Teilabschnitt (13) des einen Abschnitts (11), das in der Sekundärform in Richtung zu dem anderen Abschnitt (10) zurück gefaltet ist, selbständig an die Formgebung einer Implantationsstelle anpassbar ausgebildet ist.

20. Implantierbare Einrichtung (1) nach Anspruch 18 oder 19,
**dadurch gekennzeichnet, dass**
sich die Tragstruktur (17) in jeder Richtung ihrer Erstreckung an die Implantationsstelle anpasst oder an diese anpassbar ausgebildet ist.

21. Implantierbare Einrichtung (1) nach einem der Ansprüche 1 bis 17,
**dadurch gekennzeichnet, dass**
die implantierbare Einrichtung (1) an die Formgebung an einer Implantationsstelle angepasst vorgeformt und die Formgebung als Sekundärform eingeprägt ist.

22. Implantierbare Einrichtung (1) nach einem der vorstehenden Ansprüche,
**dadurch gekennzeichnet, dass**
der in Richtung zu dem anderen Abschnitt (10, 11) zurück gefaltete zweite Teilabschnitt (13) schmal in Form eines umlaufenden dünnen Randes ausgebildet ist.

23. Implantierbare Einrichtung (1) nach einem der vorstehenden Ansprüche,
**dadurch gekennzeichnet, dass**
der Durchmesser des Zwischenabschnitts und/oder der Durchmesser einer sich durch die implantierbare Einrichtung (1) hindurch erstreckenden Durchgangsöffnung (16) vorbestimmbar dimensioniert ist und zum Schaffen einer definierten Verbindungsöffnung zwischen Wandungen, Organen, Hohlräumen in einem menschlichen oder tierischen Körper.

24. Implantierbare Einrichtung (1) nach einem der Ansprüche 1 bis 21,
**dadurch gekennzeichnet, dass**
der in Richtung zu dem anderen Abschnitt (10, 11) zurück gefaltete zweite Teilabschnitt (13) sich bis nahe an oder bis an den Zwischenabschnitt (15) heran erstreckt, insbesondere an diesem zur Anlage kommt.

25. Implantierbare Einrichtung (1) nach einem der vorstehenden Ansprüche,
**dadurch gekennzeichnet, dass**
die Tragstruktur (17) der implantierbaren Einrichtung (1) aus einem Formgedächtnismaterial, insbesondere einem Metall oder einer Metalllegierung, insbesondere aus Nitinol, oder einem Kunststoffmaterial besteht.

26. Implantierbare Einrichtung (1) nach einem der Ansprüche 9 bis 25,
**dadurch gekennzeichnet, dass**
das Membranelement (4) aus einem Kunststoffmaterial, insbesondere aus Polyester, besteht.

27. Implantierbare Einrichtung (1) nach einem der vorstehenden Ansprüche,
**dadurch gekennzeichnet, dass**
zumindest eine der Oberflächen der implantierbaren Einrichtung (1), insbesondere der Tragstruktur (17) und/oder des Membranelements (4), mit zumindest einem Funktionsmaterial beschichtet oder behandelt ist.

28. Implantierbare Einrichtung nach Anspruch 27,
**dadurch gekennzeichnet, dass**
das Funktionsmaterial ausgewählt ist aus der Gruppe bestehend aus anorganischen Materialien, keramischen Materialien, synthetischen Polymeren, humanen Biopolymeren, Medikamentenbeschichtungen, medikamentenfreisetzenden Polymeren, Biomolekülen, funktionalen Gruppen, genetischen Materialien.

29. Implantierbare Einrichtung (1) nach Anspruch 28,
**dadurch gekennzeichnet, dass**
das Funktionsmittel ausgewählt ist aus der Gruppe bestehend aus Gold, Biogold, diamantartigem Carbon, diamantartigem Nanocomposit, Iridiumoxid, nanoporösem AL₂OL₃, Siliciumkarbid, Hydroxylapatit, Titannitritoxid, Poly(2-chlor-p-xylylen), Polybutylmethacrylat, Phosphorycholin, Polyethylen, Polyethylen Vinylacetat, Polyhexylmethacrylat, Polybis(trifluoroethoxy)phosphazen, Polytetraflourethylen, Polyurethan, Collagen, Chondroitinsulfat, Elastin, Fibrin, Hydraluronsäure, Abciximab, Heparin, Paclitaxel, Abciximab freisetzender Cellulose, Angiopeptin freisetzendem Phosphorylcholin, DNA freisetzendem Polyethylenvinylacetat oder Polybutylmethacrylat, ein Medikament oder eine Substanz freisetzendes Pol(L-lactid), Paclitaxel oder Hirudin oder Iloprost freisetzendem Polylactid, viral vector freisetzendem Polylactid-Phosphorylcholin, Forskolin freisetzendem Polyurethan, Stammzellen.

30. Implantierbare Einrichtung (1) nach einem der Ansprüche 27 bis 29,
**dadurch gekennzeichnet, dass**
die zumindest eine Oberfläche der implantierbaren Einrichtung (1) durch Plasmabehandlung, ein PVD-Verfahren, ein CVD-Verfahren, lonenimplantation, Sputtern, lonenstrahlverfahren, Laserverfahren, ein thermisches Verfahren, Spin Coating, Dip Coating, Ätzen, Elektropolieren behandelt ist.

31. Implantierbare Einrichtung (1) nach einem der vorstehenden Ansprüche,
**dadurch gekennzeichnet, dass**
der distale Abschnitt (11) der implantierbaren Einrichtung (1) unabhängig von deren proximalen Abschnitt (11) entfaltbar ist.

32. Implantierbare Einrichtung (1) nach einem der vorstehenden Ansprüche,
**dadurch gekennzeichnet, dass**
die implantierbare Einrichtung (1) aus einem einzigen oder mehreren drahtartigen Elementen (5) geformt ist.

33. Implantierbare Einrichtung (1) nach Anspruch 32,
**dadurch gekennzeichnet, dass**
das zumindest eine drahtartige Element (5) eine runde oder flache Querschnittsform oder eine beliebige Kombination aus einer flachen und runden Querschnittsform aufweist.

34. Implantierbare Einrichtung (1) nach Anspruch 32 oder 33,
**dadurch gekennzeichnet, dass**
die Enden des einen drahtartigen Elements (5) in der Fläche der Tragstruktur (17) miteinander verwoben sind.

35. Implantierbare Einrichtung (1) nach Anspruch 32, 33 oder 34,
**dadurch gekennzeichnet, dass**
die Tragstruktur (17) der implantierbaren Einrichtung (1) endseitig eine oder mehrere Schlingen (87) ausbildet.

36. Implantierbare Einrichtung (1) nach Anspruch 35,
**dadurch gekennzeichnet, dass**
die Schlingen (87) in Richtung zu dem übrigen Teil der Tragstruktur (17) in einen verdrillten oder verflochtenen Abschnitt (88) übergehen.

37. Implantierbare Einrichtung (1) nach einem der vorstehenden Ansprüche,
**dadurch gekennzeichnet, dass**
die Tragstruktur (17) der implantierbaren Einrichtung (1) miteinander verwobene oder verflochtene verdrillte drahtartige Elemente (5) oder miteinander verdrillte Abschnitte drahtartiger Elemente (5) umfasst, die an Kreuzungspunkten einzeln oder als verdrillte Elemente durch einander hindurch geflochten sind.

38. Implantierbare Einrichtung nach einem der vorstehenden Ansprüche,
**dadurch gekennzeichnet, dass**
die Tragstruktur (17) aus zumindest einem drahtartigen Element (5) einlagig, doppellagig oder mehrlagig ausgebildet ist.

39. Implantierbare Einrichtung (1) nach Anspruch 38,
**dadurch gekennzeichnet, dass**
die einzelnen Lagen oder Schichten der Tragstruktur (17) aus dem gleichen Material bestehen.

40. Implantierbare Einrichtung (1) nach Anspruch 38,
**dadurch gekennzeichnet, dass**
die einzelnen Lagen oder Schichten der Tragstruktur (17) aus unterschiedlichen Materialien bestehen.

41. Implantierbare Einrichtung (1) nach Anspruch 40,
**dadurch gekennzeichnet, dass**
zumindest eine der Lagen aus Nitinol, zumindest eine der Lagen aus Polyester und zumindest eine der Lagen aus PTFE besteht.

42. Implantierbare Einrichtung (1) nach einem der Ansprüche 38 bis 40,
**dadurch gekennzeichnet, dass**
die Tragstruktur (17) der implantierbaren Einrichtung (1) mit Fasern (105) durchflochten ist, insbesondere mit Polymerfasern.

43. Implantierbare Einrichtung (1) nach einem der Ansprüche 38 bis 41,
**dadurch gekennzeichnet, dass**
zwischen zumindest zwei Lagen der Tragstruktur (17) zumindest ein Membranelement (4) angeordnet ist.

44. Implantierbare Einrichtung (1) nach einem der vorstehenden Ansprüche,
**dadurch gekennzeichnet, dass**
zumindest ein Element (52) als Marker zum Sichtbarmachen der implantierten Einrichtung (1) unter einer Überwachungseinrichtung während eines Implantationsvorgangs vorgesehen ist.

45. Implantierbare Einrichtung (1) nach Anspruch 44
**dadurch gekennzeichnet, dass**
das zumindest eine Element (52) eine randseitig (18) an der implantierbaren Einrichtung (1) vorgesehene Mikrospirale ist.

46. Implantierbare Einrichtung (1) nach Anspruch 44 oder 45,
**dadurch gekennzeichnet, dass**
das zumindest eine Element (52) aus einem röntgensichtbaren Material besteht, insbesondere aus einem 70 bis 90% Platin und 30 bis 10% Iridium enthaltenden Material.

47. Implantierbare Einrichtung (1) nach Anspruch 44,
**dadurch gekennzeichnet, dass**
Marker ein Röntgenmarker, Ultraschallmarker, Echomarker oder Magnetresonanzmarker ist.

48. Implantierbare Einrichtung (1) nach einem der vorstehenden Ansprüche
**dadurch gekennzeichnet, dass**
der zumindest eine Abschnitt (10, 11) der Tragstruktur (17) schneckenartig aufgewickelt ist.

49. Implantierbare Einrichtung (1) nach Anspruch 48,
**dadurch gekennzeichnet, dass**
beide Abschnitte (10, 11) schneckenartig aufgewickelt sind.

50. Implantierbare Einrichtung (1) nach Anspruch 48 oder 49,
**dadurch gekennzeichnet, dass**
der zumindest eine schneckenartig aufgewickelte Abschnitt (11) mehrere zu dem jeweils anderen Abschnitt (10) weisende Teilabschnitte (13, 133) und mehrere von dem anderen Abschnitt (10) weg weisende Teilabschnitte (14, 144) aufweist.

51. Implantierbare Einrichtung (1) nach einem der Ansprüche 48 bis 50,
**dadurch gekennzeichnet, dass**
der andere Abschnitt (10) eine andere Formgebung als der schneckenartig aufgewickelte Abschnitt (11) aufweist.

52. Implantierbare Einrichtung (1) nach Anspruch 51,
**dadurch gekennzeichnet, dass**
der andere Abschnitt (10) mit einer Durchgangsöffnung versehen ist.

53. Implantierbare Einrichtung (1) nach Anspruch 51,
**dadurch gekennzeichnet, dass**
der andere Abschnitt (10) im Wesentlichen geschlossen ausgebildet ist.

54. Implantierbare Einrichtung (1) nach einem der vorstehenden Ansprüche
**dadurch gekennzeichnet, dass**
die implantierbare Einrichtung (1) zur Implantation bei einem persistierenden ductus arteriosus hutförmig mit zumindest einem doppellagigen hutkrempenförmigen Abschnitt (26) ausgebildet ist.

55. Implantierbare Einrichtung (1) nach Anspruch 54,
**dadurch gekennzeichnet, dass**
der doppellagige hutkrempenförmige Abschnitt (26) als zumindest zwei aufeinander gefaltete Teilabschnitte (13, 14) aufweisender Abschnitt (10) ausgebildet ist.

56. Implantierbare Einrichtung (1) nach Anspruch 54 oder 55,
**dadurch gekennzeichnet, dass**
der dem hutkrempenförmigen Abschnitt (26) gegenüberliegende kopfteilförmige Abschnitt (27) der implantierbaren Einrichtung (1) nach innen in das Innere der implantierbaren Einrichtung (1) eingestülpt ist.

57. Implantierbare Einrichtung (1) nach Anspruch 54 oder 55,
**dadurch gekennzeichnet, dass**
der dem hutkrempenförmige Abschnitt (26) gegenüberliegende kopfteilförmige Abschnitt (27) der implantierbaren Einrichtung (1) nach außen aus der implantierbaren Einrichtung (1) ausgestülpt ist, so dass eine Tropfenform des kopfteilförmigen Abschnitts (27) gebildet ist.

## Claims

1. Implantable device (1) for use in the human and/or animal body for closure or partial closure of defect openings (2), cavities, organ passages, etc. or for providing a defined communicating opening between walls, organs, cavities, etc., comprising a carrier structure (17), which in a primary form has a large ratio of length to transverse extent along an axis (x) and in a secondary form has a smaller ration of length to transverse extent along the axis (x), wherein the carrier structure (17) has a proximal and a distal portion (10, 11) and is formed in the manner of a weft and/or mesh and/or layered cloth and/or gauze, wherein at least the one portion (11) in the secondary form has a first part (14) facing outwardly away from the other portion (10) and a second part, which deploys first from the primary form into the secondary from and which is folded back in a direction towards or away from the other portion (10) on to the first part (14), **characterized in that** the folded-over part (13) is folded back inwardly into the implantable device (1).

2. Implantable device (1) according to claim 1, **characterized in that** between the proximal and the distal portions (10, 11) an intermediate section (15) of a diameter which is reduced in relation to the proximal and the distal portions (10, 11) is provided.

3. Implantable device (1) according to claim 1 or claim 2, **characterized in that** at least the one portion is of a substantially disk-shaped convex-bulging configuration in its edge region.

4. Implantable device (1) according to one of the previous claims, **characterized in that** the one portion is disk-shaped and the other portion is convex-shaped.

5. Implantable device (1) according to claim 3, **characterized in that** the one portion has a carrier structure (17) with a substantially closed end.

6. Implantable device (1) according to claim 5, **characterized in that** the substantially closed end is in the form of an end, which is accommodated in a sleeve element and which combines weft and/or layered cloth and/or mesh and/or gauze ends of the carrier structure (17).

7. Implantable device (1) according to claim 1, **characterized in that** the implantable device (1) is formed as a stent with a double-layer folded on to each other at the end portion (11).

8. Implantable device (1) according to claim 7, **characterized in that** the second part (13) is folded inwardly back on to the first part (14).

9. Implantable device (1) according to one of the previous claims, **characterized in that** in at least one membrane element (4) is provided the region of the proximal and/or distal portion (10, 11) and/or between the proximal and distal portions (10, 11).

10. Implantable device (1) according to claim 9, **characterized in that** the membrane element (4) is fixed to the weft, mesh, layered cloth or gauze of the carrier structure (17) of the implantable device (1).

11. Implantable device (1) according to claim 9 or claim 10, **characterized in that** the fixing between membrane element (4) and carrier structure (17) is provided by sewing in the region of the outer edge (18) of the carrier structure (17).

12. Implantable device (1) according to claim 9, 10 or 11, **characterized in that** the fixing of the membrane element (4) to the carrier structure (17) is provided by embracing an edge of the membrane element (4) and edge loops of the carrier structure (17) with a thread-like element (19) and the provision of at least one knotting at an edge loop of the carrier structure (17).

13. Implantable device (1) according to one of claims 9 to 12, **characterized in that** the dimensions of the membrane element (4) approximately correspond to those of a defect opening (2) to be closed.

14. Implantable device (1) according to one of claims 9 to 12, **characterized in that** the membrane element (4) is so dimensioned and fixed to the implantable device (1) that the membrane element (4) in the secondary form of the implantable device (1) substantially completely covers it over and in particular substantially completely covers over the distal portion (11).

15. Implantable device (1) according to one of the previous claims, **characterized in that** the weft and/or gauze and/or mesh and/or layered cloth of the carrier structure (17) of the implantable device is so dense that the carrier structure (17) acts as a membrane element.

16. Implantable device (1) according to one of claims 9 to 14, **characterized in that** the at least one membrane element (4) is insertable or inserted between the formed multi-layered legs (112A, 112B), which are formed by the carrier structure.

17. Implantable device (1) according to one of the previous claims, **characterized in that** the implantable device (1) is of a concentric or eccentric form.

18. Implantable device (1) according to one of the previous claims, **characterized in that** the carrier structure (17) of the implantable device (1) automatically adapts to the configuration at an implantation location.

19. Implantable device (1) according to claim 18, **characterized in that** the carrier structure (17) with the at least one part (13) of the one portion (11), which is folded back in the direction towards the other portion (10) in the secondary form, automatically adapts to the configuration of the implantation location.

20. Implantable device (1) according to claim 18 or claim 19, **characterized in that** the carrier structure (17) is adapted to or is designed to be adaptable to the implantation location in any direction in which it extents.

21. Implantable device (1) according to one of the claims 1 to 17, **characterized in that** the implantable device (1) is shaped to be adapted to the configuration at an implantation location and the shaping is imparted thereto as the secondary form.

22. Implantable device (1) according to one of the previous claims, **characterized in that**, the second part (13), which is folded back in the direction towards the other portion (10, 11) is narrow in the form of a peripherally extending thin edge.

23. Implantable device (1) according to one of the previous claims, **characterized in that** the diameter of the intermediate portion and/or the diameter of a through opening (16) extending through the implantable device (1) is predeterminably dimensioned for providing a defined communicating opening between walls, organs, cavities in a human and/or animal body.

24. Implantable device (1) according to one of claims 1 to 21, **characterized in that** the second part (13), which is folded back in a direction towards the other portion (10, 11) extends close to or to the intermediate portion (15), in particular comes to bear thereagainst.

25. Implantable device (1) according to one of the previous claims, **characterized in that** the carrier structure (17) of the implantable device (1) comprises a shape memory material, in particular a metal or a metal alloy, in particular Nitinol or a plastic material.

26. Implantable device (1) according to one of claims 9 to 25, **characterized in that** the membrane element (4) comprises a plastic material, in particular polyester.

27. Implantable device (1) according to one of the previous claims, **characterized in, that** at least one of the surfaces of the implantable device (1), in particular of the carrier structure (17) and/or of the membrane element (4), is coated or treated with at least one functional material.

28. Implantable device (1) according to claim 27, **characterized in that** the functional material is selected from the group consisting of inorganic materials, ceramic materials, synthetic polymers, human biopolymers, medication coatings, medication-liberating polymers, biomolecules, functional groups, genetic materials.

29. Implantable device (1) according to claim 28, **characterized in that** the functional material is selected from the group consisting of gold, biogold, diamond-like carbon, diamond-like nanocomposite, iridium oxide, nanoporous Al₂O₃, silicon carbide, hydroxylapatite, titanium nitrite oxide, poly (2-chloro-p-xylylene), polybutylmethacrylate, phosphorylcholine, polyethylene, polyethylene vinylacetate, polyhexymethacrylate, poly [bis] (trifluroetoxy) phosphazene, polytetrafluoroethylene, polyurethane, collagen, chondroitin sulfate, elastin, fibrin, hydraluronic acid, abciximab, heparin, paclitaxel, abciximab-liberating cellulose, angiopeptin-liberating phosphorylcholine, DNA-liberating polyethylene vinylacetate or polybutylmethacrylate, a medication-liberating or substance-liberating poly(L-lactide), paclitaxel or herudin or iloprost-liberating polylactide, viral vector-liberating polylactide-phosphoryl choline, Forskolin-liberating polyurethane, stem cells.

30. Implantable device (1) according to one of claims 27 to 29, **characterized in that** the at least one surface of the implantable device (1) is treated by plasma treatment, a PVD process, a CVD process, ion implantation, sputtering, an ion beam process, a laser process, a thermal process, spin coating, dip coating, etching, electro polishing.

31. Implantable device (1) according to one of the previous claims, **characterized in that** the distal portion (11) of the implantable device (1) is deployable independently of the proximal portion (11) thereof.

32. Implantable device (1) according to one of the previous claims, **characterized in that** the implantable device (1) is formed from a single or a plurality of wire-like elements (5).

33. Implantable device (1) according to claim 32, **characterized in that** the at least one wire-like element (5) is of a round or flat cross-sectional shape or is of any combination of a flat and round cross-sectional shape.

34. Implantable device (1) according to claim 32 or 33, **characterized in that** the ends of the one wire-like element (5) are woven together in the surface of the carrier structure (17).

35. Implantable device (1) according to claims 32, 33 or 34, **characterized in that** the carrier structure (17) of the implantable device (1) forms one or more loops (87) at the end.

36. Implantable device (1) according to claim 35, **characterized in that** the loops (87) migrate into a twisted or interwoven section (88) in the direction of the remaining part of the carrier structure (17).

37. Implantable device (1) according to one of the previous claims, **characterized in that** the carrier structure (17) of the implantable device (1) has twisted wire-like elements (5) which are woven together or interlaced or mutually twisted poritons of wire-like elements (5), which are woven through each other at intersection points individually or in form of twisted elements.

38. Implantable device (1) according to one of the previous claims, **characterized in that** the carrier structure (17) is formed from at least one wire-like element (5) in a single-layer, double-layer or multi-layer configuration.

39. Implantable device (1) according to claim 38, **characterized in that** the individual layers or strata of the carrier structure (17) comprise of the same material.

40. Implantable device (1) according to claim 38, **characterized in that** the individual layers or strata of the carrier structure (17) comprise of different materials.

41. Implantable device (1) according to claim 40, **characterized in that** at least one of the layers comprises Nitinol, at least one of the layers comprises polyester and at least one of the layers comprises PTFE.

42. Implantable device (1) according to one of claims 38 to 40, **characterized in that** the carrier structure (17) of the implantable device (1) is interlaced with fibers (105), in particular with fibers made of polymer.

43. Implantable device (1) according to one of claims 38 to 41, **characterized in that** at least one membrane element (4) is arranged between at least two layers of the carrier structure (17).

44. Implantable device (1) according to one of the previous claims, **characterized in that** there is provided at least one element (52) as a marker for visually indicating the implantable device (1) under a monitoring apparatus during an implantation procedure.

45. Implantable device (1) according to claim 44, **characterized in that** the at least one element (52) is a micro-spiral provided at the edge (18) on the implantable device (1).

46. Implantable device (1) according to claim 44 or 45, **characterized in that** the at least one element (52) comprises an X-ray-visible material, in particular a material containing 70 to 90 % platinum and 30 to 10 % iridium.

47. Implantable device (1) according to claim 44, **characterized in that** the marker is a X-ray-marker, an ultrasonic marker, an ecomarker or a magnetic resonance marker.

48. Implantable device (1) according to one of the previous claims, **characterized in that** the at least one portion (10, 11) of the carrier structure (17) is wound up in a helix-like configuration.

49. Implantable device (1) according to claim 48, **characterized in that** both portions (10, 11) are wound up in a helix-like configuration.

50. Implantable device (1) according to claim 48 or 49, **characterized in that** the at least one section (11), which is wounded up in a helix-like configuration, has a plurality of parts (13, 133) facing towards the respective other portion (10) and a plurality of parts (14, 144) facing away from the other portion (10).

51. Implantable device (1) according to one of claims 48 to 50, **characterized in that** the other portion (10) is of a different configuration than the portion (11), which is wound up in a helix-like configuration.

52. Implantable device (1) according to claim 51, **characterized in that** the other portion (10) is provided with a through opening.

53. Implantable device (1) according to claim 51, **characterized in that** the other portion (10) is substantially closed.

54. Implantable device (1) according to one of the previous claims, **characterized in that** the implantable device (1) is for the implantation in relation to a persistent ductus arteriosus and is of a hat-shaped configuration with at least one double-layer hat brim-shaped portion (26).

55. Implantable device (1) according to claim 54, **characterized in that** the double-layer hat brim-shaped portion (26) is in the form of a portion (10) comprising at least two parts (13, 14), which are folded on to each other.

56. Implantable device (1) according to claim 54 or 55, **characterized in that** the head part-shaped portion (27) of the implantable device (1), which is arranged opposite to the hat brim-shaped portion (26), is turned over inwardly into the interior of the implantable device (1).

57. Implantable device (1) according to claim 54 or 55, **characterized in that** the head part-shaped portion (27) of the implantable device (1), which is arranged opposite to the hat brim-shaped portion (26), is turned inside out of the implantable device (1) so that a drop shape of the head part-shaped portion (27) is formed.

## Revendications

1. Dispositif implantable (1) destiné à l'utilisation dans le corps humain et/ou animal pour obturer ou partiellement obturer des ouvertures de défaut (2), des cavités, des conduits du corps etc. ou pour créer une ouverture de communication définie entre des parois, des organes, des cavités etc., comprenant une structure portante (17), qui comprend en forme primaire un grand rapport de la longueur à l'extension transversale le long d'un axe (x) et en forme secondaire un plus petit rapport de la longueur à l'extension transversale le long d'un axe (x), la structure portante (17) comprenant une section proximale et une section distale (10, 11) et étant configurée à la façon d'un entrelacs et/ou d'un tissu et/ou d'une natte et/ou d'un filet,
dans lequel au moins l'une section (11) comprend en forme secondaire une première section partielle (14) s'écartant de l'autre section (10) et une deuxième section partielle (13), qui se déplie le premier de la forme primaire dans la forme secondaire pour former au moins une double couche et qui est repliée sur la première section partielle (14) dans un sens de rapprochement ou d'éloignement de l'autre section (10),
**caractérisé en ce que**
la section partielle (13) pliée est repliée vers l'intérieur du dispositif implantable (1).

2. Dispositif implantable (1) selon la revendication 1,
**caractérisé en ce qu'**
une section intermédiaire (15) est prévue entre la section proximale et la section distale (10, 11), la section intermédiaire comprenant un diamètre plus faible par rapport à celui de la section proximale et de la section distale (10, 11).

3. Dispositif implantable (1) selon la revendication 1 ou la revendication 2,
**caractérisé en ce qu'**
au moins la zone marginale de l'au moins une section est essentiellement conçue en forme de disque ou en forme bombée de façon convexe.

4. Dispositif implantable (1) selon l'une des revendications précédentes,
**caractérisé en ce que**
l'une section est en forme de disque et l'autre section est convexe.

5. Dispositif implantable (1) selon la revendication 3,
**caractérisé en ce que**
l'une section comprend une structure portante (17) ayant une extrémité essentiellement fermée.

6. Dispositif implantable (1) selon la revendication 5,
**caractérisé en ce que**
l'extrémité essentiellement fermée est conçue comme une extrémité qui rassemble les extrémités d'entrelacs et/ou de natte et/ou de tissu et/ou de filet de la structure portante (17) et qui est reçue dans un élément de manchon.

7. Dispositif implantable (1) selon la revendication 1,
**caractérisé en ce que**
le dispositif implantable (1) est conçu en forme de stent ayant une section d'extrémité (11) repliée sur elle-même en deux couches.

8. Dispositif implantable (1) selon la revendication 7,
**caractérisé en ce que**
la deuxième section partielle (13) est repliée vers l'intérieur sur la première section partielle (14).

9. Dispositif implantable (1) selon l'une des revendications précédentes,
**caractérisé en ce qu'**
au moins un élément de membrane (4) est prévu au niveau de la section proximale et/ou distale (10, 11) et/ou entre la section proximale et la section distale (10, 11).

10. Dispositif implantable (1) selon la revendication 9,
**caractérisé en ce que**
l'élément de membrane (4) est fixé à un entrelacs, à un tissu, à une natte ou à un filet de la structure portante (17) du dispositif implantable (1).

11. Dispositif implantable (1) selon la revendication 9 ou la revendication 10,
**caractérisé en ce qu'**
il est prévu de fixer l'élément de membrane (4) à la structure portante (17) par couture au niveau du bord extérieur (18) de la structure portante.

12. Dispositif implantable (1) selon la revendication 9, 10 ou 11,
**caractérisé en ce qu'**
il est prévu de fixer l'élément de membrane (4) à la structure portante (17) par l'enlacement d'un bord de l'élément de membrane (4) et des boucles sur le bord de la structure portante (17) par un élément en forme de fil (19) et par réalisation d'au moins un noeud dans une boucle sur le bord de la structure portante (17).

13. Dispositif implantable (1) selon l'une des revendications 9 à 12,
**caractérisé en ce que**
les dimensions de l'élément de membrane (4) correspondent approximativement à celles d'une ouverture de défaut (2) à obturer.

14. Dispositif implantable (1) selon l'une des revendications 9 à 12,
**caractérisé en ce que**
l'élément de membrane (4) est dimensionné et fixé au dispositif implantable (1) de sorte que l'élément de membrane (4) couvre en forme secondaire du dispositif implantable (1) celui-ci sensiblement de manière complète, notamment la section distale (11) sensiblement de manière complète.

15. Dispositif implantable (1) selon l'une des revendications précédentes,
**caractérisé en ce que**
l'entrelacs et/ou le filet et/ou le tissu et/ou la natte de la structure portante (17) du dispositif implantable est conçu(e) tellement dense que la structure portante (17) agit comme élément de membrane.

16. Dispositif implantable (1) selon l'une des revendications 9 à 14,
**caractérisé en ce que**
l'au moins un élément de membrane (4) peut être inséré ou est inséré entre de petites jambes (112A, 112B) formées par la structure portante et disposées en plusieurs couches.

17. Dispositif implantable (1) selon l'une des revendications précédentes,
**caractérisé en ce que**
le dispositif implantable (1) a une forme concentrique ou excentrique.

18. Dispositif implantable (1) selon l'une des revendications précédentes,
**caractérisé en ce que**
la structure portante (17) du dispositif implantable (1) est conçue de sorte qu'elle peut s'adapter elle-même à la forme étant présente à un site d'implantation.

19. Dispositif implantable (1) selon la revendication 18,
**caractérisé en ce que**
l'au moins une section partielle (13) de l'une section (11) de la structure portante (17), laquelle section partielle est repliée, en forme secondaire, dans la direction vers l'autre section (10), est conçue de sorte qu'elle peut s'adapter elle-même à la forme étant présente à un site d'implantation.

20. Dispositif implantable (1) selon la revendication 18 ou la revendication 19,
**caractérisé en ce que**
dans chaque direction de son extension, la structure portante (17) est conçue de sorte qu'elle est adaptée ou peut s'adapter au site d'implantation.

21. Dispositif implantable (1) selon l'une des revendications 1 à 17,
**caractérisé en ce que**
le dispositif implantable (1) est préformé de façon adapté à la forme d'un site d'implantation et la forme est retenue comme forme secondaire.

22. Dispositif implantable (1) selon l'une des revendications précédentes,
**caractérisé en ce que**
la deuxième section partielle (13) repliée dans la direction de l'autre section (10, 11) est étroite en forme d'un bord fin circonférentiel.

23. Dispositif implantable (1) selon l'une des revendications précédentes,
**caractérisé en ce que**
le diamètre de la section intermédiaire et/ou le diamètre d'une ouverture de passage qui s'étend à travers le dispositif implantable (1) est dimensionné de manière pré-définissable et sert à créer une ouverture de communication définie entre des parois, des organes, des cavités dans un corps humain ou animal.

24. Dispositif implantable (1) selon l'une des revendications 1 à 21,
**caractérisé en ce que**
la deuxième section partielle (13) repliée dans la direction de l'autre section (10, 11) s'étend jusqu'au voisinage de la section intermédiaire (15) ou jusqu'à la section intermédiaire (15) et notamment vient en appui sur celle-ci.

25. Dispositif implantable (1) selon l'une des revendications précédentes,
**caractérisé en ce que**
la structure portante (17) du dispositif implantable (1) est constituée d'un matériau à mémoire de forme, notamment d'un métal ou d'un alliage métallique, notamment de nitinol ou d'une matière plastique.

26. Dispositif implantable (1) selon l'une des revendications 9 à 25,
**caractérisé en ce que**
l'élément de membrane est réalisé en matière plastique, notamment en polyester.

27. Dispositif implantable (1) selon l'une des revendications précédentes,
**caractérisé en ce qu'**
au moins une des surfaces du dispositif implantable (1), notamment de la structure portante (17) et/ou de l'élément de membrane est revêtue de ou traitée avec au moins un matériau fonctionnel.

28. Dispositif implantable selon la revendication 27,
**caractérisé en ce que**
le matériau fonctionnel est sélectionné dans le groupe composé de matériaux inorganiques, de matériaux céramiques, de polymères synthétiques, de bio-polymères humains, de revêtements de médicament, de polymères libérant des médicaments, de biomolécules, de groupes fonctionnels, de matériaux génétiques.

29. Dispositif implantable (1) selon la revendication 28,
**caractérisé en ce que**
le matériau fonctionnel est sélectionné dans le groupe composé d'or, de Biogold, de carbone diamant, de nanocomposite de type diamant, d'oxyde d'iridium, d'Al₂Ol₃ nanoporeux, de carbure de silicium, d'hydroxyapatite, d'oxyde de nitrite de titane, de poly(2-chlore-p-xylylène), de polybutyleméthacyrlate, de phosphorycholine, de polyéthylène, de polyéthylène-acétate de vinyle, de méthacrylate de polyhexyle, de polybis(trifluoroéthoxy)phosphazène, de polytétraflouréthylène, de polyuréthane, de collagène, de sulfate de chondroïtine, d'élastine, de fibrine, d'acide hyaluronique, d'abciximab, d'héparine, de paclitaxel, de cellulose libérant de l'abciximab, de phosphorylcholine libérant de l'angiopeptine, de polyéthylène-acétate de vinyle ou de polybutyle-méthacrylate libérant de l'ADN, de pol(L-lactide) libérant un médicament ou une substance, de polylactide libérant du paclitaxel ou de l'hirudine ou de l'iloprost, de polylactide-phosphorylcholine libérant des vecteurs viraux, de polyuréthane libérant du forskoline, de cellules souches.

30. Dispositif implantable (1) selon l'une des revendications 27 à 29,
**caractérisé en ce qu'**
au moins une surface du dispositif implantable (1) est traitée par traitement par plasma, par un procédé PVD, par un procédé CVD, par implantation ionique, par pulvérisation cathodique, par traitement par faisceau ionique, par traitement laser, par un procédé thermique, par spin-coating, par dip-coating, par gravure, par électropolissage.

31. Dispositif implantable (1) selon l'une des revendications précédentes,
**caractérisé en ce que**
la section distale (11) du dispositif implantable (1) peut de déplier indépendamment de la section proximale (11) de celui-ci.

32. Dispositif implantable (1) selon l'une des revendications précédentes,
**caractérisé en ce que**
le dispositif implantable (1) est formé à partir d'un seul ou de plusieurs éléments en forme de fil métallique.

33. Dispositif implantable (1) selon la revendication 32,
**caractérisé en ce que**
l'au moins un élément en forme de fil métallique (5) comprend une section transversale ronde ou plate ou toute combinaison d'une section transversale plate et d'une section transversale ronde.

34. Dispositif implantable (1) selon la revendication 32 ou la revendication 33,
**caractérisé en ce que**
les extrémités de l'un élément en forme de fil métallique (5) sont entrelacées l'une à l'autre dans la zone de la structure portante (17).

35. Dispositif implantable (1) selon la revendication 32, 33 ou 34,
**caractérisé en ce que**
la structure portante (17) du dispositif implantable (1) forme une ou plusieurs boucles (87) du côté d'extrémité.

36. Dispositif implantable (1) selon la revendication 35,
**caractérisé en ce que**
dans la direction de l'autre partie de la structure portante (17) les boucles (87) passent dans une section torsadée ou entrelacée (88).

37. Dispositif implantable (1) selon l'une des revendications précédentes,
**caractérisé en ce que**
la structure portante (17) du dispositif implantable (1) comprend des éléments en forme de fil métallique (5) torsadés qui sont tissés ou entrelacés les uns avec les autres ou des sections torsadées les unes avec les autres des éléments en forme de fil métallique (5), lesquelles sont individuellement ou comme éléments torsadés entrelacées les unes à travers les autres à des points de croisement.

38. Dispositif implantable selon l'une des revendications précédentes,
**caractérisé en ce que**
la structure portante (17) est formée par au moins un élément en forme de fil métallique (5) qui forme une couche, deux couches ou plusieurs couches.

39. Dispositif implantable (1) selon la revendication 38,
**caractérisé en ce que**
les couches individuelles de la structure portante (17) sont constituées du même matériau.

40. Dispositif implantable (1) selon la revendication 38,
**caractérisé en ce que**
les couches individuelles de la structure portante (17) sont constituées de matériaux différents.

41. Dispositif implantable (1) selon la revendication 40,
**caractérisé en ce qu'**
au moins une des couches est constituée de nitinol, au moins une des couches est constituée de polyester et au moins une des couches est constituée de PTFE.

42. Dispositif implantable (1) selon l'une des revendications 38 à 40,
**caractérisé en ce que**
la structure portante (17) du dispositif implantable (1) est tressée avec des fibres (105), notamment des fibres en polymère.

43. Dispositif implantable (1) selon l'une des revendications 38 à 41,
**caractérisé en ce qu'**
au moins un élément de membrane (4) est disposé entre au moins deux couches de la structure portante (17).

44. Dispositif implantable (1) selon l'une des revendications précédentes,
**caractérisé en ce qu'**
au moins un élément (52) est prévu comme marqueur pour rendre le dispositif implantable (1) visible au-dessous d'un dispositif de surveillance pendant une procédure d'implantation.

45. Dispositif implantable (1) selon la revendication 44,
**caractérisé en ce que**
l'au moins un élément (52) est une micro-spirale prévue sur le bord (18) du dispositif implantable (1).

46. Dispositif implantable (1) selon la revendication 44 ou la revendication 45,
**caractérisé en ce que**
l'au moins un élément (52) est constitué d'un matériau radio-opaque, notamment d'un matériau contenant 70 à 90% de platine et 30 à 10% d'iridium.

47. Dispositif implantable (1) selon la revendication 44,
**caractérisé en ce que**
le marqueur est un marqueur radio-opaque, un marqueur à ultrason, un marqueur d'écho ou un marqueur à résonance magnétique.

48. Dispositif implantable (1) selon l'une des revendications précédentes,
**caractérisé en ce que**
l'au moins une section (10, 11) de la structure portante (17) est enroulée hélicoïdalement.

49. Dispositif implantable (1) selon la revendication 48,
**caractérisé en ce que**
les deux sections (10, 11) sont enroulées hélicoïdalement.

50. Dispositif implantable (1) selon la revendication 48 ou la revendication 49,
**caractérisé en ce que**
l'au moins une section (11) enroulée hélicoïdalement comprend plusieurs sections partielles (13, 133) dirigées vers l'autre section (10) respective et plusieurs sections partielles (14, 144) s'écartant de l'autre section (10).

51. Dispositif implantable (1) selon l'une des revendications 48 à 50,
**caractérisé en ce que**
l'autre section (10) comprend une autre configuration que la section (11) enroulée hélicoïdalement.

52. Dispositif implantable (1) selon la revendication 51,
**caractérisé en ce que**
l'autre section (10) est munie d'une ouverture de passage.

53. Dispositif implantable (1) selon la revendication 51,
**caractérisé en ce que**
l'autre section (10) est essentiellement fermée.

54. Dispositif implantable (1) selon l'une des revendications précédentes,
**caractérisé en ce que**
le dispositif implantable (1) est en forme de chapeau et comprend au moins une section (26) en forme de bord d'un chapeau et ayant une double couche pour l'implantation dans le cas d'une persistance du canal artériel.

55. Dispositif implantable (1) selon la revendication 54,
**caractérisé en ce que**
la section (26) en forme de bord d'un chapeau et ayant une double couche est configurée comme une section (10) comprenant deux sections partielles (13, 14) pliées l'une sur l'autre.

56. Dispositif implantable (1) selon la revendication 54 ou la revendication 55,
**caractérisé en ce que**
la section (27) en forme de tête du dispositif implantable (1), laquelle section est opposée à la section (26) en forme de bord d'un chapeau, est rentrée dans l'intérieur du dispositif implantable (1).

57. Dispositif implantable (1) selon la revendication 54 ou la revendication 55,
**caractérisé en ce que**
la section (27) en forme de tête du dispositif implantable (1), laquelle section est opposée à la section (26) en forme de bord d'un chapeau, est tournée vers l'extérieur et sort du dispositif implantable (1), de sorte que la section (27) en forme de tête adopte une forme de goutte.
